# EUROPEAN PATENT SPECIFICATION

(11) **EP 2 748 200 B1**
(45) Date of publication and mention of the grant of the patent: **11.04.2018**
(21) Application number: 12748488.9
(22) Date of filing: 21.08.2012
(51) Int. Cl.: C07K 16/30, A61P 35/00

(54) **FC-FREE ANTIBODIES COMPRISING TWO FAB FRAGMENTS AND METHODS OF USE**
FC-FREIE ANTIKÖRPER MIT ZWEI FAB-FRAGMENTEN SOWIE VERWENDUNGSVERFAHREN DAFÜR
ANTICORPS SANS FC COMPRENANT DEUX FRAGMENTS FAB ET PROCÉDÉS D'UTILISATION

(30) Priority: 23.08.2011 EP 11178391
(43) Date of publication of application: 02.07.2014
(73) Proprietor: Roche Glycart AG, 8952 Schlieren (CH)
(72) Inventor: BRUENKER, Peter, CH-8335 Hittnau (CH); JAEGER, Christiane, CH-8304 Wallisellen (CH); KLEIN, Christian, CH-8906 Bonstetten (CH); SCHAEFER, Wolfgang, 68199 Mannheim (DE); UMANA, Pablo, CH-8832 Wollerau (CH)
(74) Representative: Townsend, Carolin
(86) International application number: PCT/EP2012/066219
(87) International publication number: WO 2013/026835

(56) References cited:
- WO-A1-2009/080254
- WO-A1-2010/115551
- WO-A1-2010/136172
- W. SCHAEFER ET AL: "Immunoglobulin domain crossover as a generic approach for the production of bispecific IgG antibodies", PROCEEDINGS OF THE NATIONAL ACADEMY OF SCIENCES, vol. 108, no. 27, 5 July 2011 (2011-07-05) , pages 11187-11192, XP55003817, ISSN: 0027-8424, DOI: 10.1073/pnas.1019002108
- CHAN L A ET AL: "Variable region domain exchange in human IgGs promotes antibody complex formation with accompanying structural changes and altered effector functions", MOLECULAR IMMUNOLOGY, PERGAMON, GB, vol. 41, no. 5, 1 July 2004 (2004-07-01), pages 527-538, XP002519620, ISSN: 0161-5890, DOI: 10.1016/J.MOLIMM.2004.03.034

## Description

### FIELD OF THE INVENTION

The present invention relates to bispecific antibodies comprising at least two fab fragments, wherein the first Fab fragment comprises at least one antigen binding site specific for a first antigen; and the second Fab fragment comprises at least one antigen binding site specific for a second antigen,wherein either the variable regions or the constant regions of the second Fab heavy and light chain are exchanged; and wherein the bispecific antibody is devoid of a Fc domain; methods for their production, pharmaceutical compositions containing said antibodies, and uses thereof.

### BACKGROUND

Monoclonal antibodies (mAbs) are an increasingly important class of therapeutic agents. Apart from mAb products composed of the full-size form of IgG, a wide variety of multispecific recombinant antibody formats have been developed, e.g. tetravalent bispecific antibodies by fusion of, e.g., an IgG antibody format and single chain domains (see e.g. Coloma, M.J., et al., Nature Biotech 15 (1997) 159-163; WO 2001/077342; and Morrison, S.L., Nature Biotech 25 (2007) 1233-1234).
Several other new formats wherein the antibody core structure (IgA, IgD, IgE, IgG or IgM) is no longer retained such as dia-, tria- or tetrabodies, minibodies, several single chain formats (scFv, Bis-scFv), which are capable of binding two or more antigens, have been developed (Holliger, P., et al., Nature Biotech 23 (2005) 1126-1136; Fischer, N., Léger, O., Pathobiology 74 (2007) 3-14; Shen, J., et al., Journal of Immunological Methods 318 (2007) 65-74; Wu, C., et al., Nature Biotech. 25 (2007) 1290-1297). WO2009/080254 discloses a bispecific Fab-construct with a partial Fc-domain. All such formats use linkers either to fuse the antibody core (IgA, IgD, IgE, IgG or IgM) to a further binding protein (e.g. scFv) or to fuse e.g. two Fab fragments or scFvs (Fischer, N., Léger, O., Pathobiology 74 (2007) 3-14). Tandem scFVs are two scFv fragments linked by an extra peptide linker and are also referred to as (scFv)2. By reducing the length of the peptide linker between the variable domains, Diabodies are generated. Adding an extra peptide linker between the two polypeptides yields so called single chain diabodies. US 2007/0274985 relates to antibodies comprising single chain Fab (scFab) fragments. Antibody fragments have both pros and cons as therapeutics compared with full-size monoclonal antibodies: One advantage is that they are smaller and penetrate tissues and tumors more rapidly. In addition, the small size of fragments has been suggested to permit binding to epitopes not accessible to full-sized monoclonal antibodies. On the downside, fragments demonstrate short circulating half-lives in humans, likely due to kidney clearance. The shorter half-life may prevent sufficient accumulation of therapy at the targeted site. Production of antibody fragments is not trivial, as fragments are likely to form aggregates and can be less stable than full-size monoclonal antibodies. In addition, unwanted pairing of noncongnate heavy and light chains results in formation of inactive antigen-binding sites and/or other non-functional undesired side-products, which is a major problem in clinical-scale production and therapeutic application of antibody fragments. In particular Tandem-Fab constructs where two or more Fabs are fused with each other via one connector are not feasible due to the random association of the two light chains resulting in inactive, undesired side products.These drawbacks have now been overcome with the new antibody format of the invention. Provided therein is a new bispecific antibody format that can be easily produced with an increased yield due to a decreased amount of mispaired side-products, which show less aggregation than bispecific antibody fragments known in the art.. Using the crossover approach correct LC association can be enforced without the need for the generation of a common light chain. Common light chan approach is not possible for existing antibodies. In addition, the new bispecific antibody format has a higher molecular weight compared to many conventional bispecific antibody fragments, thus preventing excessive kidney clearance and leading to an improved half-life in vivo. The new bispecific antibody format is fully functional and has comparable or improved binding and activity as corresponding conventional bispecific antibodies.

### SUMMARY

The present invention relates to bispecific antibodies comprising at least two Fab fragments, wherein the first Fab fragment comprises at least one antigen binding site specific for a first antigen; and the second Fab fragment comprises at least one antigen binding site specific for a second antigen wherein either the variable regions or the constant regions of the second Fab heavy and light chain are exchanged; and wherein the bispecific antibody is devoid of a Fc domain such that the constant heavy chain consists solely of one or more CH1 domains and wherein the first and second Fab fragments are connected via a peptide linker. Preferably said peptide linker is a (G4S)2 linker.

In one embodiment said antibody additionally comprises a third Fab fragment. In another embodiment said third Fab fragment comprises at least one antigen binding site specific for the first or second antigen, preferably for the first antigen.

In one embodiment the third Fab fragment is connected to the light chain or the heavy chain of the first Fab fragment. In another embodiment the third Fab fragment is connected to N or C-terminus of the light chain or the heavy chain of the second Fab fragment. In one embodiment the third Fab fragment is connected to the first or second Fab fragment via a peptide linker. Preferably said peptide linker is a (G4S)2 linker.

The bispecific antibodies according to the invention are at least bivalent and can be trivalent or multivalent e.g. tetravalent. In one embodiment said bispecific antibodies are bivalent (1+1 format) with one binding site each targeting a first antigen and a second antigen, respectively. In another embodiment said bispecific antibodies are trivalent (2+1 format) with two binding sites each targeting a first antigen and one binding site targeting a second antigen, as detailed in the following section.

In a second object the present invention relates to a pharmaceutical composition comprising a bispecific antibody of the present invention.

In a third object the present invention relates to a bispecific antibody of the present invention for the treatment of cancer. In another embodiment, use of the bispecific antibody as a medicament is provided. Preferably said use is for the treatment of cancer.

In further objects the present invention relates to a nucleic acid sequence comprising a sequence encoding a heavy chain of a bispecific antibody of the present invention, a nucleic acid sequence comprising a sequence encoding a light chain of a bispecific antibody of the present invention, an expression vector comprising a nucleic acid sequence of the present invention and to a prokaryotic or eukaryotic host cell comprising a vector of the present invention. In addition a method of producing an antibody comprising culturing the host cell so that the antibody is produced is provided.

In a further embodiment an immunoconjugate comprising the bispecific antibody of the invention and a cytotoxic agent is provided.

### BRIEF DESCRIPTION OF THE FIGURES

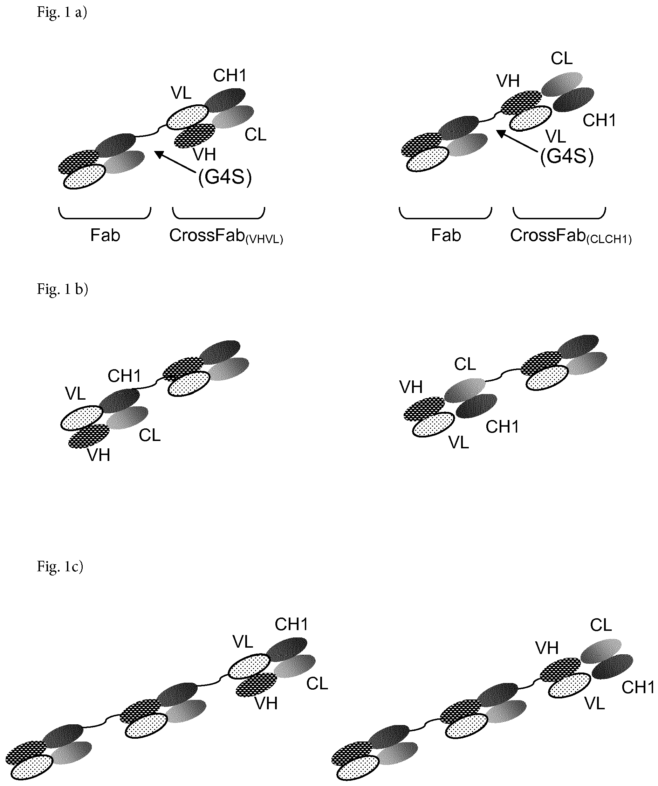

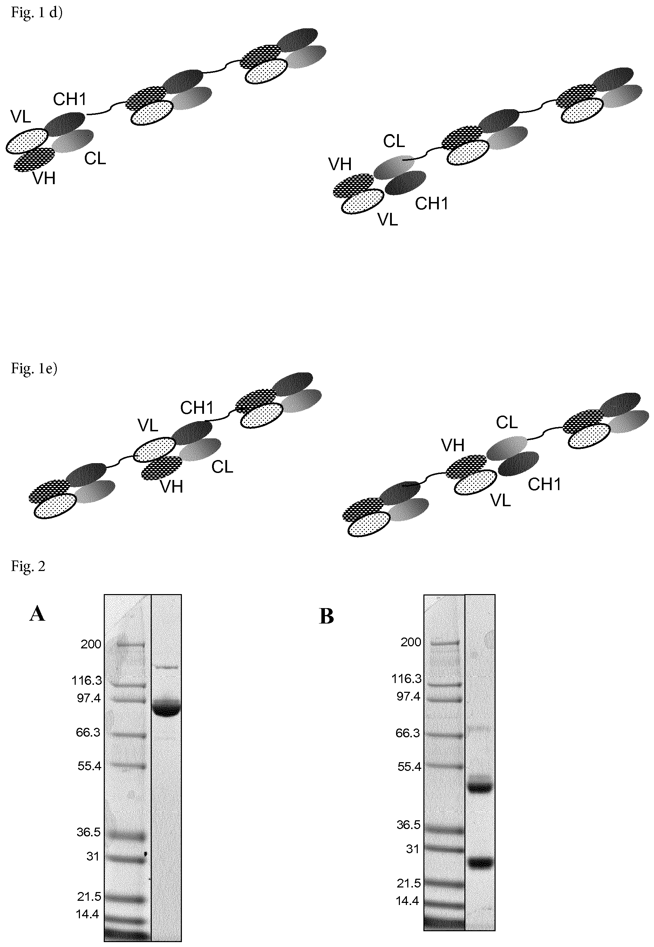

**Figure 1****:** Schematic illustration of exemplary bispecific antibody formats of the invention. a) Fab-Crossfab molecule C-terminal, b) Fab-Crossfab molecule N-terminal c) (Fab)2-Crossfab molecule C-terminal d) (Fab)2-Crossfab molecule N-terminal e) Fab-Crossfab-Fab molecule.
**Figure 2****:** Analysis of hu Fab(MCSP)-Crossfab(CD3) production and purification: SDS-Page: 4-12 % Bis/Tris (NuPage [invitrogen]; coomassie stained): a) 1 - Mark 12 (invitrogen),
   2 - hu Fab(MCSP)-Crossfab(CD3) non reduced; b) 1 - Mark 12 (invitrogen), 2 - hu Fab(MCSP)-Crossfab(CD3) reduced.

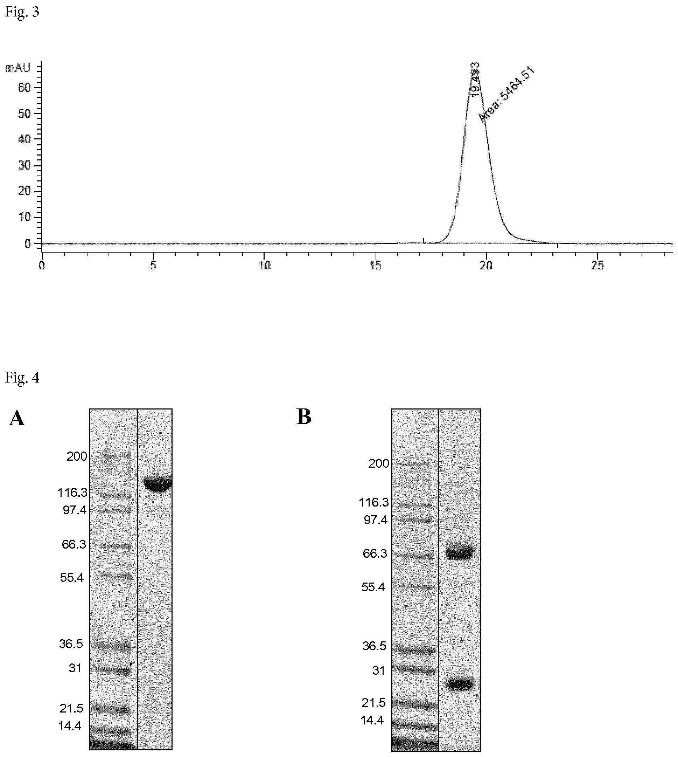

**Figure 3****:** Analysis Fab(MCSP)-Crossfab(CD3) production and purification. Analytical size exclusion chromatography, Chromatogram A280 (Superdex 200 10/300 GL [GE Healthcare]; 2 mM MOPS pH 7.3, 150 mM NaCl, 0.02 % (w/v) NaCl; 50 µg sample were injected).
**Figure 4****:** Analysis of hu Fab(MCSP)-Fab(MCSP)-Crossfab(CD3) production and purification: SDS-Page: 4-12 % Bis/Tris (NuPage [invitrogen]; coomassie stained): a) 1 - Mark 12 (invitrogen), 2 - hu Fab(MCSP)-Fab(MCSP)-Crossfab(CD3) non reduced; b) 1 - Mark 12 (invitrogen), 2 - hu Fab(MCSP)-Fab(MCSP)-Crossfab(CD3) reduced.

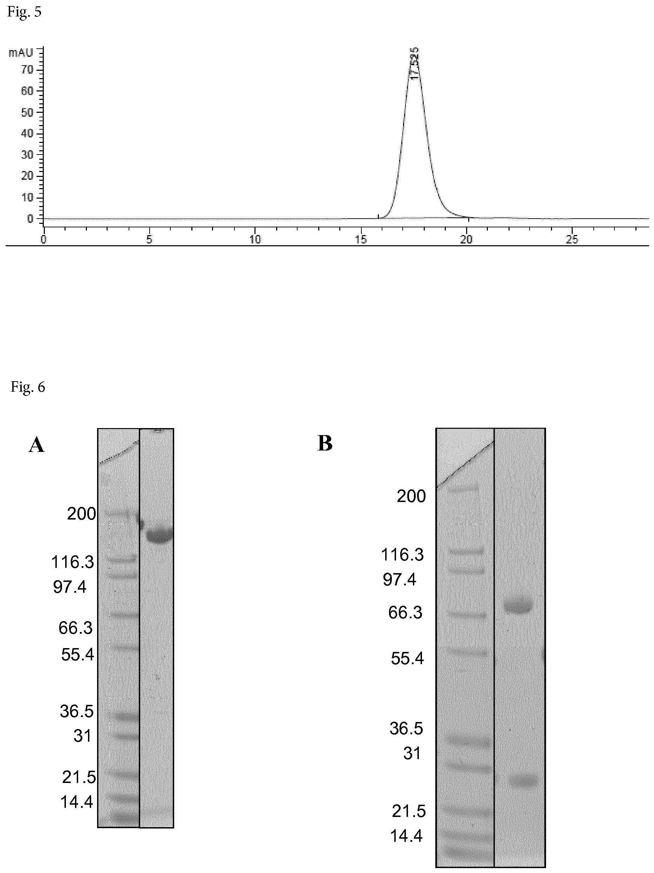

**Figure 5****:** Analysis of hu Fab(MCSP)-Fab(MCSP)-Crossfab(CD3) production and purification. Analytical size exclusion chromatography, Chromatogram A280 (Superdex 200 10/300 GL [GE Healthcare]; 2 mM MOPS pH 7.3, 150 mM NaCl, 0.02 % (w/v) NaCl; 50 µg sample were injected).
**Figure 6****:** Analysis of hu Fab(MCSP)- Crossfab(CD3) -Fab(MCSP) production and purification. SDS-Page: 4-12 % Bis/Tris (NuPage [invitrogen]; coomassie stained): a) 1 - Mark 12 (invitrogen), 2 - hu Fab(MCSP)- Crossfab(CD3) -Fab(MCSP) non reduced; b) 1 - Mark 12 (invitrogen), 2 - hu Fab(MCSP)- Crossfab(CD3) -Fab(MCSP) reduced.

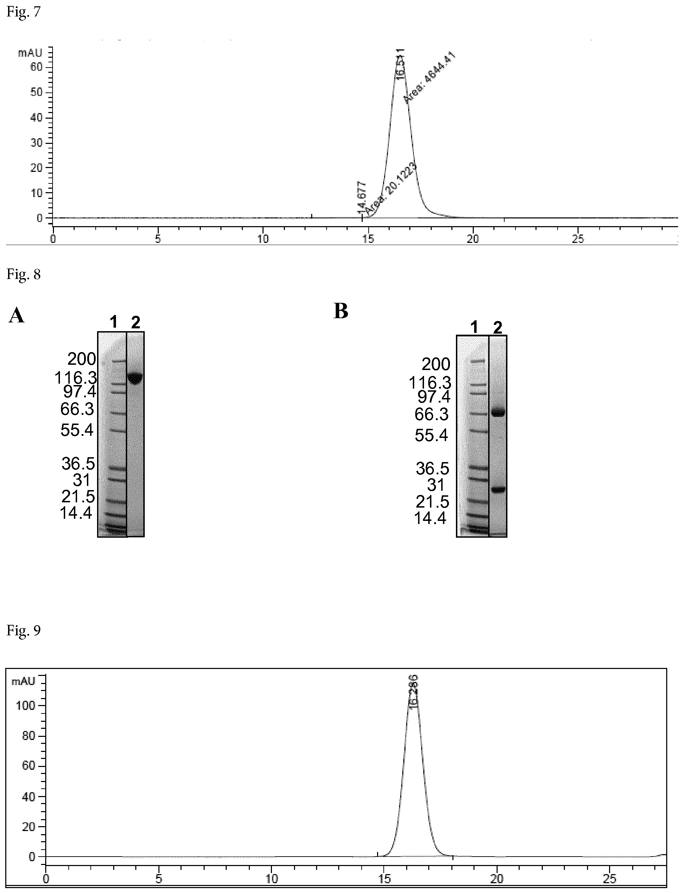

**Figure 7****:** Analysis of hu Fab(MCSP)- Crossfab(CD3) -Fab(MCSP) production and purification. Analytical size exclusion chromatography, Chromatogram A280 (Superdex 200 10/300 GL [GE Healthcare]; 2 mM MOPS pH 7.3, 150 mM NaCl, 0.02 % (w/v) NaCl; 50 µg sample were injected).
**Figure 8****:** Analysis of murine Crossfab(CD3) -Fab(MCSP)-Fab(MCSP) production and purification. SDS-Page: 4-12 % Bis/Tris (NuPage [invitrogen]; coomassie stained): a) 1 - Mark 12 (invitrogen), 2 - murine Crossfab(CD3) -Fab(MCSP)-Fab(MCSP) non reduced; b) 1 - Mark 12 (invitrogen), 2 - murine Crossfab(CD3) -Fab(MCSP)-Fab(MCSP) reduced.
**Figure 9****:** Analysis of murine Crossfab(CD3) -Fab(MCSP)-Fab(MCSP) production and purification. Analytical size exclusion chromatography, Chromatogram A280 (Superdex 200 10/300 GL [GE Healthcare]; 2 mM MOPS pH 7.3, 150 mM NaCl, 0.02 % (w/v) NaCl; 50 µg sample were injected).

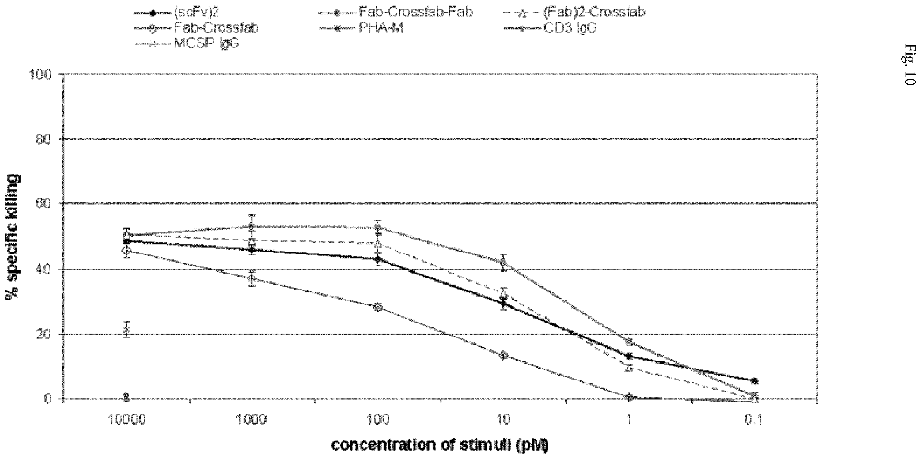

**Figure 10****:** Killing (as measured by LDH release) of MDA-MB-435 tumor cells upon co-culture with human pan T cells (E:T ratio = 5:1) and activation for 20 hours by different concentrations of the hu Fab(MCSP)-Crossfab(CD3) (="Fab-Crossfab"), hu Fab(MCSP)-Crossfab(CD3) -Fab(MCSP) (= "Fab-Crossfab-Fab"), hu Fab(MCSP)-Fab(MCSP)-Crossfab(CD3) (="(Fab)2-Crossfab"), as well as the (scFv)2 (antiMCSP/anti huCD3e) (="(scFv)2") bispecific molecules. The constructs with bivalent MCSP-targeting show comparable cytotoxic activity compared to the "(scFv)2" construct, whereas the "Fab-Crossfab" construct with monovalent MCSP binding is clearly less potent.

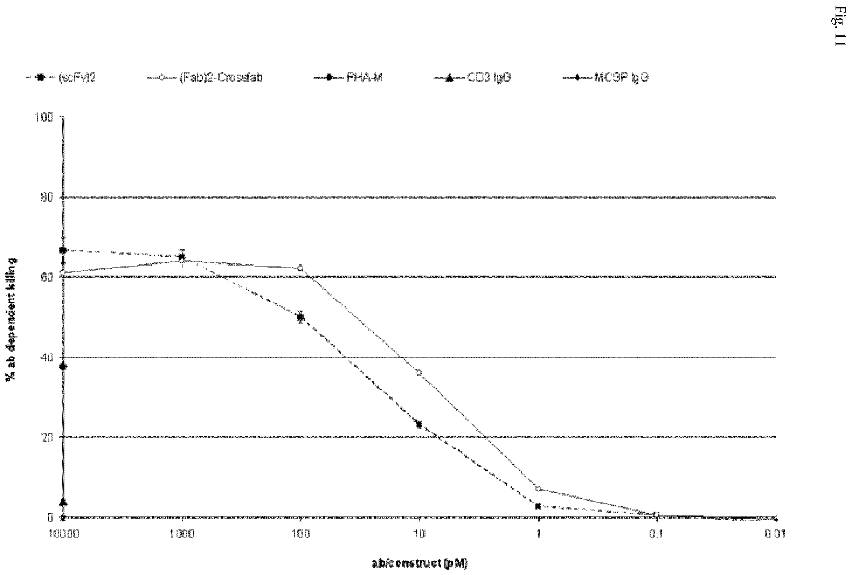

**Figure 11****:** Comparison of the hu Fab(MCSP)-Fab(MCSP)-Crossfab(CD3) (="(Fab)2-Crossfab") and the (scFv)2 (antiMCSP/anti huCD3e) (="(scFv)2") construct, Depicted is the LDH release from MDA-MB-435 tumor cells upon co-culture with human pan T cells (E/T ratio = 5:1), and activation for 21 hours by different concentrations of the bispecific constructs and corresponding IgGs. The "(Fab)2-Crossfab" induces apoptosis in target cells at least comparably good as the (scFv)2 molecule.

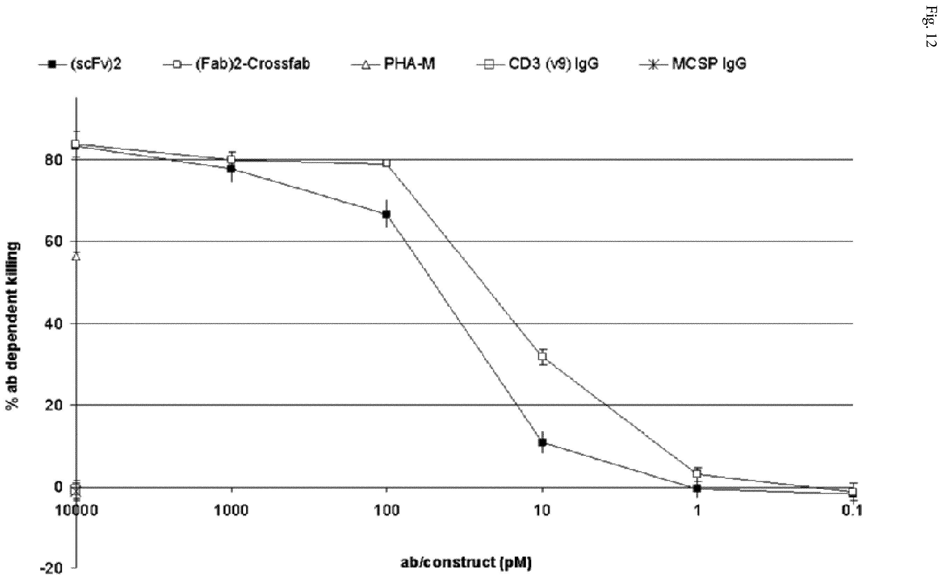

**Figure 12****:** Comparison of the hu Fab(MCSP)-Fab(MCSP)-Crossfab(CD3) (="(Fab)2-Crossfab") and the (scFv)2 (antiMCSP/anti huCD3e) (="(scFv)2") construct. Depicted is the LDH release from MV-3 human melanoma tumor cells upon co-culture with human PBMCs (E/T ratio = 10:1), and activation for 26 hours by different concentrations of the bispecific constructs and corresponding IgGs. The "(Fab)2-Crossfab" induces apoptosis in target cells at least comparably good as the (scFv)2 molecule.

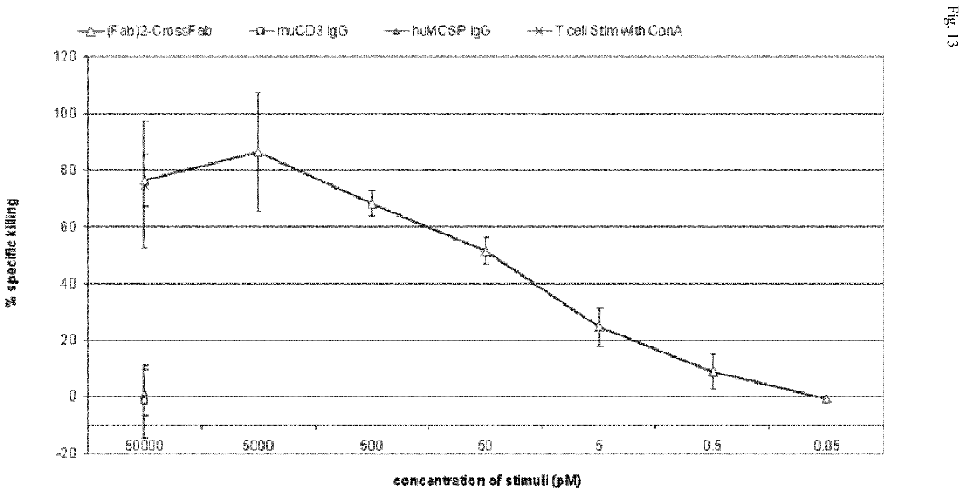

**Figure 13****:** LDH release from B16/F10-huMCSP Fluc2, clone 48 tumor cells, induced by primary murine T cell activation with the murine Crossfab(CD3) -Fab(MCSP)-Fab(MCSP) construct (=(Fab)2-CrossFab), targeting human MCSP, as well as the murine CD3. The effector to target cell ratio was 5:1. The assay was analyzed after incubation for 23.5 hours at 37°C, 5 % CO2. The construct induces concentration-dependent, T cell-mediated apoptosis of human MCSP-expressing target cells.

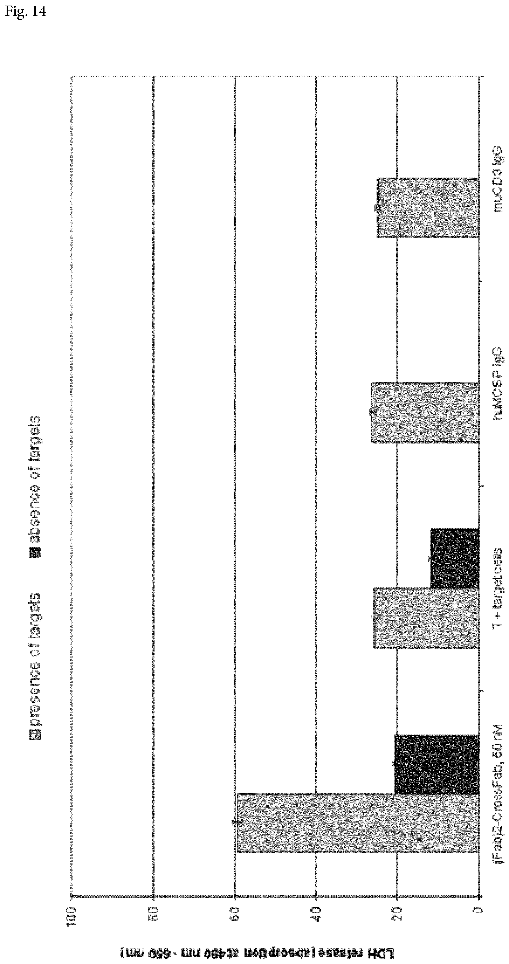

**Figure 14****:** LDH release from B16/F10-huMCSP Fluc2, clone 48 tumor cells, induced by primary murine T cell activation with 50 nM of the murine Crossfab(CD3) -Fab(MCSP)-Fab(MCSP) construct (=(Fab)2-CrossFab), targeting human MCSP, as well as the murine CD3. The effector to target cell ratio was 5:1. The assay was analyzed after incubation for 23.5 hours at 37°C, 5 % CO2. The construct induces T cell-mediated apoptosis of human MCSP-expressing target cells. There is only weak hyperactivation of T cells at this concentration of the construct.

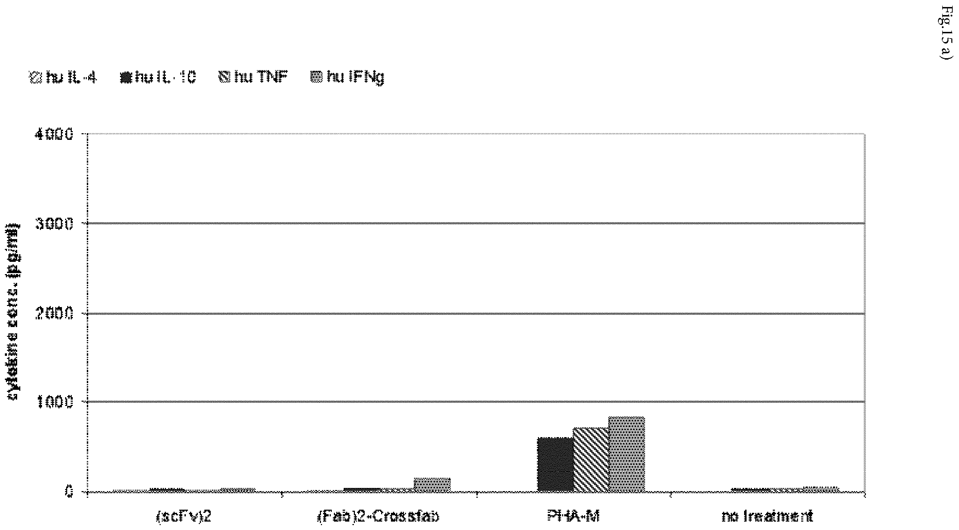

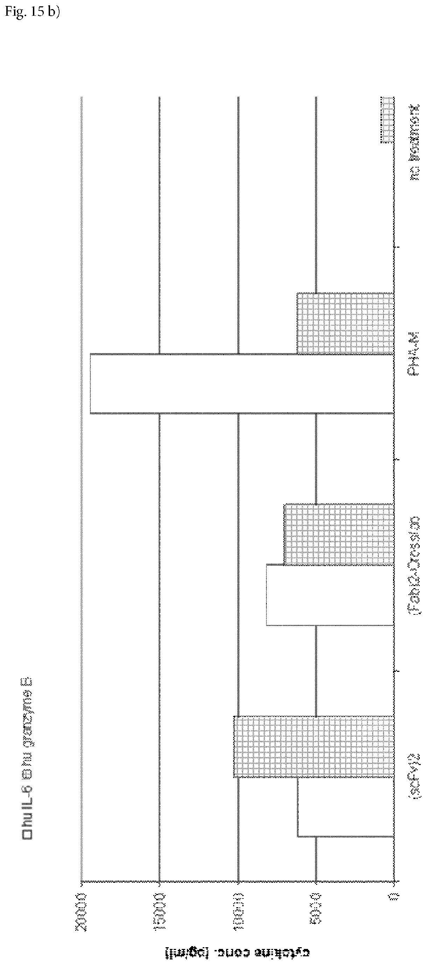

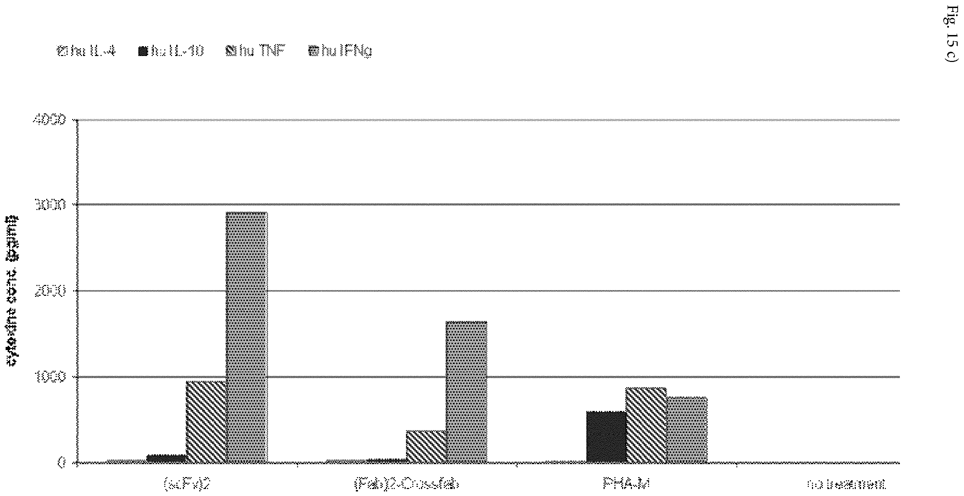

**Figure 15****:** Different cytokine levels measured in the supernatant of whole blood after treatment with 1 nM of different CD3-MCSP bispecific constructs (hu Fab(MCSP)-Fab(MCSP)-Crossfab(CD3) (="(Fab)2-Crossfab") and (scFv)2 (antiMCSP/anti huCD3e) (="(scFv)2")) in the presence (A, B) or absence (C,D) of Colo-38 tumor cells for 24 hours. 280 µl whole blood were plated per well of a 96-well plate and 30 000 Colo-38 cells added, as indicated. The main cytokine that was secreted upon activation of T cells in the presence of Colo-38 tumor cells, is IL-6, followed by IFNgamma. In addition, also the levels of granzyme B increased enormously upon activation of T cells in the presence of target cells. In general, the "(scFv)2" construct elevated the levels of TNF and IFNgamma, as well as granzyme B in the presence of target cells (A and B) a bit more compared to the other bispecific construct.
   There was no significant secretion of Th2 cytokines (IL-10 and IL-4) upon activation of T cells by the bispecific constructs in the presence (or absence) of target cells. In this assay there was also a weak secretion of IFNgamma, induced by the "(Fab)2-Crossfab" construct in the absence of target cells.

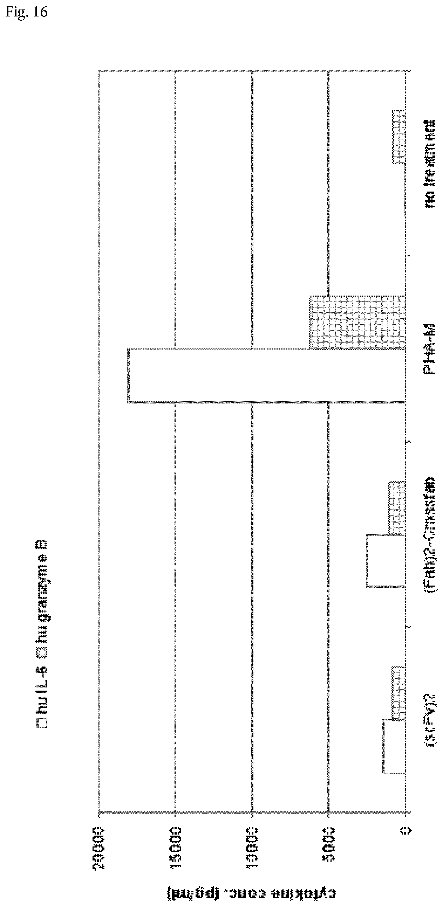

**Figure 16****:** Surface expression level of the late activation marker CD25 on murine pan T cells, isolated from splenocytes. Murine pan T cells were incubated with 50 nM of the murine Crossfab(CD3) -Fab(MCSP)-Fab(MCSP) construct (=(Fab)2-CrossFab) bispecific construct (targeting murine CD3, as well as human MCSP), in the presence or absence of B16/F10-huMCSP Fluc2 clone 48 tumor target cells, as indicated (E:T ratio is 10:1). Depicted is the expression level of the late activation marker CD25 on CD8+ T cells after 70 hours. Up-regulation of CD25 on CD8+ T cells with the (Fab)2-CrossFab construct occurs only in the presence of target cells. The reference IgGs, used adjusted to the same molarity, were not able to up-regulate CD25.

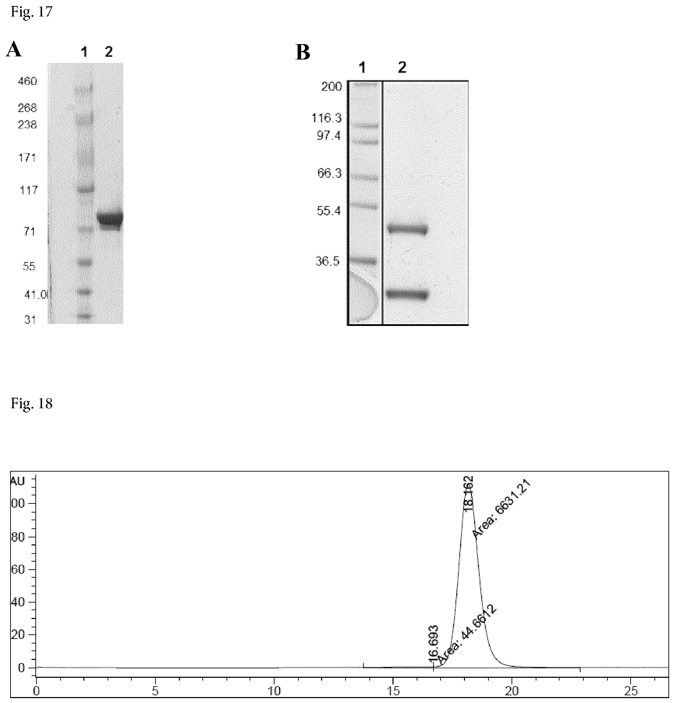

**Figure 17****:** Analysis of Fab(CD33)-CrossFab (CD3) production and purification. SDS-Page: a) 3-8% Tris/Acetate (NuPage [invitrogen]; coomassie stained): a) 1 - HiMark (invitrogen), 2 - Fab(CD33)-CrossFab (CD3) non reduced; b) 4-12 % Bis/Tris (NuPage [invitrogen]: 1 - Mark 12 (invitrogen), 2 - Fab(CD33)-CrossFab (CD3) reduced.
**Figure 18****:** Analysis of Fab(CD33)-CrossFab (CD3) production and purification. Analytical size exclusion chromatography, Chromatogram A280 (Superdex 200 10/300 GL [GE Healthcare]; 2 mM MOPS pH 7.3, 150 mM NaCl, 0.02 % (w/v) NaCl; 50µg sample were injected).

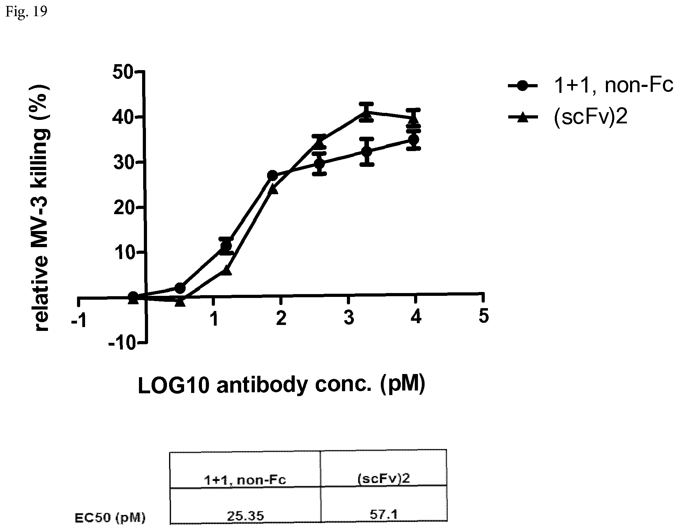

**Figure 19****:** Killing (as measured by LDH release) of MV-3 tumor cells upon co-culture with human PBMCs (E:T ratio = 10:1) and activation for 24 hours by different concentrations of CD3-MCSP bispecific constructs (hu Fab(MCSP)-Crossfab(CD3); designated as "1+1 non-Fc", and the (scFv)2 (antiMCSP/anti huCD3e) (="(scFv)2") reference molecule). The "1+1 non-Fc" construct induces apoptosis in MV-3 target cells with a calculated EC50 of 25.4 pM, whereas the calculated EC50 for the "(scFv)2" reference molecule is 57 pM, showing a slight better potency of the "1+1 non-Fc" molecule in terms of EC50.

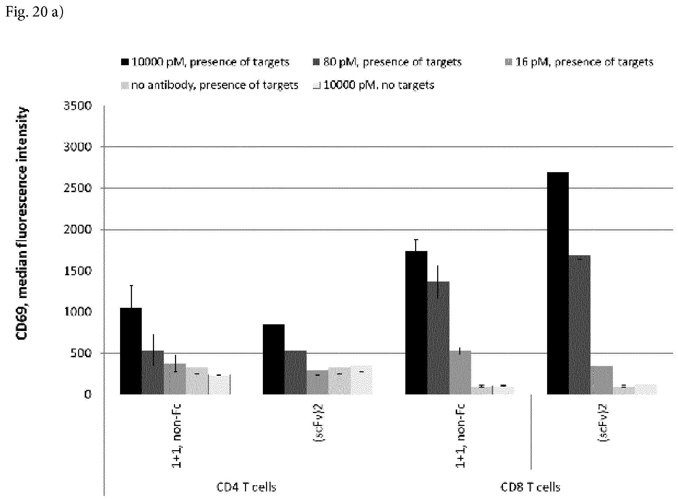

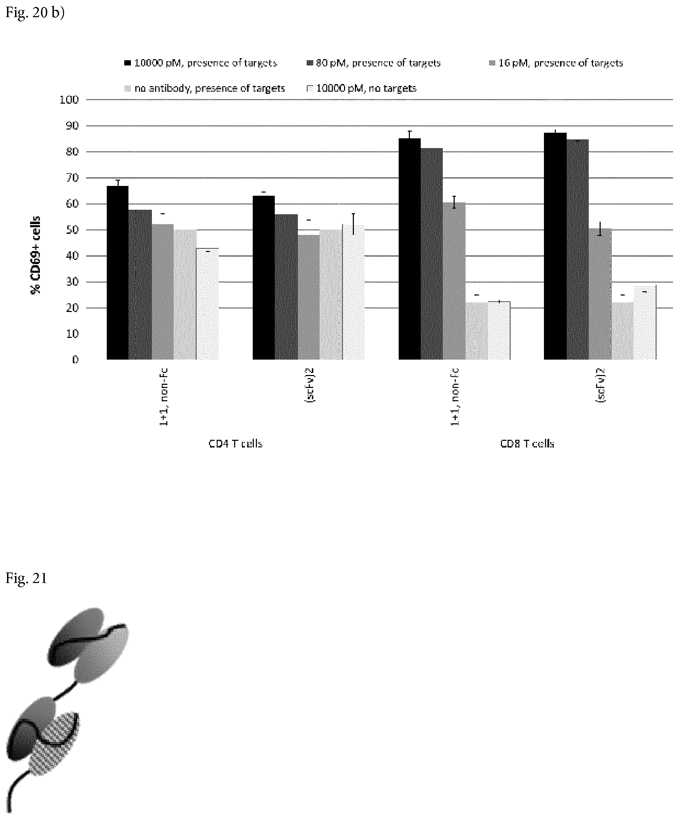

**Figure 20****:** Activation of CD4+ or CD8+ T cells, as measured by up-regulation of CD69 (A), respective increase of CD69-positive cells (B) in the presence of huMCSP-positive MV-3 tumor cells upon co-culture with human PBMCs (E:T ratio = 10:1), treated with the CD3-MCSP bispecific constructs (hu Fab(MCSP)-Crossfab(CD3); designated as "1+1 non-Fc", and the (scFv)2 (antiMCSP/anti huCD3e) (="(scFv)2") reference molecule, respectively) for ~24 hours. In general, the CD69 median values are higher on CD8+ T cells compared to CD4+ T cells. There is a clear concentration-dependent increase in both, CD69 median values, as well percentage of CD69 positive cells for both constructs.
**Figure 21****:** Illustration of (scFv)2 reference molecule.

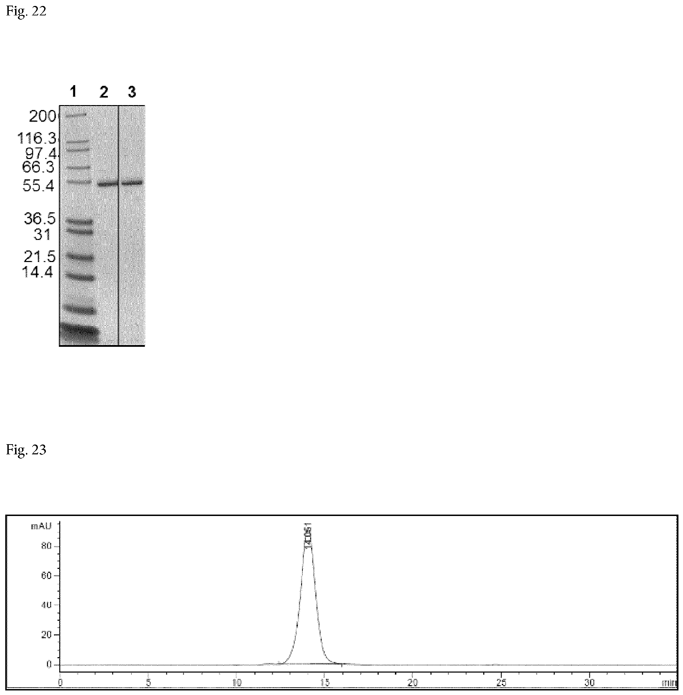

**Figure 22****:** Analysis of (scFv)2 (antiMCSP/anti huCD3e) production and purification. SDS-Page: 4-12 % Bis/Tris (NuPage [invitrogen]; coomassie stained): 1 - Mark 12 (invitrogen), 2 - (scFv)2 (antiMCSP/anti huCD3e) reduced; 3 - (scFv)2 (antiMCSP/anti huCD3e) non reduced
**Figure 23****:** Analysis of (scFv)2 (antiMCSP/anti huCD3e) production and purification Analytical size exclusion chromatography, Chromatogram A280 (Superdex 75 10/300 GL [GE Healthcare]; 2 mM MOPS pH 7.3, 150 mM NaCl, 0.02 % (w/v) NaCl; 50µg sample ((scFv)2 (antiMCSP/anti huCD3e)) were injected).

### DETAILED DESCRIPTION OF EMBODIMENTS OF THE INVENTION

### I. DEFINITIONS

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra*. In one embodiment, for the VH, the subgroup is subgroup III as in Kabat et al., *supra*.

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) The terms hypervariable regions (HVRs) and complementarity determining regions (CDRs), are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table 1 as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE 1. CDR Definitions¹**

| **CDR** | **Kabat** | **Chothia** | **AbM²** |
|---|---|---|---|
| V_{H} CDR1 | 31-35 | 26-32 | 26-35 |
| V_{H} CDR2 | 50-65 | 52-58 | 50-58 |
| V_{H} CDR3 | 95-102 | 95-102 | 95-102 |
| V_{L} CDR1 | 24-34 | 26-32 | 24-34 |
| V_{L} CDR2 | 50-56 | 50-52 | 50-56 |
| V_{L} CDR3 | 89-97 | 91-96 | 89-97 |

| | | | |
|---|---|---|---|
| ¹Numbering of all CDR definitions in Table 1 is according to the numbering conventions set forth by Kabat et al. (see below). ²"AbM" with a lowercase "b" as used in Table 1 refers to the CDRs as defined by Oxford Molecular's "AbM" antibody modeling software. | | | |

Kabat *et al*. also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra*.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity. In particular the term "antibody" also includes the bispecific antibodies of the invention comprising at least two fab fragments but no Fc domain.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

The term "chimeric" antibody refers to an antibody in which a portion of the heavy and/or light chain is derived from a particular source or species, while the remainder of the heavy and/or light chain is derived from a different source or species, usually prepared by recombinant DNA techniques. Chimeric antibodies comprising a murine variable region and a human constant region are preferred. Other preferred forms of "chimeric antibodies" encompassed by the present invention are those in which the constant region has been modified or changed from that of the original antibody to generate the properties according to the invention, especially in regard to C1q binding and/or Fc receptor (FcR) binding. Such chimeric antibodies are also referred to as "class-switched antibodies.". Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See e.g. Morrison, S.L., et al., Proc. Natl. Acad. Sci. USA 81 (1984) 6851-6855; US Patent Nos. 5,202,238 and 5,204,244.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

An "antibody fragment" refers to a molecule other than an intact antibody that comprises a portion of an intact antibody that binds the antigen to which the intact antibody binds. Examples of antibody fragments include but are not limited to Fv, Fab', Fab'-SH, F(ab')₂; diabodies; linear antibodies; single-chain antibody molecules (e.g. scFv); and multispecific antibodies formed from antibody fragments. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

As used herein, "Fab fragment" refers to an antibody fragment comprising a light chain fragment comprising a VL domain and a constant domain of a light chain (CL), and a VH domain and a first constant domain (CH1) of a heavy chain. The bispecific antibodies of the invention comprise at least two Fab fragments, wherein either the variable regions or the constant regions of the heavy and light chain of the second Fab fragment are exchanged. Due to the exchange of either the variable regions or the constant regions, said second Fab fragment is also referred to as "cross-Fab fragment" or "xFab fragment" or "crossover Fab fragment". Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab (VLVH). On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab (CLCH1).

Said Fab fragments are connected via a peptide linker. By "connected" is meant that the Fab fragments are linked by peptide bonds, either directly or via one or more peptide linker. The term "peptide linker" as used within the invention denotes a peptide with amino acid sequences, which is preferably of synthetic origin. These peptide linkers according to invention are used to connect one of the Fab fragments to the C-or N-terminus of the other Fab fragment to form a multispecific antibody according to the invention. Preferably said peptide linkers are peptides with an amino acid sequence with a length of at least 5 amino acids, preferably with a length of 5 to 100, more preferably of 10 to 50 amino acids. In one embodiment said peptide linker is (GxS)n or (GxS)nGm with G = glycine, S = serine, and (x = 3, n= 3, 4, 5 or 6, and m= 0, 1, 2 or 3) or (x = 4,n= 2, 3, 4 or 5 and m= 0, 1, 2 or 3), preferably x = 4 and n= 2 or 3, more preferably with x = 4, n= 2. Additionally, linkers may comprise (a portion of) an immunoglobulin hinge region. In one embodiment said peptide linker is (G₄S)₂ (SEQ ID: NO 28). Other peptide linkers suitable for connecting the Fab fragments, for example, (G₄S)₆-GG (SEQ ID NO: 147) or (SG₃)₂-(SEG₃)₄-(SG₃)-SG (SEQ ID NO: 148), or EPKSC(D)-(G₄S)₂ (SEQ ID NOs 145 and 146).

The term "antigen binding domain" refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more antibody variable domains (also called antibody variable regions). Preferably, an antigen binding domain comprises an antibody light chain variable region (VL) and an antibody heavy chain variable region (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "antigen-binding site of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

The term "Fc domain" herein is used to define a C-terminal region of an immunoglobulin heavy chain that contains at least a portion of the constant region. For example in natural antibodies, the Fc domain is composed of two identical protein fragments, derived from the second and third constant domains of the antibody's two heavy chains in IgG, IgA and IgD isotypes; IgM and IgE Fc domains contain three heavy chain constant domains (C_{H} domains 2-4) in each polypeptide chain. The bispecific antibodies of the invention are devoid of the Fc domain. "Devoid of the Fc domain" as used herein means that the bispecific antibodies of the invention do not comprise a CH2, CH3 or CH4 domain; i.e. the constant heavy chain consists solely of one or more CH1 domains.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (KD). Affinity can be measured by common methods known in the art, including those described herein. Specific illustrative and exemplary embodiments for measuring binding affinity are described in the following.

As used herein, the term "binding" or "specifically binding" means that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding moiety to an unrelated protein is less than about 10% of the binding of the antigen binding moiety to the antigen as measured, e.g., by SPR. In certain embodiments, an antigen binding moiety that binds to the antigen, or an antigen binding molecule comprising that antigen binding moiety, has a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

In one embodiment, the extent of binding of a bispecific antibody that specifically binds a first antigen and a second antigen to an unrelated, protein is less than about 10% of the binding of the antibody to the first or second antigen as measured, e.g., by a radioimmunoassay (RIA) or flow cytometry (FACS). In certain embodiments, a bispecific antibody that specifically binds a first antigen and a second antigen has a dissociation constant (KD) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M). In certain embodiments, a bispecific antibody that specifically binds a first antigen and a second antigen binds to an epitope of the first antigen or the second antigen that is conserved among the first or second antigen from different species.

Antibody specificity refers to selective recognition of the antibody for a particular epitope of an antigen. Natural antibodies, for example, are monospecific. "Bispecific antibodies" according to the invention are antibodies which have two different antigen-binding specificities. Antibodies of the present invention are specific for two different antigens, i.e. for a first antigen and a second antigen.

The term "monospecific" antibody as used herein denotes an antibody that has one or more binding sites each of which bind to the same epitope of the same antigen.

The term "bispecific" antibody as used herein denotes an antibody that has at least two binding sites each of which bind to different epitopes of the same antigen or a different antigen.

The antibody provided herein is a multispecific antibody, e.g. a bispecific antibody. Multispecific antibodies are monoclonal antibodies that have binding specificities for at least two different sites. Provided herein is a bispecific antibody, with binding specificities for a first antigen and a second antigen. In certain embodiments, bispecific antibodies may bind to two different epitopes of a first antigen or a second antigen.

Bispecific antibodies may also be used to localize cytotoxic agents to cells which express a first antigen or a second antigen.

The term "valent" as used within the current application denotes the presence of a specified number of binding sites in an antibody molecule. As such, the terms "bivalent", "tetravalent", and "hexavalent" denote the presence of two binding sites, four binding sites, and six binding sites, respectively, in an antibody molecule. The bispecific antibodies according to the invention are at least "bivalent" and may be "trivalent" or "multivalent" (e.g."tetravalent" or "hexavalent").

Antibodies of the present invention have two or more binding sites and are bispecific. That is, the antibodies may be bispecific even in cases where there are more than two binding sites (i.e. that the antibody is trivalent or multivalent).

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

"No substantial cross-reactivity" means that a molecule (*e.g.,* an antibody) does not recognize or specifically bind an antigen different from the actual target antigen of the molecule (e.g. an antigen closely related to the target antigen), particularly when compared to that target antigen. For example, an antibody may bind less than about 10% to less than about 5% to an antigen different from the actual target antigen, or may bind said antigen different from the actual target antigen at an amount selected from the group consisting of less than about 10%, 9%, 8% 7%, 6%, 5%, 4%, 3%, 2%, 1%, 0.5%, 0.2%, or 0.1%, preferably less than about 2%, 1%, or 0.5%, and most preferably less than about 0.2% or 0.1% antigen different from the actual target antigen.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding a bispecific antibody of the invention" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "amino acid" as used within this application denotes the group of naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

As used herein, the expressions "cell", "cell line", and "cell culture" are used interchangeably and all such designations include progeny. Thus, the words "transfectants" and "transfected cells" include the primary subject cell and cultures derived there from without regard for the number of transfers. It is also understood that all progeny may not be precisely identical in DNA content, due to deliberate or inadvertent mutations. Variant progeny that have the same function or biological activity as screened for in the originally transformed cell are included.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "naked antibody" refers to an antibody that is not conjugated to a heterologous moiety (e.g., a cytotoxic moiety) or radiolabel. The naked antibody may be present in a pharmaceutical formulation.

An "immunoconjugate" is an antibody conjugated to one or more heterologous molecule(s), including but not limited to a cytotoxic agent.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "N-terminus" denotes the last amino acid of the N-terminus, the term "C-terminus" denotes the last amino acid of the C-terminus.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "effective amount" of an agent, e.g., a pharmaceutical formulation, refers to an amount effective, at dosages and for periods of time necessary, to achieve the desired therapeutic or prophylactic result.

The term "cancer" as used herein refers to proliferative diseases, such as lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

The term "package insert" is used to refer to instructions customarily included in commercial packages of therapeutic products, that contain information about the indications, usage, dosage, administration, combination therapy, contraindications and/or warnings concerning the use of such therapeutic products.

### II. COMPOSITIONS AND METHODS

### A. Exemplary bispecific antibodies

The present invention relates to bispecific antibodies comprising at least two Fab fragments, wherein the first Fab fragment comprises at least one antigen binding site specific for a first antigen; and the second Fab fragment comprises at least one antigen binding site specific for a second antigen, wherein either the variable regions or the constant regions of the heavy and light chain of the second Fab fragment are exchanged; and wherein the bispecific antibody is devoid of a Fc domain such that the constant heavy chain consists solely of one or more CH1 domains and wherein the first and second Fab fragments are connected via a peptide linker. Preferably said peptide linker is a peptide with an amino acid sequence with a length of at least 5 amino acids, preferably with a length of 5 to 100, more preferably of 10 to 50 amino acids. In one embodiment said peptide linker is (GxS)n or (GxS)nGm with G = glycine, S = serine, and (x = 3, n= 3, 4, 5 or 6, and m= 0, 1, 2 or 3) or (x = 4,n= 2, 3, 4 or 5 and m= 0, 1, 2 or 3), preferably x = 4 and n= 2 or 3, more preferably with x = 4, n= 2. In one embodiment said peptide linker is (G₄S)₂. The peptide linker is used to connect the first and the second Fab fragment. In one embodiment the first Fab fragment is connected to the C- or N- terminus of the second Fab fragment.

In one embodiment the first Fab fragment is connected to the N-terminus of the second Fab fragment. Depending on whether the variable or the constant domains of the heavy and the light chains of the second Fab fragment are exchanged, different bispecific antibody molecules are possible when the first Fab fragment is connected to the N-terminus of the second Fab fragment.

In one embodiment the variable domains of the second Fab fragment are exchanged (i.e. the second Fab fragment is a CrossFab _{(VHVL)}), and the C-terminus of the heavy or light chain of the first Fab fragment is connected to the N-terminus of the VLCH1 chain of the second Fab fragment. Preferably, the C-terminus heavy chain of the first Fab fragment is connected to the N-terminus of the VLCH1 chain of the second Fab fragment. Thus, in one embodiment the bispecific antibody comprises three chains: a light chain (VLCL) of the first Fab fragment, the heavy chain of the first Fab fragment connected to the VLCH1 chain of the second Fab fragment via a peptide linker (VHCH1-linker-VLCH1) and a VHCL chain of the second Fab fragment.

In another embodiment the constant domains of the second Fab fragment are exchanged (i.e the second Fab fragment is a CrossFab _{(CLCH1)}) and the C-terminus of the heavy or light chain of the first Fab fragment is connected to the N-terminus of the VHCL chain of the second Fab fragment. Preferably, the C-terminus of the heavy chain of the first Fab fragment is connected to the N-terminus of the VHCL chain of the second Fab fragment. Thus, in one embodiment the bispecific antibody comprises three chains: a light chain (VLCL) of the first Fab fragment, the heavy chain of the first Fab fragment connected to the VHCL chain of the second Fab fragment via a peptide linker (VHCH1-linker-VHCL) and a VLCH1 chain of the second Fab fragment.

In one embodiment the first Fab fragment is connected to the C-terminus of the second Fab fragment. Depending on whether the variable or the constant domains of the heavy and the light chains of the second Fab fragment are exchanged different bispecific antibody molecules are possible when the first Fab fragment is connected to the C-terminus of the second Fab fragment.

In one embodiment the variable domains of the second Fab fragment are exchanged (i.e. the second Fab fragment is a CrossFab _{(VHVL)}), and the CH1 domain of the second Fab fragment is connected to the N-terminus of the heavy or light chain of the first Fab fragment. Preferably, the CH1 domain of the second Fab fragment is connected to the N-terminus of the heavy chain of the first Fab fragment. Thus, in one embodiment the bispecific antibody comprises three chains: a light chain (VLCL) of the first Fab fragment, the VLCH1 chain of the second Fab fragment connected to the heavy chain of the first Fab fragment via a peptide linker (VLCH1-linker-VHCH1) and a VHCL chain of the second Fab fragment.

In another embodiment the constant domains of the second Fab fragment are exchanged (i.e. the second Fab fragment is a CrossFab _{(CLCH1)}), and the CL domain of the second Fab fragment is connected to the N-terminus of the heavy of light chain of the first Fab fragment. Preferably, the CL domain of the second Fab fragment is connected to the N-terminus of the heavy chain of the first Fab fragment. Thus, in one embodiment the bispecific antibody comprises three chains: a light chain (VLCL) of the first Fab fragment, the VHCL chain of the second Fab fragment connected to the heavy chain of the first Fab fragment via a peptide linker (VLCH1-linker-VHCH1) and a VLCH1 chain of the second Fab fragment.

The bispecific antibodies according to the invention are at least bivalent and can be trivalent or multivalent e.g. tetravalent. In one embodiment said bispecific antibodies are bivalent (1+1 format) with one binding site each targeting a first antigen and a second antigen, respectively. In another embodiment said bispecific antibodies are trivalent (2+1 format) with two binding sites each targeting a first antigen and one binding site targeting a second antigen, as detailed in the following section.

In one embodiment said antibody additionally comprises a third Fab fragment. In another embodiment said third Fab fragment comprises at least one antigen binding site specific for the first or second antigen, preferably for the first antigen.

In one embodiment the third Fab fragment is connected to the N or C-terminus of the first Fab fragment. In one embodiment the third Fab fragment is connected to the first Fab fragment via a peptide linker. Preferably said peptide linker is a (G4S)2 linker.

In one embodiment the third Fab fragment is connected to the N or C-terminus of the light chain or the heavy chain of the first Fab fragment. Depending on which terminus of the first Fab fragment is connected to the second Fab fragment (as detailed above), the third Fab fragment is connected on the opposite (free) terminus of the first fragment.

In one embodiment, the bispecific antibody of the invention comprises three Fab fragments wherein said Fab fragments and said linker are connected in the following order from N-terminal to C-terminal direction: Fab fragment 3- linker- Fab fragment 1- linker- Fab fragment 2, wherein either the variable regions or the constant regions of the heavy and light chain of the second Fab fragment are exchanged. In this embodiment the C-terminus of the third Fab fragment is connected to the N-terminus of the first Fab fragment. As detailed above, the Fab fragments can be connected to each other via the heavy or the light chains. In one embodiment the C-terminus of the heavy chain of the third Fab fragment is connected to the N-terminus of the heavy chain of the first Fab fragment via a peptide linker; and the C-terminus of the first Fab fragment is connected to the N-terminus of the second Fab fragment, wherein either the variable regions or the constant regions of the heavy and light chain of the second Fab fragment are exchanged. Depending on whether the variable or the constant domains of the heavy and the light chains of the second Fab fragment are exchanged different bispecific antibody molecules are possible.

In one embodiment the variable domains of the second Fab fragment are exchanged (i.e. the second Fab fragment is a CrossFab _{(VHVL)}), and the chains of the three Fab fragments are connected in the following order from N-terminal to C-terminal direction: VHCH1-linker-VHCH1-linker-VLCH1. In one embodiment the bispecific antibody comprises four chains: a light chain (VLCL) of the third Fab fragment, a light chain (VLCL) of the first Fab fragment, the heavy chain of the third fragment connected to the heavy chain of the first Fab fragment which itself is connected to the VLCH1 chain of the second Fab fragment via a peptide linker (VHCH1-linker-VHCH1-linker-VLCH1) and a VHCL chain of the second Fab fragment.

In one embodiment the constant domains of the second Fab fragment are exchanged (i.e. the second Fab fragment is a CrossFab _{(CLCH1)}), and the chains of the three Fab fragments are connected in the following order from N-terminal to C-terminal direction: VHCH1-linker-VHCH1-linker-VHCL. In one embodiment the bispecific antibody comprises four chains: a light chain (VLCL) of the third Fab fragment, a light chain (VLCL) of the first Fab fragment, the heavy chain of the third fragment connected to the heavy chain of the first Fab fragment which itself is connected to the VHCL chain of the second Fab fragment via a peptide linker (VHCH1-linker-VHCH1-linker-VHCL) and a VLCH1 chain of the second Fab fragment.

In one embodiment the bispecific antibody of the invention comprises three Fab fragments wherein said Fab fragments and said linker are connected in the following order from N-terminal to C-terminal direction: Fab fragment 2- linker- Fab fragment 1- linker- Fab fragment 3, wherein either the variable regions or the constant regions of the heavy and light chain of the second Fab fragment are exchanged. In this embodiment the N-terminus of the third Fab fragment is connected to the C-terminus of the first Fab fragment. As detailed above, the Fab fragments can be connected to each other via the heavy or the light chains. In one embodiment the N-terminus of the heavy chain of the third Fab fragment is connected to the C-terminus of the heavy chain of the first Fab fragment via a peptide linker; and the N-terminus of the first Fab fragment is connected to the C-terminus of the second Fab fragment, wherein either the variable regions or the constant regions of the heavy and light chain of the second Fab fragment are exchanged. Depending on whether the variable or the constant domains of the heavy and the light chains of the second Fab fragment are exchanged different bispecific antibody molecules are possible.

In one embodiment the variable domains of the second Fab fragment are exchanged (i.e. the second Fab fragment is a CrossFab _{(VHVL)}), and the chains of the three Fab fragments are connected in the following order from N-terminal to C-terminal direction: VLCH1-linker-VHCH1-linker-VHCH1. In one embodiment the bispecific antibody comprises four chains: a light chain (VLCL) of the third Fab fragment, a light chain (VLCL) of the first Fab fragment, the the VLCH1 chain of the second Fab fragment connected to the heavy chain of the first fragment which itself is connected to the heavy chain of the first Fab fragment via a peptide linker (VLCH1-linker-VHCH1-linker-VHCH1) and a VHCL chain of the second Fab fragment.

In one embodiment the constant domains of the second Fab fragment are exchanged (i.e. the second Fab fragment is a CrossFab _{(CLCH1)}), and the chains of the three Fab fragments are connected in the following order from N-terminal to C-terminal direction: VHCL-linker-VHCH1-linker-VHCH1. In one embodiment the bispecific antibody comprises four chains: a light chain (VLCL) of the third Fab fragment, a light chain (VLCL) of the first Fab fragment, the the VHCL chain of the second Fab fragment connected to the heavy chain of the first fragment which itself is connected to the heavy chain of the first Fab fragment via a peptide linker (VHCL-linker-VHCH1-linker-VHCH1) and a VLCH1 chain of the second Fab fragment.

In another embodiment the third Fab fragment is connected to N or C-terminus of the light chain or the heavy chain of the second Fab fragment. In one embodiment the third Fab fragment is connected to the second Fab fragment via a peptide linker. Preferably said peptide linker is a (G4S)2 linker. As detailed above, the Fab fragments can be connected to each other via the heavy or the light chains.

In one embodiment the bispecific antibody of the invention comprises three Fab fragments wherein said Fab fragments and said linker are connected in the following order from N-terminal to C-terminal direction: Fab fragment 1- linker- Fab fragment 2- linker- Fab fragment 3, wherein either the variable regions or the constant regions of the heavy and light chain of the second Fab fragment are exchanged. In one embodiment the N-terminus of the third Fab fragment is connected to the C-terminus of the second Fab fragment.

In another embodiment the C-terminus of the heavy chain of the third Fab fragment is connected to the N-terminus of the second Fab fragment via a peptide linker; and the N-terminus of the first Fab fragment is connected to the C-terminus of the second Fab fragment, wherein either the variable regions or the constant regions of the heavy and light chain of the second Fab fragment are exchanged.

Depending on whether the variable or the constant domains of the heavy and the light chains of the second Fab fragment are exchanged different bispecific antibody molecules are possible.

In one embodiment the variable domains of the second Fab fragment are exchanged (i.e. the second Fab fragment is a CrossFab _{(VHVL)}), and the chains of the three Fab fragments are connected in the following order from N-terminal to C-terminal direction:VHCH1-linker-VLCH1-linker-VHCH1. In one embodiment the bispecific antibody comprises four chains: a light chain (VLCL) of the third Fab fragment, a light chain (VLCL) of the first Fab fragment, the heavy chain of the third fragment connected to to the N-terminus of the VLCH1 chain of the second Fab fragment, and the C-terminus of said VLCH1 chain connected to the N-terminus of the heavy chain of the first Fab fragment via a peptide linker (VHCH1-linker-VLCH1-linker-VHCH1) and a VHCL chain of the second Fab fragment.

In one embodiment the constant domains of the second Fab fragment are exchanged (i.e. the second Fab fragment is a CrossFab _{(CLCH1)}), and the chains of the three Fab fragments are connected in the following order from N-terminal to C-terminal direction:VHCH1-linker-VHCL-linker-VHCH1. In one embodiment the bispecific antibody comprises four chains: a light chain (VLCL) of the third Fab fragment, a light chain (VLCL) of the first Fab fragment, the heavy chain of the third fragment connected to to the N-terminus of the VHCL chain of the second Fab fragment, and the C-terminus of said VHCL chain connected to the N-terminus of the heavy chain of the first Fab fragment via a peptide linker (VHCH1-linker-VHCL-linker-VHCH1) and a VLCH1 chain of the second Fab fragment.

In one embodiment of the invention the bispecific antibody is a humanized antibody, as detailed below.

In another embodiment of the invention the bispecific antibody is a human antibody, as detailed below.

In a second object the present invention relates to a pharmaceutical composition comprising a bispecific antibody of the present invention.

In a third object the present invention relates to a bispecific antibody of the present invention for the treatment of cancer. In another embodiment, use of the bispecific antibody as a medicament is provided. Preferably said use is for the treatment of cancer.

In further objects the present invention relates to a nucleic acid sequence comprising a sequence encoding a heavy chain of a bispecific antibody of the present invention, a nucleic acid sequence comprising a sequence encoding a light chain of a bispecific antibody of the present invention, an expression vector comprising a nucleic acid sequence of the present invention and to a prokaryotic or eukaryotic host cell comprising a vector of the present invention. In addition a method of producing an antibody comprising culturing the host cell so that the antibody is produced is provided.

In a further embodiment an immunoconjugate comprising the bispecific antibody of the invention and a cytotoxic agent is provided.

In a further aspect, a bispecific antibody according to any of the above embodiments may incorporate any of the features, singly or in combination, as described in Sections 1-5 below:

### 1. Antibody Affinity

The affinity of the bispecific antibody provided herein for a target antigen can be determined in accordance with the methods set forth in the Examples by surface plasmon resonance (SPR), using standard instrumentation such as a BIAcore instrument (GE Healthcare), and receptors or target proteins such as may be obtained by recombinant expression. Alternatively, binding of bispecific antibodies for different receptors or target antigens may be evaluated using cell lines expressing the particular receptor or target antigen, for example by flow cytometry (FACS).

In certain embodiments, a bispecific antibody provided herein has a dissociation constant (KD) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

According to one embodiment, KD is measured using surface plasmon resonance assays using a BIACORE®-2000 or a BIACORE®-3000 (BIAcore, Inc., Piscataway, NJ) at 25°C with immobilized antigen CM5 chips at ~10 response units (RU). Briefly, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (~0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 µl/min. Association rates (ka or kₒₙ) and dissociation rates (kd or k_{off}) are calculated using a simple one-to-one Langmuir binding model (BIACORE® Evaluation Software version 3.2) by simultaneously fitting the association and dissociation sensorgrams. The equilibrium dissociation constant (KD) is calculated as the ratio k_{off}/kₒₙ. See, e.g., Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

### 2. Chimeric and Humanized Antibodies

In certain embodiments, a bispecific antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 3. Human Antibodies

In certain embodiments, a bispecific antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HuMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 4. Library-Derived Antibodies

Bispecific ntibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 5. Antibody Variants

In certain embodiments, amino acid sequence variants of the bispecific antibodies provided herein are contemplated. For example, it may be desirable to improve the binding affinity and/or other biological properties of the bispecific antibody. Amino acid sequence variants of a bispecific antibody may be prepared by introducing appropriate modifications into the nucleotide sequence encoding the bispecific antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "conservative substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding or decreased immunogenicity.

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Cysteine engineered antibody variants

In certain embodiments, it may be desirable to create cysteine engineered bispecific antibodies, e.g., "thioMAbs," in which one or more residues of a bispecific antibody are substituted with cysteine residues. In particular embodiments, the substituted residues occur at accessible sites of the bispecific antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain and A118 (EU numbering) of the heavy chain. Cysteine engineered antibodies may be generated as described, e.g., in U.S. Patent No. 7,521,541.

### c) Antibody Derivatives

In certain embodiments, a bispecific antibody provided herein may be further modified to contain additional nonproteinaceous moieties that are known in the art and readily available. The moieties suitable for derivatization of the bispecific antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of a bispecific antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Bispecific antibodies of the invention may be obtained, for example, by solid-state peptide synthesis (e.g. Merrifield solid phase synthesis) or recombinant production. For recombinant production one or more polynucleotide encoding the bispecific anitbody (fragment), e.g., as described above, is isolated and inserted into one or more vectors for further cloning and/or expression in a host cell. Such polynucleotide may be readily isolated and sequenced using conventional procedures. In one embodiment a vector, preferably an expression vector, comprising one or more of the polynucleotides of the invention is provided. Methods which are well known to those skilled in the art can be used to construct expression vectors containing the coding sequence of a bispecific antibody (fragment) along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the bispecific antibody (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the bispecific antibody (fragment) of the invention, or variant or derivative thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoter inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the bispecific antigen binding molecule is desired, DNA encoding a signal sequence may be placed upstream of the nucleic acid encoding a bispecific antibody of the invention or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, *e.g.* an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the bispecific antibody may be included within or at the ends of the bispecific antibody (fragment) encoding polynucleotide.

In a further embodiment, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one such embodiment a host cell comprises (e.g. has been transformed or transfected with) a vector comprising a polynucleotide that encodes (part of) a bispecific antibody of the invention. As used herein, the term "host cell" refers to any kind of cellular system which can be engineered to generate the bispecific antibodies of the invention or fragments thereof. Host cells suitable for replicating and for supporting expression of bispecific antibodies are well known in the art. Such cells may be transfected or transduced as appropriate with the particular expression vector and large quantities of vector containing cells can be grown for seeding large scale fermenters to obtain sufficient quantities of the bispecific antibody for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr- CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an antigen binding domain such as an antibody, may be engineered so as to also express the other of the antibody chains such that the expressed product is an antibody that has both a heavy and a light chain.

In one embodiment, a method of producing a bispecific antibody according to the invention is provided, wherein the method comprises culturing a host cell comprising a polynucleotide encoding the bispecific antibody, as provided herein, under conditions suitable for expression of the bispecific antigen binding molecule, and recovering the bispecific antibody from the host cell (or host cell culture medium).

The components of the bispecific antibody are genetically fused to each other. bispecific antibody can be designed such that its components are fused directly to each other or indirectly through a linker sequence. The composition and length of the linker may be determined in accordance with methods well known in the art and may be tested for efficacy. Examples of linker sequences between different components of bispecific antibodies are found in the sequences provided herein. Additional sequences may also be included to incorporate a cleavage site to separate the individual components of the fusion if desired, for example an endopeptidase recognition sequence.

In certain embodiments the one or more antigen binding moieties of the bispecific antibodies comprise at least an antibody variable region capable of binding an antigenic determinant. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of antibody, antibody fragment, antigen binding domain or variable region can be used in the bispecific antibodies of the invention. Non-limiting antibodies, antibody fragments, antigen binding domains or variable regions useful in the present invention can be of murine, primate, or human origin. If the antibody is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain embodiments, the bispecific antibodies of the present invention are engineered to have enhanced binding affinity according to, for example, the methods disclosed in U.S. Pat. Appl. Publ. No. 2004/0132066. The ability of the bispecific antibody of the invention to bind to a specific antigenic determinant can be measured either through an enzyme-linked immunosorbent assay (ELISA) or other techniques familiar to one of skill in the art, e.g. surface plasmon resonance technique (analyzed on a BIACORE T100 system) (Liljeblad, et al., Glyco J 17, 323-329 (2000)), and traditional binding assays (Heeley, Endocr Res 28, 217-229 (2002)). Competition assays may be used to identify an antibody, antibody fragment, antigen binding domain or variable domain that competes with a reference antibody for binding to a particular antigen, e.g. an antibody that competes with the V9 antibody for binding to CD3. In certain embodiments, such a competing antibody binds to the same epitope (e.g. a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen (e.g. CD3) is incubated in a solution comprising a first labeled antibody that binds to the antigen (e.g. V9 antibody) and a second unlabeled antibody that is being tested for its ability to compete with the first antibody for binding to the antigen. The second antibody may be present in a hybridoma supernatant. As a control, immobilized antigen is incubated in a solution comprising the first
labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

Bispecific antibodies prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the bispecific antibody binds. For example, for affinity chromatography purification of bispecific antibody of the invention, a matrix with protein A or protein G may be used. Sequential Protein A or G affinity chromatography and size exclusion chromatography can be used to isolate a bispecific antibody essentially as described in the Examples. The purity of the bispecific antibodies can be determined by any of a variety of well known analytical methods including gel electrophoresis, high pressure liquid chromatography, and the like.

### C. Assays

Bispecific antibodies provided herein may be identified, screened for, or characterized for their physical/chemical properties and/or biological activities by various assays known in the art.

### 1. Binding assays and other assays

In one aspect, a bispecific antibody of the invention is tested for its antigen binding activity, e.g., by known methods such as ELISA, Western blot, etc.

In another aspect, competition assays may be used to identify an antibody that competes with a specific antibody for binding to the first or second antigen. In certain embodiments, such a competing antibody binds to the same epitope (e.g., a linear or a conformational epitope) that is bound by a specific antibody. Detailed exemplary methods for mapping an epitope to which an antibody binds are provided in Morris (1996) "Epitope Mapping Protocols," in Methods in Molecular Biology vol. 66 (Humana Press, Totowa, NJ).

### 2. Activity assays

In one aspect, assays are provided for identifying bispecific antibodies thereof having biological activity. Biological activity may include, e.g., lysis of targeted cells, or induction of apoptosis. Antibodies having such biological activity in vivo and/or in vitro are also provided.

In certain embodiments, a bispecific antibody of the invention is tested for such biological activity. Assays for detecting cell lysis (e.g. by measurement of LDH release) or apoptosis (e.g. using the TUNEL assay) are well known in the art.

### D. Immunoconjugates

The invention also provides immunoconjugates comprising a bispecific antibody of the invention conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises a bispecific antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises a bispecific antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example tc99m or I123, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

Conjugates of a bispecific antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### E. Methods and Compositions for Diagnostics and Detection

In certain embodiments, any of the bispecific antibodies provided herein is useful for detecting the presence of a first and / or a second antigenin a biological sample. The term "detecting" as used herein encompasses quantitative or qualitative detection. In certain embodiments, a biological sample comprises a cell or tissue.

In one embodiment, a bispecific antibody for use in a method of diagnosis or detection is provided. In a further aspect, a method of detecting the presence of a first and / or a second antigen in a biological sample is provided. In certain embodiments, the method comprises contacting the biological sample with a bispecific antibody as described herein under conditions permissive for binding of the bispecific antibody to a first and / or a second antigen , and detecting whether a complex is formed between the bispecific antibody a first and / or a second antigen. Such method may be an *in vitro* or *in vivo* method. In one embodiment, a bispecific antibody is used to select subjects eligible for therapy with a bispecific antibody, e.g. where a first and / or a second antigen is a biomarker for selection of patients.

Exemplary disorders that may be diagnosed using an antibody of the invention include cancer.

In certain embodiments, labeled bispecific antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### F. Pharmaceutical Formulations

Pharmaceutical formulations of a bispecific antibody as described herein are prepared by mixing such bispecific antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

### G. Therapeutic Methods and Compositions

Any of the bispecific antibodies provided herein may be used in therapeutic methods.

In one aspect, a bispecific antibody for use as a medicament is provided. In further aspects, a bispecific antibody for use in treating cancer is provided. In certain embodiments, a bispecific antibody for use in a method of treatment is provided. In certain embodiments, the invention provides a bispecific antibody for use in a method of treating an individual having cancer comprising administering to the individual an effective amount of the bispecific antibody . In one such embodiment, the method further comprises administering to the individual an effective amount of at least one additional therapeutic agent, e.g., as described below. An "individual" according to any of the above embodiments is preferably a human.

In a further aspect, the invention provides for the use of a bispecific antibody in the manufacture or preparation of a medicament. In one embodiment, the medicament is for treatment of cancer. In a further embodiment, the medicament is for use in a method of treating cancer comprising administering to an individual having cancer an effective amount of the medicament. In one such embodiment, the method further comprises administering to the individual an effective amount of at least one additional therapeutic agent, e.g., as described below. An "individual" according to any of the above embodiments may be a human.

In a further aspect, the invention provides a method for treating cancer. In one embodiment, the method comprises administering to an individual having cancer an effective amount of a bispecific antibody. In one such embodiment, the method further comprises administering to the individual an effective amount of at least one additional therapeutic agent, as described below. An "individual" according to any of the above embodiments may be a human.

In a further aspect, the invention provides pharmaceutical formulations comprising any of the bispecific antibodies provided herein, e.g., for use in any of the above therapeutic methods. In one embodiment, a pharmaceutical formulation comprises any of the bispecific antibody provided herein and a pharmaceutically acceptable carrier. In another embodiment, a pharmaceutical formulation comprises any of the bispecific antibodies provided herein and at least one additional therapeutic agent, e.g., as described below.

The bispecific antibodies of the invention can be used either alone or in combination with other agents in a therapy. For instance, a bispecific antibody of the invention may be co-administered with at least one additional therapeutic agent.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent and/or adjuvant. Bispecific antibodies of the invention can also be used in combination with radiation therapy.

A bispecific antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Bispecific antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The bispecific antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of a bispecific antibody of the invention (when used alone or in combination with one or more other additional therapeutic agents) will depend on the type of disease to be treated, the type of antibody, the severity and course of the disease, whether the bispecific antibody is administered for preventive or therapeutic purposes, previous therapy, the patient's clinical history and response to the bispecific antibody, and the discretion of the attending physician. The antibody is suitably administered to the patient at one time or over a series of treatments. Depending on the type and severity of the disease, about 1 µg/kg to 15 mg/kg (e.g. 0.1mg/kg-10mg/kg) of bispecific antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the bispecific antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g.* such that the patient receives from about two to about twenty, or *e.g.* about six doses of the bispecific antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to a bispecific antibody of the invention.

### H. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a bispecific antibody of the invention. The label or package insert indicates that the composition is used for treating the condition of choice. Moreover, the article of manufacture may comprise (a) a first container with a composition contained therein, wherein the composition comprises a bispecific antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to a bispecific antibody of the invention.

### III. EXAMPLES

The following are examples of methods and compositions of the invention. It is understood that various other embodiments may be practiced, given the general description provided above.

Although the foregoing invention has been described in some detail by way of illustration and example for purposes of clarity of understanding, the descriptions and examples should not be construed as limiting the scope of the invention.

### Example 1: Preparation of Fab (MCSP)-CrossFab(CD3)

The resulting variable region of heavy and light chain DNA sequences have been subcloned in frame with either the constant heavy chain or the constant light chain pre-inserted into the respective recipient mammalian expression vector. The antibody expression is driven by an MPSV promoter and carries a synthetic polyA signal sequence at the 3' end of the CDS. In addition each vector contains an EBV OriP sequence.

The molecule is produced by co-transfecting HEK293-EBNA cells with the mammalian expression vectors using a calcium phosphate-transfection. Exponentially growing HEK293-EBNA cells are transfected by the calcium phosphate method. Alternatively, HEK293-EBNA cells growing in suspension are transfected by polyethylenimine. The cells are transfected with the corresponding expression vectors in a 1:1:1 ratio ("vector CH1-VH - CK-VH" : "vector light chain" : "vector light chain CH1-VL").

For transfection using calcium phosphate cells are grown as adherent monolayer cultures in T-flasks using DMEM culture medium supplemented with 10 % (v/v) FCS, and are transfected when they are between 50 and 80 % confluent. For the transfection of a T150 flask, 15 million cells are seeded 24 hours before transfection in 25 ml DMEM culture medium supplemented with FCS (at 10% v/v final), and cells are placed at 37 °C in an incubator with a 5 % CO2 atmosphere overnight. For each T150 flask to be transfected, a solution of DNA, CaC12 and water is prepared by mixing 94 µg total plasmid vector DNA divided in the corresponding ratio, water to a final volume of 469 µl and 469 µl of a 1 M CaC12 solution. To this solution, 938 µl of a 50 mM HEPES, 280 mM NaCl, 1.5 mM Na2HPO4 solution at pH 7.05 are added, mixed immediately for 10 s and left to stand at room temperature for 20 s. The suspension is diluted with 10 ml of DMEM supplemented with 2 % (v/v) FCS, and added to the T150 in place of the existing medium. Then additional 13 ml of transfection medium are added. The cells are incubated at 37 °C, 5 % CO2 for about 17 to 20 hours, then medium is replaced with 25 ml DMEM, 10 % FCS. The conditioned culture medium is harvested approx. 7 days post-media exchange by centrifugation for 15 min at 210 x g, the solution is sterile filtered (0.22 µm filter) and sodium azide in a final concentration of 0.01 % (w/v) is added, and kept at 4 °C.

For transfection using polyethylenimine HEK293 EBNA cells are cultivated in suspension serum free in CD CHO culture medium. For the production in 500 ml shake flask 400 million HEK293 EBNA cells are seeded 24 hours before transfection. For transfection cells are centrifuged for 5 min by 210 x g, supernatant is replaced by pre-warmed 20 ml CD CHO medium. Expression vectors are mixed in 20 ml CD CHO medium to a final amount of 200 µg DNA. After addition of 540 µl PEI solution is vortexed for 15 s and subsequently incubated for 10 min at room temperature. Afterwards cells are mixed with the DNA/PEI solution, transferred to a 500 ml shake flask and incubated for 3 hours by 37 °C in an incubator with a 5 % CO2 atmosphere. After incubation time 160 ml F17 medium is added and cell are cultivated for 24 hours. One day after transfection 1 mM valporic acid and 7 % Feed 1 (Lonza) is added. After 7 days cultivation supernatant is collected for purification by centrifugation for 15 min at 210 x g, the solution is sterile filtered (0.22 µm filter) and sodium azide in a final concentration of 0.01 % w/v is added, and kept at 4 °C.

The secreted protein is purified from cell culture supernatants by affinity chromatography using Protein A and Protein G affinity chromatography, followed by a size exclusion chromatographic step. For affinity chromatography supernatant is loaded on a HiTrap Protein A HP column (CV = 5 ml, GE Healthcare) coupled to a HiTrap Protein G HP column (CV = 5 ml, GE Healthcare) each column equilibrated with 30 ml 20 mM sodium phosphate, 20 mM sodium citrate, pH 7.5. Unbound protein is removed by washing both columns with 6 column volume 20 mM sodium phosphate, 20 mM sodium citrate, pH 7.5. Subsequently an additional wash step is necessary to wash only the HiTrap Protein G HP column using at least 8 column volume 20 mM sodium phosphate, 20 mM sodium citrate, pH 7.5. The target protein is eluted from HiTrap Protein G HP column using a step gradient with 7 column volume 8.8 mM formic acid, pH 3.0. Protein solution is neutralized by adding 1/10 of 0.5 M sodium phosphate, pH 8.0. Target protein is concentrated and filtrated prior loading on a HiLoad Superdex 200 column (GE Healthcare) equilibrated with 25 mM potassium phosphate, 125 mM sodium chloride, 100 mM glycine solution of pH 6.7.

The protein concentration of purified protein samples is determined by measuring the optical density (OD) at 280 nm, using the molar extinction coefficient calculated on the basis of the amino acid sequence. Purity and molecular weight of antibodies are analyzed by SDS-PAGE in the presence and absence of a reducing agent (5 mM 1,4-dithiotreitol) and staining with Coomassie (SimpleBlue™ SafeStain from Invitrogen). The NuPAGE® Pre-Cast gel system (Invitrogen, USA) is used according to the manufacturer's instruction (4-12 % Tris-Acetate gels or 4-12 % Bis-Tris). The aggregate content of antibody samples is analyzed using a Superdex 200 10/300GL analytical size-exclusion column (GE Healthcare, Sweden) in 2 mM MOPS, 150 mM NaCl, 0.02 % (w/v) NaN3, pH 7.3 running buffer at 25°C.

Analysis of production and purification of an exemplary Fab-Crossfab molecule (consisting of three chains: VHCH1(MCSP)-VLCH1(CD3_{V9}) = SEQ ID NO:25, VLCL(MCSP) = SEQ ID NO:17 and VHCL(CD3_{V9}) = SEQ ID NO:23; with an orientation as depicted in Figure 1 a)) is shown in Figures 2 and 3. This molecule is further referred to as Fab (MCSP)-Crossfab (CD3) or hu Fab (MCSP)-Crossfab (CD3).

### Example 2: Preparation of Fab (MCSP)- Fab (MCSP)-CrossFab(CD3) and Fab (MCSP)- CrossFab(CD3)- Fab (MCSP)

The resulting variable region of heavy and light chain DNA sequences have been subcloned in frame with either the constant heavy chain or the constant light chain pre-inserted into the respective recipient mammalian expression vector. The antibody expression is driven by an MPSV promoter and carries a synthetic polyA signal sequence at the 3' end of the CDS. In addition each vector contains an EBV OriP sequence.

The molecule is produced by co-transfecting HEK293-EBNA cells with the mammalian expression vectors using a calcium phosphate-transfection. Exponentially growing HEK293-EBNA cells are transfected by the calcium phosphate method. Alternatively, HEK293-EBNA cells growing in suspension are transfected by polyethylenimine. The cells are transfected with the corresponding expression vectors in a 1:2:1 ratio ("vector CH1-VH - CH1-VH - CK-VH" : "vector light chain" : "vector light chain CH1-VL").

For transfection using calcium phosphate cells are grown as adherent monolayer cultures in T-flasks using DMEM culture medium supplemented with 10 % (v/v) FCS, and are transfected when they are between 50 and 80 % confluent. For the transfection of a T150 flask, 15 million cells are seeded 24 hours before transfection in 25 ml DMEM culture medium supplemented with FCS (at 10% v/v final), and cells are placed at 37 °C in an incubator with a 5 % CO2 atmosphere overnight. For each T150 flask to be transfected, a solution of DNA, CaC12 and water is prepared by mixing 94 µg total plasmid vector DNA divided in the corresponding ratio, water to a final volume of 469 µl and 469 µl of a 1 M CaC12 solution. To this solution, 938 µl of a 50 mM HEPES, 280 mM NaCl, 1.5 mM Na2HPO4 solution at pH 7.05 are added, mixed immediately for 10 s and left to stand at room temperature for 20 s. The suspension is diluted with 10 ml of DMEM supplemented with 2 % (v/v) FCS, and added to the T150 in place of the existing medium. Then additional 13 ml of transfection medium are added. The cells are incubated at 37 °C, 5 % CO2 for about 17 to 20 hours, then medium is replaced with 25 ml DMEM, 10 % FCS. The conditioned culture medium is harvested approx. 7 days post-media exchange by centrifugation for 15 min at 210 x g, the solution is sterile filtered (0.22 µm filter) and sodium azide in a final concentration of 0.01 % (w/v) is added, and kept at 4 °C. For transfection using polyethylenimine HEK293 EBNA cells are cultivated in suspension serum free in CD CHO culture medium. For the production in 500 ml shake flask 400 million HEK293 EBNA cells are seeded 24 hours before transfection. For transfection cells are centrifuged for 5 min by 210 x g, supernatant is replaced by pre-warmed 20 ml CD CHO medium. Expression vectors are mixed in 20 ml CD CHO medium to a final amount of 200 µg DNA. After addition of 540 µl PEI solution is vortexed for 15 s and subsequently incubated for 10 min at room temperature. Afterwards cells are mixed with the DNA/PEI solution, transferred to a 500 ml shake flask and incubated for 3 hours by 37 °C in an incubator with a 5 % CO2 atmosphere. After incubation time 160 ml F17 medium is added and cell are cultivated for 24 hours. One day after transfection 1 mM valporic acid and 7 % Feed 1 (Lonza) is added. After 7 days cultivation supernatant is collected for purification by centrifugation for 15 min at 210 x g, the solution is sterile filtered (0.22 µm filter) and sodium azide in a final concentration of 0.01 % w/v is added and kept at 4 °C.

The secreted protein is purified from cell culture supernatants by affinity chromatography using Protein A and Protein G affinity chromatography, followed by a size exclusion chromatographic step.

For affinity chromatography supernatant is loaded on a HiTrap Protein A HP column (CV = 5 ml, GE Healthcare) coupled to a HiTrap Protein G HP column (CV = 5 ml, GE Healthcare) each column equilibrated with 30 ml 20 mM sodium phosphate, 20 mM sodium citrate, pH 7.5. Unbound protein is removed by washing both columns with 6 column volume 20 mM sodium phosphate, 20 mM sodium citrate, pH 7.5. Subsequently an additional wash step is necessary to wash only the HiTrap Protein G HP column using at least 8 column volume 20 mM sodium phosphate, 20 mM sodium citrate, pH 7.5. The target protein is eluted from HiTrap Protein G HP column using a step gradient with 7 column volume 8.8 mM formic acid, pH 3.0. Protein solution is neutralized by adding 1/10 of 0.5 M sodium phosphate, pH 8.0. Target protein is concentrated and filtrated prior loading on a HiLoad Superdex 200 column (GE Healthcare) equilibrated with 25 mM potassium phosphate, 125 mM sodium chloride, 100 mM glycine solution of pH 6.7.

The protein concentration of purified protein samples is determined by measuring the optical density (OD) at 280 nm, using the molar extinction coefficient calculated on the basis of the amino acid sequence. Purity and molecular weight of antibodies are analyzed by SDS-PAGE in the presence and absence of a reducing agent (5 mM 1,4-dithiotreitol) and staining with Coomassie (SimpleBlue™ SafeStain from Invitrogen). The NuPAGE® Pre-Cast gel system (Invitrogen, USA) is used according to the manufacturer's instruction (4-12 % Tris-Acetate gels or 4-12 % Bis-Tris). The aggregate content of antibody samples is analyzed using a Superdex 200 10/300GL analytical size-exclusion column (GE Healthcare, Sweden) in 2 mM MOPS, 150 mM NaCl, 0.02 % (w/v) NaN3, pH 7.3 running buffer at 25°C and compared with prior art antibody fragment (scFv)2 (results see table below).

**HMW = High Molecular Weight; LMW = Low Molecular Weight**

| Construct | Yield [mg/l] | Aggregate after 1^{st} purification step [%] | HMW [%] | LMW [%] | Monomer [%] |
|---|---|---|---|---|---|
| (scFv)2 | 3.84 | 80 | 0 | 0 | 100 |
| Fab-Crossfab | 7.85 | 13.8 | 0 | 0 | 100 |
| (Fab)2-Crossfab | 7.8 | 3.6 | 0 | 0 | 100 |
| Fab-Crossfab-Fab | 5.3 | 1.7 | 0.4 | 0 | 99.56 |

Analysis of production and purification of an exemplary Fab-Fab-Crossfab molecule (consisting of four chains: VHCH1(MCSP)-VHCH1(MCSP)-VLCH1(CD3_{V9}) = SEQ ID NO:26, 2 VLCL(MCSP) chains = SEQ ID NO: 17 and one VHCL(CD3_{V9}) chain = SEQ ID NO:23; with an orientation as depicted in Figure 1 c)) is shown in Figures 4 and 5. This molecule is further referred to as Fab (MCSP)- Fab (MCSP)-Crossfab (CD3) or hu Fab (MCSP)- Fab (MCSP)-Crossfab (CD3).

Analysis of production and purification of an exemplary Fab-Crossfab-Fab molecule (consisting of four chains: VHCH1(MCSP)-VLCH1(CD3_{V9})- VHCH1(MCSP)= SEQ ID NO:27, 2 VLCL(MCSP) chains = SEQ ID NO: 17 and one VHCL(CD3_{V9}) chain= SEQ ID NO:23; with an orientation as depicted in Figure 1 e)) is shown in Figures 6 and 7. This molecule is further referred to as Fab (MCSP)- Fab (MCSP)-Crossfab (CD3) or hu Fab (MCSP)- Fab (MCSP)-Crossfab (CD3).

Analysis of production and purification of an exemplary Crossfab-Fab-Fab molecule (consisting of four chains: VLCH1(CD3_{2C11})- VHCH1(MCSP)- VHCH1(MCSP)= SEQ ID NO:42, 2 VLCL(MCSP) chains = SEQ ID NO: 17 and one VHCL(CD3_{2C11}) chain = SEQ ID NO:43; with an orientation as depicted in Figure 1 d)) is shown in Figures 8 and 9. This molecule is further referred to as murine Crossfab (CD3)-Fab (MCSP)- Fab (MCSP).

### Example 3: Preparation of Fab(CD33)-CrossFab (CD3)

The resulting variable region of heavy and light chain DNA sequences have been subcloned in frame with either the constant heavy chain or the constant light chain pre-inserted into the respective recipient mammalian expression vector. The antibody expression is driven by an MPSV promoter and carries a synthetic polyA signal sequence at the 3' end of the CDS. In addition each vector contains an EBV OriP sequence.

The molecule is produced by co-transfecting HEK293-EBNA cells with the mammalian expression vectors using a calcium phosphate-transfection. Exponentially growing HEK293-EBNA cells are transfected by the calcium phosphate method. Alternatively, HEK293-EBNA cells growing in suspension are transfected by polyethylenimine. The cells are transfected with the corresponding expression vectors in a 1:1:1 ratio ("vector CH1-VH - CK-VH" : "vector light chain" : "vector light chain CH1-VL").

For transfection using calcium phosphate cells are grown as adherent monolayer cultures in T-flasks using DMEM culture medium supplemented with 10 % (v/v) FCS, and are transfected when they are between 50 and 80 % confluent. For the transfection of a T150 flask, 15 million cells are seeded 24 hours before transfection in 25 ml DMEM culture medium supplemented with FCS (at 10% v/v final), and cells are placed at 37 °C in an incubator with a 5 % CO2 atmosphere overnight. For each T150 flask to be transfected, a solution of DNA, CaC12 and water is prepared by mixing 94 µg total plasmid vector DNA divided in the corresponding ratio, water to a final volume of 469 µl and 469 µl of a 1 M CaC12 solution. To this solution, 938 µl of a 50 mM HEPES, 280 mM NaCl, 1.5 mM Na2HPO4 solution at pH 7.05 are added, mixed immediately for 10 s and left to stand at room temperature for 20 s. The suspension is diluted with 10 ml of DMEM supplemented with 2 % (v/v) FCS, and added to the T150 in place of the existing medium. Then additional 13 ml of transfection medium are added. The cells are incubated at 37 °C, 5 % CO2 for about 17 to 20 hours, then medium is replaced with 25 ml DMEM, 10 % FCS. The conditioned culture medium is harvested approx. 7 days post-media exchange by centrifugation for 15 min at 210 x g, the solution is sterile filtered (0.22 µm filter) and sodium azide in a final concentration of 0.01 % (w/v) is added, and kept at 4 °C.

For transfection using polyethylenimine HEK293 EBNA cells are cultivated in suspension serum free in CD CHO culture medium. For the production in 500 ml shake flask 400 million HEK293 EBNA cells are seeded 24 hours before transfection. For transfection cells are centrifuged for 5 min by 210 x g, supernatant is replaced by pre-warmed 20 ml CD CHO medium. Expression vectors are mixed in 20 ml CD CHO medium to a final amount of 200 µg DNA. After addition of 540 µl PEI solution is vortexed for 15 s and subsequently incubated for 10 min at room temperature. Afterwards cells are mixed with the DNA/PEI solution, transferred to a 500 ml shake flask and incubated for 3 hours by 37 °C in an incubator with a 5 % CO2 atmosphere. After incubation time 160 ml F17 medium is added and cell are cultivated for 24 hours. One day after transfection 1 mM valporic acid and 7 % Feed 1 (LONZA) is added. After 7 days cultivation supernatant is collected for purification by centrifugation for 15 min at 210 x g, the solution is sterile filtered (0.22 µm filter) and sodium azide in a final concentration of 0.01 % w/v is added, and kept at 4°C.

The secreted protein is purified from cell culture supernatants by affinity chromatography using Protein A and ProteinG affinity chromatography, followed by a size exclusion chromatographic step.

For affinity chromatography supernatant is loaded on a HiTrap ProteinA HP column (CV=5 mL, GE Healthcare) coupled to a HiTrap ProteinG HP column (CV=5 mL, GE Healthcare) each column equilibrated with 30 ml 20 mM sodium phosphate, 20 mM sodium citrate, pH 7.5. Unbound protein is removed by washing both columns with 6 column volume 20 mM sodium phosphate, 20 mM sodium citrate, pH 7.5. Subsequently an additional wash step is necessary to wash only the HiTrap ProteinG HP column using at least 8 column volume 20 mM sodium phosphate, 20 mM sodium citrate, pH 7.5. The target protein is eluted from HiTrap ProteinG HP column using a step gradient with 7 column volume 8.8 mM formic acid, pH 3.0. Protein solution is neutralized by adding 1/10 of 0.5M sodium phosphate, pH 8.0. Target protein is concentrated and filtrated prior loading on a HiLoad Superdex 200 column (GE Healthcare) equilibrated with 25 mM potassium phosphate, 125 mM sodium chloride, 100 mM glycine solution of pH 6.7.

The protein concentration of purified protein samples is determined by measuring the optical density (OD) at 280 nm, using the molar extinction coefficient calculated on the basis of the amino acid sequence. Purity and molecular weight of antibodies are analyzed by SDS-PAGE in the presence and absence of a reducing agent (5 mM 1,4-dithiotreitol) and staining with Coomassie (SimpleBlue™ SafeStain from Invitrogen). The NuPAGE® Pre-Cast gel system (Invitrogen, USA) is used according to the manufacturer's instruction (4-12% Tris-Acetate gels or 4-12% Bis-Tris). The aggregate content of antibody samples is analyzed using a Superdex 200 10/300GL analytical size-exclusion column (GE Healthcare, Sweden) in 2 mM MOPS, 150 mM NaCl, 0.02 % (w/v) NaN3, pH 7.3 running buffer at 25°C.

Analysis of production and purification of an exemplary Fab-Crossfab molecule (consisting of three chains: VHCH1(CD33)- VLCH1(CD3_{V9}) = SEQ ID NO:102, VLCL(CD33) = SEQ ID NO:100 and VHCL(CD3_{V9}) = SEQ ID NO:23 or SEQ ID NO:101; with an orientation as depicted in Figure 1 a)) is shown in Figures 17 and 18. This molecule is further referred to as Fab(CD33)-CrossFab (CD3) or hu Fab(CD33)-CrossFab (CD3).

### Example 4: Preparation of the reference molecule (scFv)2

### Cloning and production

The resulting variable region of heavy and light chain DNA sequences have been subcloned in frame into the respective recipient mammalian expression vector. The antibody expression is driven by an MPSV promoter and carries a synthetic polyA signal sequence at the 3' end of the CDS. In addition each vector contains an EBV OriP sequence.

The molecule is produced by transfecting HEK293-EBNA cells with the mammalian expression vector using polyethylenimine. HEK293 EBNA cells are cultivated in suspension serum free in CD CHO culture medium. For the production in 500 ml shake flask 400 million HEK293 EBNA cells are seeded 24 hours before transfection. For transfection cells are centrifuged for 5 min by 210 x g, supernatant is replaced by pre-warmed 20 ml CD CHO medium. Expression vectors are mixed in 20 ml CD CHO medium to a final amount of 200 µg DNA. After addition of 540 µl PEI solution is vortexed for 15 s and subsequently incubated for 10 min at room temperature. Afterwards cells are mixed with the DNA/PEI solution, transferred to a 500 ml shake flask and incubated for 3 hours by 37 °C in an incubator with a 5 % CO2 atmosphere. After incubation time 160 ml F17 medium is added and cell are cultivated for 24 hours. One day after transfection 1 mM valporic acid and 7 % Feed 1 (LONZA) are added. After 7 days cultivation supernatant is collected for purification by centrifugation for 15 min at 210 x g, the solution is sterile filtered (0.22 µm filter) and sodium azide in a final concentration of 0.01 % w/v is added, and kept at 4°C.

### Purification of (scFv)2 (anti MCSP/anti huCD3)

The secreted protein is purified from cell culture supernatants by affinity chromatography using Immobilized Metal Ion Affinity Chromatography (IMAC), followed by a size exclusion chromatographic step.

Prior first purification step disturbing components from the supernatant are removed by diafiltration using the tangential flow filtration system Sarcojet (Sartorius) equipped with a 5.000 MWCO membrane (Sartocon Slice Cassette, Hydrosart; Sartorius). Supernatant is concentrated to 210 ml and subsequently diluted in 11 20 mM sodium phosphate, 500 mM sodium chloride, pH 6.5. The protein solution is concentrated again to 210 ml. This process is repeated twice to ensure a complete buffer exchange.

For affinity chromatography retentate of the diafiltration process is loaded on a NiNTA Superflow Cartridge (CV=5 mL, Qiagen) equilibrated with 25 ml 20 mM sodium phosphate, 500 mM sodium chloride, 15 mM imidazole, pH 6.5. Unbound protein is removed by washing with at least 2 column volume 20 mM sodium phosphate, 500 mM sodium chloride, 15 mM imidazole, pH 6.5 followed by an additional wash step using 3 column volume 20 mM sodium phosphate, 500 mM sodium chloride, 62.5 mM imidazole, pH 6.5. Target protein is eluted in 2 column volume 20 mM sodium phosphate, 500 mM sodium chloride, 125 mM imidazole, pH 6.5. Column is washed subsequently with 20 mM sodium phosphate, 500 mM sodium chloride, 250 mM imidazole, pH 6.5.

Target protein is concentrated prior loading on a HiLoad Superdex 75 column (GE Healthcare) equilibrated with 25 mM KH₂PO₄, 12 mM NaCl, 200 mM Arginine, pH 6.7. Yields, aggregate content after the first purification step and final monomer content is shown in the table above. Comparison of the aggregate content after the first purification step indicates the superior stability of the Fab-Crossfab construct in contrast to the (scFv)2.

### Characterization of (scFv)2

The protein concentration of purified protein samples is determined by measuring the optical density (OD) at 280 nm, using the molar extinction coefficient calculated on the basis of the amino acid sequence. Purity and molecular weight of antibodies are analyzed by SDS-PAGE in the presence and absence of a reducing agent (5 mM 1,4-dithiotreitol) and staining with Coomassie (SimpleBlue™ SafeStain from Invitrogen). The NuPAGE® Pre-Cast gel system (Invitrogen, USA) is used according to the manufacturer's instruction (4-12% Tris-Acetate gels or 4-12% Bis-Tris). The aggregate content of antibody samples is analyzed using a Superdex 75 10/300GL analytical size-exclusion column (GE Healthcare, Sweden) in 2 mM MOPS, 150 mM NaCl, 0.02 % (w/v) NaN3, pH 7.3 running buffer at 25°C.

A schematic drawing of the (scFv)2 molecule is shown in Figure 21.

Analysis of production and purification of an exemplary (scFv)2 molecule (antiMCSP/anti huCD3; consisting two single chain Fvs: VL-VH (MCSP) and VH-VL (CD3_{V9}) = SEQ ID NO:149; is shown in Figures 22 and 23. This molecule is further referred to as (scFv)2 (antiMCSP/anti huCD3e).

### Example 5: Isolation of primary human pan T cells from PBMCs

Peripheral blood mononuclar cells (PBMCs) were prepared by Histopaque density centrifugation from enriched lymphocyte preparations (buffy coats) obtained from local blood banks or from fresh blood from healthy human donors.

T-cell enrichment from PBMCs was performed using the Pan T Cell Isolation Kit II (Miltenyi Biotec #130-091-156), according to the manufacturer's instructions. Briefly, the cell pellets were diluted in 40µl cold buffer per 10 Mio cells (PBS with 0.5 % BSA, 2 mM EDTA - sterile filtered) and incubated with 10µl Biotin-Antibody Cocktail per 10 Mio cells for 10 min at 4 °C.

30µl cold buffer and 20µl Anti-Biotin magnetic beads per 10 Mio cells were added, and the mixture incubated for another 15 min at 4 °C.

Cells were washed by adding 10-20x of labeling volume and a subsequent centrifugation step at 300g for 10 min. Up to 100 Mio cells were resuspended in 500 µl buffer.

Magnetic separation of unlabeled human pan T cells was performed using LS columns (Miltenyi Biotec #130-042-401) according to the manufacturer's instructions. The resulting T cell population was counted automatically (ViCell) and stored in AIM-V medium at 37°C, 5 % CO2 in the incubator until assay start (not longer than 24 h).

### Example 6: Isolation of murine pan T cells from splenocytes

Spleens were isolated from C57BL/6 mice, transferred into a GentleMACS C-tube (Miltenyi Biotech #130-093-237) containing MACS buffer (PBS + 0.5 % BSA + 2 mM EDTA) and dissociated with the GentleMACS Dissociator to obtain single-cell suspensions according to the manufacturers' instructions.

The cell suspension was passed through a pre-separation filter to get rid-off remaining undissociated tissue particles. After centrifugation at 400 g for 4 minutes at 4°C, ACK Lysis Buffer was added to to lyse red blood cells (incubation for 5 minutes at room temperature). The remaining cells were washed with MACS buffer twice, counted and used for the isolation of murine pan T cells. The negative (magnetic) selection was performed using the Pan T Cell Isolation Kit from Miltenyi Biotec (#130-090-861), following the manufacturers' instructions. The resulting T cell population was counted automatically (ViCell) and used immediately for further assays.

### Example 7: Re-directed T cell cytotoxicity mediated by cross-linked bispecific constructs targeting CD3 on T cells and MCSP on tumor cells (LDH release assay)

Bispecific constructs targeting CD3 on human, or mouse T cells and human on tumor cells, are analyzed by a LDH release assay regarding their potential to induce T cell-mediated apoptosis of target cells.

Briefly, target cells (human Colo-38, human MDA-MB-435, human melanoma MV-3 or murine B16/F10-huMCSP Fluc 2 clone 48 cells, all expressing human MCSP) are harvested with Cell Dissociation Buffer (MCSP is trypsin-sensitive) or trypsin (and then plated the day before), washed and resuspendend in the appropriate cell culture medium (see detailed description of the different figures). 20 000 - 30 000 cells per well are plated in a round-bottom 96-well-plate and the respective antibody dilution was added as indicated (triplicates). Effector cells were added to obtain a final E:T ratio of 5:1 (for human pan T cells), 10:1 (for human PBMCs).

In addition, 1-10 µg/ml PHA-M (Sigma #L8902), a mixture of isolectins, isolated from Phaseolus vulgaris, was used as a mitogenic stimulus to induce human or cynomolgus T cell activation. For murine T cells, a 5 % solution of "rat T-Stim with ConA" (BD #354115) was used as a positive control for T cell activation.

For normalization, maximal lysis of the target cells (= 100 %) is achieved by incubation of the target cells with a final concentration of 1 % Triton-X-100. Minimal lysis (= 0 %) refers to target cells co-incubated with effector cells, but without any construct or antibody.

After an overnight incubation of at least 18 h at 37°C, 5 % CO2, LDH release of apoptotic/necrotic target cells into the supernatant is measured with the LDH detection kit (Roche Applied Science, # 11 644 793 001), according to the manufacturer's instructions.

### LDH release assay with Fab (MCSP)-Crossfab (CD3) and Fab (MCSP)- Fab (MCSP)-Crossfab (CD3) bispecific constructs

Purified Fab (MCSP)-Crossfab (CD3), Fab (MCSP)- Fab (MCSP)-Crossfab (CD3) and the (scFv)2 (antiMCSP/anti huCD3e) reference molecule were analyzed for their potential to induce T cell-mediated apoptosis in tumor target cells upon crosslinkage of the construct via binding of both targeting moieties to the respective antigens on cells. Briefly, huMCSP-expressing MDA-MB-435 human melanoma target cells are harvested with Cell Dissociation Buffer, washed and resuspendend in AIM-V medium (Invitrogen # 12055-091). 30 000 cells per well were plated in a round-bottom 96-well-plate and the respective antibody dilution was added at the indicated concentrations. All constructs and controls were adjusted to the same molarity.

Human pan T effector cells were added to obtain a final E:T ratio of 5:1. As a positive control for the activation of human pan T cells, 1 µg/ml PHA-M (Sigma #L8902) was used. For normalization, maximal lysis of the target cells (= 100 %) was determined by incubation of the target cells with a final concentration of 1 % Triton-X-100. Minimal lysis (= 0 %) refers to target cells co-incubated with effector cells, but without any construct or antibody.

After an overnight incubation of 20 h at 37°C, 5 % CO2, LDH release of apoptotic/necrotic target cells into the supernatant was measured with the LDH detection kit (Roche Applied Science, # 11 644 793 001), according to the manufacturer's instructions.

As depicted in Figure 10, the constructs with bivalent MCSP-targeting show comparable cytotoxic activity compared to the (scFv)2 (antiMCSP/anti huCD3e) construct, whereas the Fab (MCSP)-Crossfab (CD3) construct with monovalent MCSP binding is clearly less potent.

### LDH release assay with Fab (MCSP)- Fab (MCSP)-Crossfab (CD3) bispecific construct with MDA-MB-435 human melanoma target cells

Purified Fab (MCSP)- Fab (MCSP)-Crossfab (CD3) and the (scFv)2 (antiMCSP/anti huCD3e) reference molecule were analyzed for their potential to induce T cell-mediated apoptosis in tumor target cells upon crosslinkage of the construct via binding of both targeting moieties to the respective antigens on cells.

Briefly, huMCSP-expressing MDA-MB-435 human melanoma target cells are harvested with Cell Dissociation Buffer, washed and resuspendend in AIM-V medium (Invitrogen # 12055-091). 30 000 cells per well were plated in a round-bottom 96-well-plate and the respective antibody dilution was added at the indicated concentrations. All constructs and controls were adjusted to the same molarity.

Human pan T effector cells were added to obtain a final E:T ratio of 5:1. As a positive control for the activation of human pan T cells, 5 µg/ml PHA-M (Sigma #L8902) was used. For normalization, maximal lysis of the target cells (= 100 %) was determined by incubation of the target cells with a final concentration of 1 % Triton-X-100. Minimal lysis (= 0 %) refers to target cells co-incubated with effector cells, but without any construct or antibody.

After an overnight incubation of 21 h at 37°C, 5 % CO2, LDH release of apoptotic/necrotic target cells into the supernatant was measured with the LDH detection kit (Roche Applied Science, # 11 644 793 001), according to the manufacturer's instructions.

As depicted in Figure 11, the Fab (MCSP)- Fab (MCSP)-Crossfab (CD3) induces apoptosis in target cells at least comparably good as the (scFv)2 (antiMCSP/anti huCD3e) molecule.

### LDH release assay with Fab (MCSP)- Fab (MCSP)-Crossfab (CD3) bispecific construct with MV-3 human melanoma target cells

Purified Fab (MCSP)- Fab (MCSP)-Crossfab (CD3) and the (scFv)2 (antiMCSP/anti huCD3e) molecule were analyzed for their potential to induce T cell-mediated apoptosis in tumor target cells upon crosslinkage of the construct via binding of both targeting moieties to the respective antigens on cells.

Briefly, huMCSP-expressing MV-3 human melanoma target cells are harvested with trypsin on the day before the LDH release assay was started. Cell were washed and resuspendend in the appropriate cell culture medium. 30 000 cells per well were plated in a round-bottom 96-well-plate. The next day, the supernatant was discarded and 100 µl/well AIM-V medium (Invitrogen # 12055-091), as well as the respective antibody dilution were added at the indicated concentrations. All constructs and controls were adjusted to the same molarity.

Human PBMC effector cells were added to obtain a final E:T ratio of 10:1. As a positive control for the activation of human pan T cells, 5 µg/ml PHA-M (Sigma #L8902) was used. For normalization, maximal lysis of the target cells (= 100 %) was determined by incubation of the target cells with a final concentration of 1 % Triton-X-100. Minimal lysis (= 0 %) refers to target cells co-incubated with effector cells, but without any construct or antibody.

After an overnight incubation of 26 h at 37°C, 5 % CO2, LDH release of apoptotic/necrotic target cells into the supernatant was measured with the LDH detection kit (Roche Applied Science, # 11 644 793 001), according to the manufacturer's instructions.

As depicted in Figure 12, the Fab (MCSP)- Fab (MCSP)-Crossfab (CD3) induces apoptosis in target cells at least comparably good as the (scFv)2 (antiMCSP/anti huCD3e) molecule.

### LDH release assay with Fab (MCSP)- Crossfab (CD3) bispecific construct with MV-3 human melanoma target cells

An LDH release assay was performed as outlined above. Figure 19 shows killing of huMCSP-positive MV-3 tumor cells upon co-culture with human PBMCs (E:T ratio = 10:1), treated with Fab (MCSP)- Crossfab (CD3), respective the (scFv)2 (antiMCSP/anti huCD3e) reference molecule for ∼ 24 hours.

### LDH release assay with murine Crossfab (CD3)-Fab (MCSP)- Fab (MCSP) bispecific construct

Purified with murine Crossfab (CD3)-Fab (MCSP)- Fab (MCSP), targeting murine CD3, as well as human MCSP, was analyzed for its potential to induce T cell-mediated apoptosis in tumor target cells upon crosslinkage of the construct via binding of both targeting moieties to the respective antigens on cells.

Briefly, huMCSP-expressing B16/F10-huMCSP Fluc2 clone 48 tumor target cells are harvested with Cell Dissociation Buffer, washed and resuspendend in RPMI1640 medium, including 1x NEAA, 10 mM Hepes, 50 µm 2-b-ME and 1 mM sodium pyruvate.

20 000 cells per well were plated in a round-bottom 96-well-plate and the respective antibody dilution was added at the indicated concentrations. The bispecific construct and the different IgG controls were adjusted to the same molarity. As an additional control for the activation of murine T cells "T Cell Stim with ConA" (BD #354115) was used, diluted 1:160 with assay medium.

Murine pan T effector cells, isolated from splenocytes (C57BL/6 mice) were added to obtain a final E:T ratio of 10:1. For normalization, maximal lysis of the target cells (= 100 %) was determined by incubation of the target cells with a final concentration of 1 % Triton-X-100. Minimal lysis (= 0 %) refers to target cells co-incubated with effector cells, but without any construct or antibody.

After an incubation for 70 h at 37°C, 5 % CO2, LDH release of apoptotic/necrotic target cells into the supernatant was measured with the LDH detection kit (Roche Applied Science, # 11 644 793 001), according to the manufacturer's instructions.

As depicted in Figure 13, the bispecific construct induces concentration-dependent LDH release from target cells, comparable to the positive control with "T Cell Stim with ConA".

### LDH release assay with murine Crossfab (CD3)-Fab (MCSP)- Fab (MCSP) bispecific construct

Purified murine Crossfab (CD3)-Fab (MCSP)- Fab (MCSP), targeting murine CD3, as well as human MCSP, was analyzed for its potential to induce T cell-mediated apoptosis in tumor target cells upon crosslinkage of the construct via binding of both targeting moieties to the respective antigens on cells.

Briefly, huMCSP-expressing B16/F10-huMCSP Fluc2 clone 48 tumor target cells are harvested with Cell Dissociation Buffer, washed and resuspendend in RPMI1640 medium, including 1x NEAA, 10 mM Hepes, 50 µM 2-b-ME and 1 mM sodium pyruvate.

20 000 cells per well were plated in a round-bottom 96-well-plate and the respective antibody dilution was added to obtain a final concentration of 50 nM. The bispecific construct and the different IgG controls were adjusted to the same molarity.

Murine pan T effector cells, isolated from splenocytes (C57BL/6 mice) were added to obtain a final E:T ratio of 10:1. To assess the level of hyperactivation of murine T cells in the absence of target cells, control wells with 50 nM bispecific construct and T cells were plated accordingly.

For normalization, maximal lysis of the target cells (= 100 %) was determined by incubation of the target cells with a final concentration of 1 % Triton-X-100. Minimal lysis (= 0 %) refers to target cells co-incubated with effector cells, but without any construct or antibody.

After an incubation for 70 h at 37°C, 5 % CO2, LDH release of apoptotic/necrotic target cells into the supernatant was measured with the LDH detection kit (Roche Applied Science, # 11 644 793 001), according to the manufacturer's instructions.

As depicted in Figure 14, the bispecific construct induces strong LDH release from target cells. In the absence of target cells, there is only a slight increase of LDH (reflecting hyperactivation of T cells) compared to untreated murine T cells, co-incubated with target cells. None of the control IgGs induces LDH release of target cells.

### Example 8: Cytokine release assay (CBA analysis)

To assess the *de novo* secretion of different cytokines upon T cell activation with CD3-bispecific constructs in the presence or absence of target cells, human PBMCs were isolated from Buffy Coats and 0.3 Mio cells per well were plated into a round-bottom 96-well plate. Alternatively, 280 µl whole blood from a healthy donor were plated per well of a deep-well 96-well plate.

Tumor target cells (e.g. MDA-MB-435 cells for CD3-MCSP-bispecific constructs) were added to obtain a final E/T-ratio of 10:1. Bispecific constructs and controls were added as indicated. After an incubation of up to 24 h at 37°C, 5 % CO2, the assay plate was centrifuged for 5 min at 350 g and the supernatant was transferred into a new deep-well 96-well-plate for the subsequent analysis.

The CBA analysis was performed according to manufacturers' instructions for FACS CantoII, using the combination of the following CBA Flex Sets: human granzyme B (BD 560304), human IFN-γ Flex Set (BD 558269), human TNF Flex Set (BD 558273), human IL-10 Flex Set (BD 558274), human IL-6 Flex Set (BD 558276), human IL-4 Flex Set (BD 558272).

### Cytokine Release Assay with MCSP-CD3 bispecific constructs

The following purified bispecific constructs targeting human MCSP and human CD3 were analyzed for their ability to induce T cell-mediated *de novo* secretion of cytokines in the presence (A, B) versus absence (C, D) of tumor target cells: "Fab (MCSP)- Fab (MCSP)-Crossfab (CD3) and the (scFv)2 (antiMCSP/anti huCD3e) reference molecule.

Briefly, 280 µl whole blood from a healthy donor were plated per well of a deep-well 96-well plate. 30 000 Colo-38 tumor target cells, expressing human MCSP, as well as the different bispecific constructs and IgG controls were added were added at 1 nM final concentration. The cells were incubated for 24 h at 37°C, 5 % CO2 and then centrifuged for 5 min at 350 x g. The supernatant was transferred into a new deep-well 96-well-plate for the subsequent analysis.

The CBA analysis was performed according to manufacturers' instructions for FACS CantoII, using the combination of the following CBA Flex Sets: human granzyme B (BD 560304), human IFN-γ Flex Set (BD 558269), human TNF Flex Set (BD 558273), human IL-10 Flex Set (BD 558274), human IL-6 Flex Set (BD 558276), human IL-4 Flex Set (BD 558272).

FIGURE 15 depicts different cytokine levels, that were measured in the supernatant of whole blood after treatment with 1 nM of different CD3-MCSP bispecific constructs (Fab (MCSP)- Fab (MCSP)-Crossfab (CD3) and the (scFv)2 (antiMCSP/anti huCD3e)) in the presence (A, B) or absence (C,D) of Colo-38 tumor cells for 24 hours. 280 µl whole blood were plated per well of a 96-well plate and 30 000 Colo-38 cells added, as indicated.

The main cytokine that was secreted upon activation of T cells in the presence of Colo-38 tumor cells, is IL-6, followed by IFNgamma. In addition, also the levels of granzyme B increased enormously upon activation of T cells in the presence of target cells. In general, the (scFv)2 (antiMCSP/anti huCD3e) construct elevated the levels of TNF and IFNgamma, as well as granzyme B in the presence of target cells (A and B) a bit more compared to the other bispecific construct.

There was no significant secretion of Th2 cytokines (IL-10 and IL-4) upon activation of T cells by the bispecific constructs in the presence (or absence) of target cells.

In this assay, there was also a weak secretion of IFNgamma, induced by the Fab (MCSP)-Fab (MCSP)-Crossfab (CD3) construct in the absence of target cells.

### Cytokine Release Assay with MCSP-murineCD3 bispecific constructs

The purified huMCSP-muCD3-targeting bispecific molecule as murine Crossfab (CD3)-Fab (MCSP)- Fab (MCSP) was tested by flow cytometry for its potential to up-regulate the late activation marker CD25 on CD8+ T cells in the presence of human MCSP-expressing tumor cells.

Briefly, MCSP-positive B16/F10-huMCSP Fluc2 clone 48 tumor cells were harvested with Cell Dissociation buffer, counted and checked for viability. Cells were adjusted to 0.3 x 10⁶ (viable) cells per ml in RPMI1640 medium (including 1x NEAA, 10 mM Hepes, 50 µm 2-b-ME, 1 mM sodium pyruvate), 100 µl of this cell suspension were pipetted per well into a round-bottom 96-well plate (as indicated). 50 µl of the (diluted) bispecific construct was added to the cell-containing wells to obtain a final concentration of 50 nM. Human murine T effector cells were isolated from splenocytes (C57BL/6 mice) and adjusted to 3 x 10⁶ (viable) cells per ml in AIM-V medium. 50 µl of this cell suspension was added per well of the assay plate (see above) to obtain a final E:T ratio of 10:1. To analyze, if the bispecific construct is able to activate T cells only in the presence of target cells, expressing huMCSP, wells were included that contained 50 nM of the respective bispecific molecule, as well as T effector, but no target cells.

After incubation for 70 hours at 37°C, 5 % CO2, cells were centrifuged (5 min, 350 x g) and washed twice with 150 µl/well PBS, including 0.1 % BSA.

Surface staining for CD8a (rat IgG2a; clone 53-6.7; BioLegend #100712) and CD25 (rat IgG2b; clone 3C7; BD #553075) was performed according to the suppliers' suggestions. Cells were washed twice with 150 µl/well PBS, including 0.1 % BSA and fixed for 15 min at 4°C, using 100 µl/well fixation buffer (BD ##554655).

After centrifugation, the samples were resuspended in 200 µl/well PBS, 0.1 % BSA and analyzed using a FACS CantoII machine (Software FACS Diva).

Figure 16 shows that the as murine Crossfab (CD3)-Fab (MCSP)- Fab (MCSP) construct induces up-regulation of CD25 in the presence of target cells only.

### Example 9: Expression of surface activation markers on primary human T cells upon engagement of bispecific constructs

To check for specific activation of T cells upon binding of CD3 bispecific constructs exclusively in the presence of tumor target cells, primary human PBMCs (isolated as described above) were incubated with the indicated concentrations of bispecific constructs for at least 24 h in the presence or absence of tumor antigen-positive target cells.

Briefly, 0.3 million primary human PBMCs were plated per well of a flat-bottom 96-well plate, containing the huMCSP-positive target cells (MV-3 tumor cells) or medium. The final effector to target cell (E:T) ratio was 10:1. The cells were incubated with the indicated concentration of the CD3-MCSP bispecific constructs (Fab (MCSP)-Crossfab (CD3); designated as "1+1 non-Fc", and the (scFv)2 (antiMCSP/anti huCD3e) reference molecule (designated as"(scFv)2")for the indicated incubation times at 37°C, 5% CO2. The effector cells were stained for CD8, and the early activation marker CD69 or the late activation marker CD25 and analyzed by FACS CantoII.

Figure 20 shows the result of this experiment.

While there are shown and described presently preferred embodiments of the invention, it is to be distinctly understood that the invention is not limited thereto but may be otherwise variously embodied and practiced within the scope of the following claims.

### Sequences

While there are shown and described presently preferred embodiments of the invention, it is to be distinctly understood that the invention is not limited thereto but may be otherwise variously embodied and practiced within the scope of the following claims. Legend: GA201= EGFR binder, 3F2= FAP binder, CH1A1A=CEA binder.

**Protein sequences**

| SEQ ID. NO. | Description | Sequence |
|---|---|---|
| 1 | CDR1 VL MCSP | SASQGIRNYLN |
| 2 | CDR2 VL MCSP | YTSSLHS |
| 3 | CDR3 VL MCSP | QQYSKLPWT |
| 4 | CDR1 VH MCSP | GYSITSGYYWN |
| 5 | CDR2 VH MCSP | YITYDGSNNYNPSLKN |
| 6 | CDR3 VH MCSP | FDY |
| 7 | CDR1 VL CD3_{(V9)} | RASQDIRNYLN |
| 8 | CDR2 VL CD3_{(V9)} | YTSRLES |
| 9 | CDR3 VL CD3_{(V9)} | QQGNTLPWT |
| 10 | CDR1 VH CD3_{(V9)} | GYTMN |
| 11 | CDR2 VH CD3_{(V9)} | LINPYKGVSTYNQKFKD |
| 12 | CDR3 VH CD3_{(V9)} | SGYYGDSDWYFDV |
| 29 | CDR1 VL CD3_{(H2C)} | GSSTGAVTSGYYPN |
| 30 | CDR2 VL CD3_{(H2C)} | GTKFLAP |
| 31 | CDR3 VL CD3_{(H2C)} | ALWYSNRWV |
| 32 | CDR1 VH CD3_{(H2C)} | GFTFNKYAMN |
| 33 | CDR2 VH CD3_{(H2C)} | RIRSKYNNYATYYADSVKD |
| 34 | CDR3 VH CD3_{(H2C)} | HGNFGNSYISYWAY |
| 13 | VL MCSP | |
| 14 | VH MCSP | |
| 15 | CL MCSP | |
| 16 | CH1 MCSP | |
| 17 | LIGHT CHAIN MCSP | |
| 18 | HEAVY CHAIN MCSP | |
| 19 | VL CD3_{(V9)} | |
| 20 | VH CD3_{(V9)} | |
| 21 | CL CD3_{(V9)} | |
| 22 | CH CD3_{(V9)} | |
| 23 | LIGHT CHAIN CD3_{(V9)} (VHCL) | |
| | | |
| 24 | HEAVY CHAIN CD3_{(V9)} (VLCH1) | |
| 35 | VL CD3_{(H2C)} | |
| 36 | VH CD3_{(H2C)} | |
| 37 | CL CD3_{(H2C)} | |
| 38 | CH1 CD3_{(H2C)} | |
| 39 | LIGHT CHAIN CD3_{(H2C)} (VHCL) | |
| 40 | HEAVY CHAIN CD3_{(H2C)} (VLCH1) | |
| 25 | FAB (MCSP)-XFAB (CD3_{(V9)}) (VH-CH1-VL-CH1) | |
| 26 | FAB (MCSP)-FAB (MCSP)-XFAB (CD3_{(V9)}) (VH-CH1-VH-CH1-VL-CH1) | |
| | | |
| 27 | FAB (MCSP)-XFAB (CD3_{(V9)})-FAB (MCSP) (VH-CH1-VL-CH1-VH-CH1) | |
| 28 | LINKER 1 | GGGGSGGGGS |
| 41 | FAB (MCSP)-FAB (MCSP)-XFAB (CD3_{(H2C)}) (VH-CH1-VH-CH1-VL-CH1) | |
| 42 | Murine LIGHT CHAIN CD3_{(2C11)} (VHCL) | |
| 43 | Murine XFAB (CD3_{(2C11)})-FAB (MCSP)-FAB (MCSP) (VL-CH1-VH-CH1-VH-CH1) | |
| 68 | GA201 CDR1 VH | DYKIH |
| 69 | GA201 CDR2 VH | YFNPNSGYSTYAQKFQG |
| 70 | GA201 CDR3 VH | LSPGGYYVMDA |
| 71 | GA201 CDR1 VL | RASQGINNYLN |
| 72 | GA201 CDR2 VL | NTNNLQT |
| 73 | GA201 CDR3 VL | LQHNSFPT |
| 74 | GA201 VH | |
| 75 | GA201 VL | |
| 76 | 3F2 CDR1 VH | SYAMS |
| 77 | 3F2 CDR2 VH | AISGSGGSTYYADSVK |
| 78 | 3F2 CDR3 VH | YCAKGWFG |
| 79 | 3F2 CDR1 VL | RASQSVTSSYL |
| 80 | 3F2 CDR2 VL | NVGSRRA |
| 81 | 3F2 CDR3 VL | CQQGIMLPP |
| 82 | 3F2 VH | |
| 83 | 3F2 VL | |
| 84 | CH1A1ACDR1 VH | EFGMN |
| 85 | CH1A1A CDR2 VH | WINTKTGEATYVEEFKG |
| 86 | CH1A1A CDR3 VH | WDFAYYVEAMDY |
| 87 | CH1A1ACDR1 VL | KASAAVGTYVA |
| 88 | CH1A1A CDR2 VL | SASYRKR |
| 89 | CH1A1A CDR3 VL | HQYYTYPLFT |
| 90 | CH1A1A VH | |
| 91 | CH1A1A VL | |
| 92 | Anti-CD33 CDR1 VH | GYTITDSNIH |
| 93 | Anti-CD33 CDR2 VH | YIYPYNGGTDYNQ |
| 94 | Anti-CD33 CDR3 VH | GNPWLAY |
| 95 | Anti-CD33 CDR1 VL | RASESLDNYGIRFLT |
| 96 | Anti-CD33 CDR1 VL | AASNQGS |
| 97 | Anti-CD33 CDR1 VL | QQTKEVPWS |
| 98 | Anti-CD33 VH | |
| 99 | Anti-CD33 VL | |
| | | |
| 100 | Light Chain antiCD33 | |
| 101 | Light Chain CD3_{(V9)} (VH-CL) | |
| 102 | Fab (CD33)-XFab (CD3_{(V9)}) (VH-CH1-VL-CH1) | |
| 145 | Linker 2 | EPKSCGGGGSGGGGS |
| 146 | Linker 3 | EPKSCDGGGGSGGGGS |
| 147 | Linker 4 | GGGGSGGGGSGGGGSGGGGSGGGGSGGGGSGG |
| 148 | Linker 5 | SGGGSGGGSEGGGSEGGGSEGGGSEGGGSGGGSG |
| 149 | (scFv)2 antiMCSP/anti huCD3e (MCSP(VL-VH)-CD3_{(V9)} (VH-VL)) | |
| 151 | Light Chain | |
| | antiCD33 _{(Myelotarg)} | |
| 152 | Light Chain CD3_{(V9)} (VL-CH1) | |
| 153 | Fab (CD33_{(Myelotarg)})-XFab (CD3_{(V9)}) (VH-CH1-VH-CL) | |
| 157 | CD3_{(CH2527)} VL | |
| 158 | CD3_{(CH2527)} VH | |
| 159 | CEA_{(CH1A1A (98/99))} VH | |
| 160 | CEA_{(CH1A1A (98/99))} VL | |
| 161 | MCSP_{(M4-3 ML2)} VH | |
| 162 | MCSP_{(M4-3 ML2)} VL | |

**DNA Sequences**

| SEQ ID. NO. | Description | Sequence |
|---|---|---|
| 44 | VL MCSP | |
| 45 | VH MCSP | |
| 46 | CL MCSP | |
| 47 | CH1 MCSP | |
| 48 | LIGHT CHAIN MCSP | |
| | | |
| 49 | HEAVY CHAIN MCSP | |
| 50 | VL CD3 _{(V9)} | |
| 51 | VH CD3 _{(V9)} | |
| | | |
| 52 | CL CD3 _{(V9)} | |
| 53 | CH CD3 _{(V9)} | |
| 54 | LIGHT CHAIN CD3_{(V9)} (VHCL) | |
| 55 | HEAVY CHAIN CD3_{(V9)} (VLCH1) | |
| | | |
| 56 | VL CD3 _{(H2C)} | |
| 57 | VH CD3 _{(H2C)} | |
| 58 | CL CD3 _{(H2C)} | |
| 59 | CH1 CD3 _{(H2C)} | |
| | | |
| 60 | LIGHT CHAIN CD3 _{(H2C)} (VHCL) | |
| 61 | HEAVY CHAIN CD3 _{(H2C)} (VLCH1) | |
| 62 | FAB (MCSP)-XFAB (CD3)_{(V9)} (VH-CH1-VL-CH1) | |
| | | |
| 63 | FAB (MCSP)-FAB (MCSP)-XFAB (CD3)_{(V9)} (VH-CH1-VH-CH1-VL-CH1) | |
| | | |
| 64 | FAB (MCSP)-XFAB (CD3_{(V9)})-FAB (MCSP) (VH-CH1-VL-CH1-VH-CH1) | |
| | | |
| 65 | FAB (MCSP)-FAB (MCSP)-XFAB (CD3_{(H2C)}) (VH-CH1-VH-CH1-VL-CH1) | |
| | | |
| 66 | Murine LIGHT | |
| | CHAIN CD3_{(2C11)} (VHCL) | |
| 67 | Murine XFAB (CD3_{(2C11)})-FAB (MCSP)-FAB (MCSP) (VL-CH1-VH-CH1-VH-CH1) | |
| | | |
| 104 | Light Chain antiCD33 | |
| 105 | Light Chain (CD3)_{(V9)} | |
| | (VH-CL) | |
| 106 | Fab(CD33)-CrossFab (CD3_{(V9)}) (VH-CH1-VL-CH1) | |
| | | |
| 107 | MCSP CDR1 VH | GGCTACTCCATCACCAGTGGTTATTACTGGAAC |
| 108 | MCSP CDR2 VH | |
| 109 | MCSP CDR3 VH | TTTGACTAC |
| 110 | MCSP CDR1 VL | AGTGCAAGTCAGGGCATTAGAAATTATTTAAAC |
| 111 | MCSP CDR2 VL | TACACATCAAGTTTACACTCA |
| 112 | MCSP CDR3VL | CAGCAGTATAGTAAGCTTCCTTGGACG |
| 113 | GA201 CDR1 VH | GACTACAAGATACAC |
| 114 | GA201 CDR2 VH | |
| 115 | GA201 CDR3 VH | CTATCCCCAGGCGGTTACTATGTTATGGATGCC |
| 116 | GA201 CDR1 VL | CGGGCAAGTCAGGGCATTAACAATTACTTAAAT |
| 117 | GA201 CDR2 VL | AATACCAACAACTTGCAGACA |
| 118 | GA201 CDR3 VL | TTGCAGCATAATAGTTTTCCCACG |
| 119 | GA201 VH | |
| 120 | GA201 VL | |
| | | |
| 121 | 3F2 CDR1 VH | AGCTACGCCATGAGC |
| 122 | 3F2 CDR2 VH | |
| 123 | 3F2 CDR3 VH | TATTGCGCCAAGGGATGGTTCGGC |
| 124 | 3F2 CDR1 VL | AGAGCCAGCCAGAGCGTGACCAGCAGCTACCTG |
| 125 | 3F2 CDR2 VL | AACGTGGGCAGCAGACGGGCC |
| 126 | 3F2 CDR3 VL | TGCCAGCAGGGCATCATGCTGCCCCCC |
| 127 | 3F2 VH | |
| 128 | 3F2 VL | |
| 129 | CH1A1ACDR1 VH | GAGTTCGGCATGAAC |
| 130 | CH1A1A CDR2 VH | |
| 131 | CH1A1A CDR3 VH | TGGGACTTCGCCTATTACGTGGAAGCCATGGACTAC |
| 132 | CH1A1ACDR1 VL | AAGGCCAGTGCGGCTGTGGGTACGTATGTTGCG |
| 133 | CH1A1A CDR2 VL | TCGGCATCCTACCGCAAAAGG |
| 134 | CH1A1A CDR3 VL | CACCAATATTACACCTATCCTCTATTCACG |
| 135 | CH1A1A VH | |
| | | |
| 136 | CH1A1A VL | |
| 137 | Anti-CD33 CDR1 VH | GGCTACACCATCACCGACAGCAACATCCAC |
| 138 | Anti-CD33 CDR2 VH | |
| 139 | Anti-CD33 CDR3 VH | GGCAACCCCTGGCTGGCCTAT |
| 140 | Anti-CD33 CDR1 VL | |
| 141 | Anti-CD33 CDR2 VL | GCCGCCAGCAACCAGGGCAGC |
| 142 | Anti-CD33 CDR3 VL | CAGCAGACCAAAGAGGTGCCCTGGTCC |
| 143 | Anti-CD33 VH | |
| 144 | Anti-CD33 VL | |
| | | |
| 150 | (scFv)2 antiMCSP/anti huCD3 (LC007(VL-VH)-V9(VH-VL)) | |
| 154 | Light Chain antiCD33 | |
| | (Myelotarg) | |
| 155 | Light Chain CD3_{(V9)} (VL-CH1) | |
| 156 | Fab (CD33_{(Myelotarg)})-XFab (CD3_{(V9)}) (VH-CH1-VH-CL) | |
| | | |

While there are shown and described presently preferred embodiments of the invention, it is to be distinctly understood that the invention is not limited thereto but may be otherwise variously embodied and practiced within the scope of the following claims.

## Claims

1. A bispecific antibody comprising at least two Fab fragments, wherein
the first Fab fragment comprises at least one antigen binding site specific for a first antigen; and the second Fab fragment comprises at least one antigen binding site specific for a second antigen wherein either the variable regions or the constant regions of the second Fab heavy and light chain are exchanged; wherein the bispecific antibody is devoid of a Fc domain such that the constant heavy chain consists solely of one or more CH1 domains and wherein the Fab fragments are connected via a peptide linker.

2. The bispecific antibody of claim 1, additionally comprising a third Fab fragment.

3. The bispecific antibody of claim 2, wherein the third Fab fragment comprises at least one antigen binding site specific for the first or second antigen.

4. The bispecific antibody of claim 2, wherein the third Fab fragment comprises at least one antigen binding site specific for the first antigen.

5. The bispecific antibody of any of claims 2 to 4, wherein the third Fab fragment is connected to the first Fab fragment.

6. The bispecific antibody of claim 5, wherein the C-terminus of the third Fab fragment is connected to the N-terminus of the first Fab fragment.

7. The bispecific antibody of claims 2 to 4, wherein the third Fab fragment is connected to the second Fab fragment.

8. The bispecific antibody of claim 7, wherein the N-terminus of the third Fab fragment is connected to the C-terminus of the second Fab fragment.

9. The bispecific antibody of any one of claims 1 to 8, wherein the peptide linker is a (G4S)2 linker.

10. A pharmaceutical composition comprising a bispecific antibody of claims 1 to 9.

11. A prokaryotic or eukaryotic host cell comprising vectors comprising nucleic acid molecules encoding the light chains and heavy chains of the bispecific antibody of any of claims 1 to 9.

12. A method of producing an antibody comprising culturing the host cell of claim 11 so that the antibody is produced.

13. An immunoconjugate comprising the antibody of any of claims 1 to 9 and a cytotoxic agent.

14. Nucleic acid comprising nucleic acid molecules encoding the light chains and heavy chains of the bispecific antibody of any of claims 1 to 9.

15. The bispecific antibody of any one of claims 1 to 9 for use as a medicament, in particular for use in the treatment of cancer.

## Patentansprüche

1. Bispezifischer Antikörper, umfassend wenigstens zwei Fab-Fragmente, wobei das erste Fab-Fragment wenigstens eine für ein erstes Antigen spezifische Antigenbindungsstelle umfasst; und das zweite Fab-Fragment wenigstens eine für ein zweites Antigen spezifische Antigenbindungsstelle umfasst, wobei entweder die variablen Regionen oder die konstanten Regionen der schweren und der leichten Kette des zweiten Fab ausgetauscht sind; wobei dem bispezifischen Antikörper eine Fc-Domäne fehlt, so dass die konstante schwere Kette nur aus einer oder mehreren CH1-Domänen besteht, und wobei die Fab-Fragmente über einen Peptidlinker verbunden sind.

2. Bispezifischer Antikörper nach Anspruch 1, der zusätzlich ein drittes Fab-Fragment umfasst.

3. Bispezifischer Antikörper nach Anspruch 2, wobei das dritte Fab-Fragment wenigstens eine für das erste oder zweite Antigen spezifische Antigenbindungsstelle umfasst.

4. Bispezifischer Antikörper nach Anspruch 2, wobei das dritte Fab-Fragment wenigstens eine für das erste Antigen spezifische Antigenbindungsstelle umfasst.

5. Bispezifischer Antikörper nach einem der Ansprüche 2 bis 4, wobei das dritte Fab-Fragment mit dem ersten Fab-Fragment verbunden ist.

6. Bispezifischer Antikörper nach Anspruch 5, wobei der C-Terminus des dritten Fab-Fragments mit dem N-Terminus des ersten Fab-Fragments verbunden ist.

7. Bispezifischer Antikörper nach den Ansprüchen 2 bis 4, wobei das dritte Fab-Fragment mit dem zweiten Fab-Fragment verbunden ist.

8. Bispezifischer Antikörper nach Anspruch 7, wobei der N-Terminus des dritten Fab-Fragments mit dem C-Terminus des zweiten Fab-Fragments verbunden ist.

9. Bispezifischer Antikörper nach einem der Ansprüche 1 bis 8, wobei der Peptidlinker ein (G4S)2-Linker ist.

10. Pharmazeutische Zusammensetzung, umfassend einen bispezifischen Antikörper nach den Ansprüchen 1 bis 9.

11. Prokaryotische oder eukaryotische Wirtszelle, umfassend Vektoren, die Nukleinsäuremoleküle umfassen, welche für die leichten Ketten und die schweren Ketten des bispezifischen Antikörpers nach einem der Ansprüche 1 bis 9 kodieren.

12. Verfahren zum Herstellen eines Antikörpers, umfassend das Züchten der Wirtszelle nach Anspruch 11, so dass der Antikörper hergestellt wird.

13. Immunkonjugat, umfassend den Antikörper nach einem der Ansprüche 1 bis 9 und ein zytotoxisches Mittel.

14. Nukleinsäure, umfassend Nukleinsäuremoleküle, welche für die leichten Ketten und die schweren Ketten des bispezifischen Antikörpers nach einem der Ansprüche 1 bis 9 kodieren.

15. Bispezifischer Antikörper nach einem der Ansprüche 1 bis 9 zur Verwendung als Medikament, insbesondere zur Verwendung bei der Behandlung von Krebs.

## Revendications

1. Anticorps bispécifique comprenant au moins deux fragments Fab, dans lequel le premier fragment Fab comprend au moins un site de liaison à l'antigène spécifique d'un premier antigène ; et le deuxième fragment Fab comprend au moins un site de liaison à l'antigène spécifique d'un second antigène, les régions variables ou les régions constantes de la chaîne lourde et de la chaîne légère du deuxième fragment Fab étant échangées ; l'anticorps bispécifique étant dépourvu de domaine Fc, de telle sorte que la chaîne lourde constante consiste uniquement en un ou plusieurs domaines CH1 et les fragments Fab étant reliés par le biais d'un lieur peptidique.

2. Anticorps bispécifique selon la revendication 1, comprenant en outre un troisième fragment Fab.

3. Anticorps bispécifique selon la revendication 2, dans lequel le troisième fragment Fab comprend au moins un site de liaison à l'antigène spécifique premier ou du second antigène.

4. Anticorps bispécifique selon la revendication 2, dans lequel le troisième fragment Fab comprend au moins un site de liaison à l'antigène spécifique du premier antigène.

5. Anticorps bispécifique selon l'une quelconque des revendications 2 à 4, dans lequel le troisième fragment Fab est relié au premier fragment Fab.

6. Anticorps bispécifique selon la revendication 5, dans lequel l'extrémité C-terminale du troisième fragment Fab est reliée à l'extrémité N-terminale du premier fragment Fab.

7. Anticorps bispécifique selon les revendications 2 à 4, dans lequel le troisième fragment Fab est relié au deuxième fragment Fab.

8. Anticorps bispécifique selon la revendication 7, dans lequel l'extrémité N-terminale du troisième fragment Fab est reliée à l'extrémité C-terminale du deuxième fragment Fab.

9. Anticorps bispécifique selon l'une quelconque des revendications 1 à 8, dans lequel le lieur peptidique est un lieur (G4S)2.

10. Composition pharmaceutique comprenant un anticorps bispécifique selon les revendications 1 à 9.

11. Cellule hôte procaryote ou eucaryote comprenant des vecteurs comprenant des molécules d'acides nucléiques codant pour les chaînes légères et les chaînes lourdes de l'anticorps bispécifique selon l'une quelconque des revendications 1 à 9.

12. Procédé de production d'un anticorps comprenant la culture de la cellule hôte selon la revendication 11 de sorte à produire l'anticorps.

13. Immunoconjugué comprenant l'anticorps selon l'une quelconque des revendications 1 à 9 et un agent cytotoxique.

14. Acide nucléique comprenant des molécules d'acides nucléiques codant pour les chaînes légères et les chaînes lourdes de l'anticorps bispécifique selon l'une quelconque des revendications 1 à 9.

15. Anticorps bispécifique selon l'une quelconque des revendications 1 à 9, destiné à être utilisé comme médicament, en particulier destiné à être utilisé dans le traitement d'un cancer.


### SEQUENCE LISTING

<110> Roche Glycart AG
<120> FC FREE ANTIBODIES COMPRISING TWO FAB FRAGMENTS AND METHODS OF USE
<130> 30600
<150> EP11178391.6
   <151> 2011-08-23
<160> 162
<170> PatentIn version 3.5
<210> 1
   <211> 11
   <212> PRT
   <213> Artificial Sequence
<220>
   <223> CDR1 VL MCSP
<400> 1
   Ser Ala Ser Gln Gly Ile Arg Asn Tyr Leu Asn
   1               5                   10
<210> 2
   <211> 7
   <212> PRT
   <213> Artificial Sequence
<220>
   <223> CDR2 VL MCSP
<400> 2
   Tyr Thr Ser Ser Leu His Ser
   1               5
<210> 3
   <211> 9
   <212> PRT
   <213> Artificial Sequence
<220>
   <223> CDR3 VL MCSP
<400> 3
   Gln Gln Tyr Ser Lys Leu Pro Trp Thr
   1               5
<210> 4
   <211> 11
   <212> PRT
   <213> Artificial Sequence
<220>
   <223> CDR1 VH MCSP
<400> 4
   Gly Tyr Ser Ile Thr Ser Gly Tyr Tyr Trp Asn
   1 5 10
<210> 5
   <211> 16
   <212> PRT
   <213> Artificial Sequence
<220>
   <223> CDR2 VH MCSP
<400> 5
   Tyr Ile Thr Tyr Asp Gly Ser Asn Asn Tyr Asn Pro Ser Leu Lys Asn
   1 5 10 15
<210> 6
   <211> 3
   <212> PRT
   <213> Artificial Sequence
<220>
   <223> CDR3 VH MCSP
<400> 6
1
<210> 7
   <211> 11
   <212> PRT
   <213> Artificial Sequence
<220>
   <223> CDR1 VL CD3
<400> 7
   Arg Ala Ser Gln Asp Ile Arg Asn Tyr Leu Asn
   1 5 10
<210> 8
   <211> 7
   <212> PRT
   <213> Artificial Sequence
<220>
   <223> CDR2 VL CD3
<400> 8
   Tyr Thr Ser Arg Leu Glu Ser
   1 5
<210> 9
   <211> 9
   <212> PRT
   <213> Artificial Sequence
<220>
   <223> CDR3 VL CD3
<400> 9
   Gln Gln Gly Asn Thr Leu Pro Trp Thr
   1 5
<210> 10
   <211> 5
   <212> PRT
   <213> Artificial Sequence
<220>
   <223> CDR1 VH CD3
<400> 10
   Gly Tyr Thr Met Asn
   1 5
<210> 11
   <211> 17
   <212> PRT
   <213> Artificial Sequence
<220>
   <223> CDR2 VH CD3
<400> 11
   Leu Ile Asn Pro Tyr Lys Gly Val Ser Thr Tyr Asn Gln Lys Phe Lys
   1 5 10 15
   Asp
<210> 12
   <211> 13
   <212> PRT
   <213> Artificial Sequence
<220>
   <223> CDR3 VH CD3
<400> 12
   Ser Gly Tyr Tyr Gly Asp Ser Asp Trp Tyr Phe Asp Val
   1 5 10
<210> 13
   <211> 107
   <212> PRT
   <213> Artificial Sequence
<220>
   <223> VL MCSP
<400> 13
   Asp Ile Val Leu Thr Gln Ser Pro Ser Ser Leu Ser Ala Ser Leu Gly
   1               5                   10                  15
   Asp Arg Val Thr Ile Ser Cys Ser Ala Ser Gln Gly Ile Arg Asn Tyr
               20                  25                  30
   Leu Asn Trp Tyr Gln Gln Arg Pro Asp Gly Thr Val Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Lys Leu Pro Trp
                   85                  90                  95
   Thr Phe Gly Gly Gly Thr Lys Leu Glu Ile Lys
               100                 105
<210> 14
   <211> 112
   <212> PRT
   <213> Artificial Sequence
<220>
   <223> VH MCSP
<400> 14
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
<210> 15
   <211> 107
   <212> PRT
   <213> Artificial Sequence
<220>
   <223> CL MCSP
<400> 15
<210> 16
   <211> 104
   <212> PRT
   <213> Artificial Sequence
<220>
   <223> CH1 MCSP
<400> 16
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys Asp
   100
<210> 17
   <211> 214
   <212> PRT
   <213> Artificial Sequence
<220>
   <223> LIGHT CHAIN MCSP
<400> 17
   Asp Ile Val Leu Thr Gln Ser Pro Ser Ser Leu Ser Ala Ser Leu Gly
   1               5                   10                  15
   Asp Arg Val Thr Ile Ser Cys Ser Ala Ser Gln Gly Ile Arg Asn Tyr
               20                  25                  30
   Leu Asn Trp Tyr Gln Gln Arg Pro Asp Gly Thr Val Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Lys Leu Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 18
   <211> 216
   <212> PRT
   <213> Artificial Sequence
<220>
   <223> HEAVY CHAIN MCSP
<400> 18
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp
       210                 215
<210> 19
   <211> 107
   <212> PRT
   <213> Artificial Sequence
<220>
   <223> VL CD3 (V9)
<400> 19
<210> 20
   <211> 122
   <212> PRT
   <213> Artificial Sequence
<220>
   <223> VH CD3 (V9)
<400> 20
<210> 21
   <211> 105
   <212> PRT
   <213> Artificial Sequence
<220>
   <223> CL CD3 (V9)
<400> 21
<210> 22
   <211> 103
   <212> PRT
   <213> Artificial Sequence
<220>
   <223> CH1 CD3 (V9)
<400> 22
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys
               100
<210> 23
   <211> 229
   <212> PRT
   <213> Artificial Sequence
<220>
   <223> Light chain CD3 (VHCL) (V9)
<400> 23
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
   50 55 60
   Lys Asp Arg Phe Thr Ile Ser Val Asp Lys Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Ser Gly Tyr Tyr Gly Asp Ser Asp Trp Tyr Phe Asp Val Trp
   100 105 110
   Gly Gln Gly Thr Leu Val Thr Val Ser Ser Ala Ser Val Ala Ala Pro
   115 120 125
   Ser Val Phe Ile Phe Pro Pro Ser Asp Glu Gln Leu Lys Ser Gly Thr
   130 135 140
   Ala Ser Val Val Cys Leu Leu Asn Asn Phe Tyr Pro Arg Glu Ala Lys
   145 150 155 160
   Val Gln Trp Lys Val Asp Asn Ala Leu Gln Ser Gly Asn Ser Gln Glu
   165 170 175
   Ser Val Thr Glu Gln Asp Ser Lys Asp Ser Thr Tyr Ser Leu Ser Ser
   180 185 190
   Thr Leu Thr Leu Ser Lys Ala Asp Tyr Glu Lys His Lys Val Tyr Ala
   195 200 205
   Cys Glu Val Thr His Gln Gly Leu Ser Ser Pro Val Thr Lys Ser Phe
   210 215 220
   Asn Arg Gly Glu Cys
   225
<210> 24
   <211> 212
   <212> PRT
   <213> Artificial Sequence
<220>
   <223> Heavy Chain CD3 (VLCH1) V9
<400> 24
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Arg Asn Tyr
   20 25 30
   Leu Asn Trp Tyr Gln Gln Lys Pro Gly Lys Ala Pro Lys Leu Leu Ile
   35 40 45
   Tyr Tyr Thr Ser Arg Leu Glu Ser Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Gln Gln Gly Asn Thr Leu Pro Trp
   85 90 95
   Thr Phe Gly Gln Gly Thr Lys Val Glu Ile Lys Ser Ser Ala Ser Thr
   100 105 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Lys Asp Tyr Phe Pro Glu
   130 135 140
   Pro Val Thr Val Ser Trp Asn Ser Gly Ala Leu Thr Ser Gly Val His
   145 150 155 160
   Thr Phe Pro Ala Val Leu Gln Ser Ser Gly Leu Tyr Ser Leu Ser Ser
   165 170 175
   Val Val Thr Val Pro Ser Ser Ser Leu Gly Thr Gln Thr Tyr Ile Cys
   180 185 190
   Asn Val Asn His Lys Pro Ser Asn Thr Lys Val Asp Lys Lys Val Glu
   195 200 205
   Pro Lys Ser Cys
   210
<210> 25
   <211> 438
   <212> PRT
   <213> Artificial Sequence
<220>
   <223> FAB (MCSP)-XFAB (CD3)
<400> 25
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Gly Gly Gly Gly Ser Gly Gly Gly
       210                 215                 220
   Gly Ser Asp Ile Gln Met Thr Gln Ser Pro Ser Ser Leu Ser Ala Ser
   225                 230                 235                 240
   Val Gly Asp Arg Val Thr Ile Thr Cys Arg Ala Ser Gln Asp Ile Arg
                   245                 250                 255
   Asn Tyr Leu Asn Trp Tyr Gln Gln Lys Pro Gly Lys Ala Pro Lys Leu
               260                 265                 270
   Leu Ile Tyr Tyr Thr Ser Arg Leu Glu Ser Gly Val Pro Ser Arg Phe
           275                 280                 285
   Ser Gly Ser Gly Ser Gly Thr Asp Tyr Thr Leu Thr Ile Ser Ser Leu
   290 295 300
   Gln Pro Glu Asp Phe Ala Thr Tyr Tyr Cys Gln Gln Gly Asn Thr Leu
   305 310 315 320
   Pro Trp Thr Phe Gly Gln Gly Thr Lys Val Glu Ile Lys Ser Ser Ala
   325 330 335
   Ser Thr Lys Gly Pro Ser Val Phe Pro Leu Ala Pro Ser Ser Lys Ser
   340 345 350
   Thr Ser Gly Gly Thr Ala Ala Leu Gly Cys Leu Val Lys Asp Tyr Phe
   355 360 365
   Pro Glu Pro Val Thr Val Ser Trp Asn Ser Gly Ala Leu Thr Ser Gly
   370 375 380
   Val His Thr Phe Pro Ala Val Leu Gln Ser Ser Gly Leu Tyr Ser Leu
   385 390 395 400
   Ser Ser Val Val Thr Val Pro Ser Ser Ser Leu Gly Thr Gln Thr Tyr
   405 410 415
   Ile Cys Asn Val Asn His Lys Pro Ser Asn Thr Lys Val Asp Lys Lys
   420 425 430
   Val Glu Pro Lys Ser Cys
   435
<210> 26
   <211> 664
   <212> PRT
   <213> Artificial Sequence
<220>
   <223> FAB (MCSP)-FAB (MCSP)-XFAB (CD3)
<400> 26
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65 70 75 80
   Leu Lys Leu Asn Ser Val Thr Thr Glu Asp Thr Ala Thr Tyr Tyr Cys
   85 90 95
   Ala Asp Phe Asp Tyr Trp Gly Gln Gly Thr Thr Leu Thr Val Ser Ser
   100 105 110
   Ala Ser Thr Lys Gly Pro Ser Val Phe Pro Leu Ala Pro Ser Ser Lys
   115 120 125
   Ser Thr Ser Gly Gly Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
   180 185 190
   Tyr Ile Cys Asn Val Asn His Lys Pro Ser Asn Thr Lys Val Asp Lys
   195 200 205
   Lys Val Glu Pro Lys Ser Cys Asp Gly Gly Gly Gly Ser Gly Gly Gly
   210 215 220
   Gly Ser Glu Val Gln Leu Gln Glu Ser Gly Pro Gly Leu Val Lys Pro
   225 230 235 240
   Ser Gln Ser Leu Ser Leu Thr Cys Ser Val Thr Gly Tyr Ser Ile Thr
   245 250 255
   Ser Gly Tyr Tyr Trp Asn Trp Ile Arg Gln Phe Pro Gly Asn Lys Leu
   260 265 270
   Glu Trp Met Gly Tyr Ile Thr Tyr Asp Gly Ser Asn Asn Tyr Asn Pro
   275 280 285
   Ser Leu Lys Asn Arg Ile Ser Ile Thr Arg Asp Thr Ser Lys Asn Gln
   290 295 300
   Phe Phe Leu Lys Leu Asn Ser Val Thr Thr Glu Asp Thr Ala Thr Tyr
   305 310 315 320
   Tyr Cys Ala Asp Phe Asp Tyr Trp Gly Gln Gly Thr Thr Leu Thr Val
   325 330 335
   Ser Ser Ala Ser Thr Lys Gly Pro Ser Val Phe Pro Leu Ala Pro Ser
   340 345 350
   Ser Lys Ser Thr Ser Gly Gly Thr Ala Ala Leu Gly Cys Leu Val Lys
   355 360 365
   Asp Tyr Phe Pro Glu Pro Val Thr Val Ser Trp Asn Ser Gly Ala Leu
   370 375 380
   Thr Ser Gly Val His Thr Phe Pro Ala Val Leu Gln Ser Ser Gly Leu
   385 390 395 400
   Tyr Ser Leu Ser Ser Val Val Thr Val Pro Ser Ser Ser Leu Gly Thr
   405 410 415
   Gln Thr Tyr Ile Cys Asn Val Asn His Lys Pro Ser Asn Thr Lys Val
   420 425 430
   Asp Lys Lys Val Glu Pro Lys Ser Cys Asp Gly Gly Gly Gly Ser Gly
   435 440 445
   Gly Gly Gly Ser Asp Ile Gln Met Thr Gln Ser Pro Ser Ser Leu Ser
   450 455 460
   Ala Ser Val Gly Asp Arg Val Thr Ile Thr Cys Arg Ala Ser Gln Asp
   465 470 475 480
   Ile Arg Asn Tyr Leu Asn Trp Tyr Gln Gln Lys Pro Gly Lys Ala Pro
   485 490 495
   Lys Leu Leu Ile Tyr Tyr Thr Ser Arg Leu Glu Ser Gly Val Pro Ser
   500 505 510
   Arg Phe Ser Gly Ser Gly Ser Gly Thr Asp Tyr Thr Leu Thr Ile Ser
   515 520 525
   Ser Leu Gln Pro Glu Asp Phe Ala Thr Tyr Tyr Cys Gln Gln Gly Asn
   530 535 540
   Thr Leu Pro Trp Thr Phe Gly Gln Gly Thr Lys Val Glu Ile Lys Ser
   545 550 555 560
   Ser Ala Ser Thr Lys Gly Pro Ser Val Phe Pro Leu Ala Pro Ser Ser
   565 570 575
   Lys Ser Thr Ser Gly Gly Thr Ala Ala Leu Gly Cys Leu Val Lys Asp
   580 585 590
   Tyr Phe Pro Glu Pro Val Thr Val Ser Trp Asn Ser Gly Ala Leu Thr
   595 600 605
   Ser Gly Val His Thr Phe Pro Ala Val Leu Gln Ser Ser Gly Leu Tyr
   610 615 620
   Ser Leu Ser Ser Val Val Thr Val Pro Ser Ser Ser Leu Gly Thr Gln
   625 630 635 640
   Thr Tyr Ile Cys Asn Val Asn His Lys Pro Ser Asn Thr Lys Val Asp
   645 650 655
   Lys Lys Val Glu Pro Lys Ser Cys
   660
<210> 27
   <211> 663
   <212> PRT
   <213> Artificial Sequence
<220>
   <223> FAB (MCSP)-XFAB (CD3)-FAB (MCSP)
<400> 27
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Gly Gly Gly Gly Ser Gly Gly Gly
       210                 215                 220
   Gly Ser Asp Ile Gln Met Thr Gln Ser Pro Ser Ser Leu Ser Ala Ser
   225                 230                 235                 240
   Val Gly Asp Arg Val Thr Ile Thr Cys Arg Ala Ser Gln Asp Ile Arg
                   245                 250                 255
   Asn Tyr Leu Asn Trp Tyr Gln Gln Lys Pro Gly Lys Ala Pro Lys Leu
               260                 265                 270
   Leu Ile Tyr Tyr Thr Ser Arg Leu Glu Ser Gly Val Pro Ser Arg Phe
           275                 280                 285
   Ser Gly Ser Gly Ser Gly Thr Asp Tyr Thr Leu Thr Ile Ser Ser Leu
       290                 295                 300
   Gln Pro Glu Asp Phe Ala Thr Tyr Tyr Cys Gln Gln Gly Asn Thr Leu
   305                 310                 315                 320
   Pro Trp Thr Phe Gly Gln Gly Thr Lys Val Glu Ile Lys Ser Ser Ala
                   325                 330                 335
   Ser Thr Lys Gly Pro Ser Val Phe Pro Leu Ala Pro Ser Ser Lys Ser
               340                 345                 350
   Thr Ser Gly Gly Thr Ala Ala Leu Gly Cys Leu Val Lys Asp Tyr Phe
           355                 360                 365
   Pro Glu Pro Val Thr Val Ser Trp Asn Ser Gly Ala Leu Thr Ser Gly
       370                 375                 380
   Val His Thr Phe Pro Ala Val Leu Gln Ser Ser Gly Leu Tyr Ser Leu
   385                 390                 395                 400
   Ser Ser Val Val Thr Val Pro Ser Ser Ser Leu Gly Thr Gln Thr Tyr
                   405                 410                 415
   Ile Cys Asn Val Asn His Lys Pro Ser Asn Thr Lys Val Asp Lys Lys
               420                 425                 430
   Val Glu Pro Lys Ser Cys Gly Gly Gly Gly Ser Gly Gly Gly Gly Ser
           435                 440                 445
   Glu Val Gln Leu Gln Glu Ser Gly Pro Gly Leu Val Lys Pro Ser Gln
       450                 455                 460
   Ser Leu Ser Leu Thr Cys Ser Val Thr Gly Tyr Ser Ile Thr Ser Gly
   465                 470                 475                 480
   Tyr Tyr Trp Asn Trp Ile Arg Gln Phe Pro Gly Asn Lys Leu Glu Trp
                   485                 490                 495
   Met Gly Tyr Ile Thr Tyr Asp Gly Ser Asn Asn Tyr Asn Pro Ser Leu
               500                 505                 510
   Lys Asn Arg Ile Ser Ile Thr Arg Asp Thr Ser Lys Asn Gln Phe Phe
           515                 520                 525
   Leu Lys Leu Asn Ser Val Thr Thr Glu Asp Thr Ala Thr Tyr Tyr Cys
       530                 535                 540
   Ala Asp Phe Asp Tyr Trp Gly Gln Gly Thr Thr Leu Thr Val Ser Ser
   545                 550                 555                 560
   Ala Ser Thr Lys Gly Pro Ser Val Phe Pro Leu Ala Pro Ser Ser Lys
                   565                 570                 575
   Ser Thr Ser Gly Gly Thr Ala Ala Leu Gly Cys Leu Val Lys Asp Tyr
               580                 585                 590
   Phe Pro Glu Pro Val Thr Val Ser Trp Asn Ser Gly Ala Leu Thr Ser
           595                 600                 605
   Gly Val His Thr Phe Pro Ala Val Leu Gln Ser Ser Gly Leu Tyr Ser
       610                 615                 620
   Leu Ser Ser Val Val Thr Val Pro Ser Ser Ser Leu Gly Thr Gln Thr
   625                 630                 635                 640
   Tyr Ile Cys Asn Val Asn His Lys Pro Ser Asn Thr Lys Val Asp Lys
   645 650 655
   Lys Val Glu Pro Lys Ser Cys
   660
<210> 28
   <211> 10
   <212> PRT
   <213> Artificial Sequence
<220>
   <223> Linker Sequence
<400> 28
   Gly Gly Gly Gly Ser Gly Gly Gly Gly Ser
   1 5 10
<210> 29
   <211> 14
   <212> PRT
   <213> Artificial Sequence
<220>
   <223> CDR1 VL CD3 (H2C)
<400> 29
   Gly Ser Ser Thr Gly Ala Val Thr Ser Gly Tyr Tyr Pro Asn
   1 5 10
<210> 30
   <211> 7
   <212> PRT
   <213> Artificial Sequence
<220>
   <223> CDR2 VL CD3 (H2C)
<400> 30
   Gly Thr Lys Phe Leu Ala Pro
   1 5
<210> 31
   <211> 9
   <212> PRT
   <213> Artificial Sequence
<220>
   <223> CDR3 VL CD3 (H2C)
<400> 31
   Ala Leu Trp Tyr Ser Asn Arg Trp Val
   1 5
<210> 32
   <211> 10
   <212> PRT
   <213> Artificial Sequence
<220>
   <223> CDR1 VH CD3 (H2C)
<400> 32
   Gly Phe Thr Phe Asn Lys Tyr Ala Met Asn
   1 5 10
<210> 33
   <211> 19
   <212> PRT
   <213> Artificial Sequence
<220>
   <223> CDR2 VH CD3 (H2C)
<400> 33
   Arg Ile Arg Ser Lys Tyr Asn Asn Tyr Ala Thr Tyr Tyr Ala Asp Ser
   1 5 10 15
   Val Lys Asp
<210> 34
   <211> 14
   <212> PRT
   <213> Artificial Sequence
<220>
   <223> CDR3 VH CD3 (H2C)
<400> 34
   His Gly Asn Phe Gly Asn Ser Tyr Ile Ser Tyr Trp Ala Tyr
   1 5 10
<210> 35
   <211> 109
   <212> PRT
   <213> Artificial Sequence
<220>
   <223> VL CD3 (H2C)
<400> 35
   Gln Thr Val Val Thr Gln Glu Pro Ser Leu Thr Val Ser Pro Gly Gly
   1               5                   10                  15
   Thr Val Thr Leu Thr Cys Gly Ser Ser Thr Gly Ala Val Thr Ser Gly
               20                  25                  30
   Tyr Tyr Pro Asn Trp Val Gln Gln Lys Pro Gly Gln Ala Pro Arg Gly
           35                  40                  45
   Leu Ile Gly Gly Thr Lys Phe Leu Ala Pro Gly Thr Pro Ala Arg Phe
       50                  55                  60
   Ser Gly Ser Leu Leu Gly Gly Lys Ala Ala Leu Thr Leu Ser Gly Val
   65                  70                  75                  80
   Gln Pro Glu Asp Glu Ala Glu Tyr Tyr Cys Ala Leu Trp Tyr Ser Asn
                   85                  90                  95
   Arg Trp Val Phe Gly Gly Gly Thr Lys Leu Thr Val Leu
               100                 105
<210> 36
   <211> 125
   <212> PRT
   <213> Artificial Sequence
<220>
   <223> VH CD3 (H2C)
<400> 36
   Glu Val Gln Leu Val Glu Ser Gly Gly Gly Leu Val Gln Pro Gly Gly
   1               5                   10                  15
   Ser Leu Lys Leu Ser Cys Ala Ala Ser Gly Phe Thr Phe Asn Lys Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Lys Asn Thr
   65                  70                  75                  80
   Ala Tyr Leu Gln Met Asn Asn Leu Lys Thr Glu Asp Thr Ala Val Tyr
                   85                  90                  95
   Tyr Cys Val Arg His Gly Asn Phe Gly Asn Ser Tyr Ile Ser Tyr Trp
               100                 105                 110
   Ala Tyr Trp Gly Gln Gly Thr Leu Val Thr Val Ser Ser
           115                 120                 125
<210> 37
   <211> 105
   <212> PRT
   <213> Artificial Sequence
<220>
   <223> CL CD3 (H2C)
<400> 37
<210> 38
   <211> 103
   <212> PRT
   <213> Artificial Sequence
<220>
   <223> CH1 CD3 (H2C)
<400> 38
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys
   100
<210> 39
   <211> 232
   <212> PRT
   <213> Artificial Sequence
<220>
   <223> LIGHT CHAIN CD3 (VHCL)
<400> 39
   Glu Val Gln Leu Val Glu Ser Gly Gly Gly Leu Val Gln Pro Gly Gly
   1               5                   10                  15
   Ser Leu Lys Leu Ser Cys Ala Ala Ser Gly Phe Thr Phe Asn Lys Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Lys Asn Thr
   65                  70                  75                  80
   Ala Tyr Leu Gln Met Asn Asn Leu Lys Thr Glu Asp Thr Ala Val Tyr
                   85                  90                  95
   Tyr Cys Val Arg His Gly Asn Phe Gly Asn Ser Tyr Ile Ser Tyr Trp
               100                 105                 110
   Ala Tyr Trp Gly Gln Gly Thr Leu Val Thr Val Ser Ser Ala Ser Val
           115                 120                 125
   Ala Ala Pro Ser Val Phe Ile Phe Pro Pro Ser Asp Glu Gln Leu Lys
       130                 135                 140
   Ser Gly Thr Ala Ser Val Val Cys Leu Leu Asn Asn Phe Tyr Pro Arg
   145                 150                 155                 160
   Glu Ala Lys Val Gln Trp Lys Val Asp Asn Ala Leu Gln Ser Gly Asn
                   165                 170                 175
   Ser Gln Glu Ser Val Thr Glu Gln Asp Ser Lys Asp Ser Thr Tyr Ser
               180                 185                 190
   Leu Ser Ser Thr Leu Thr Leu Ser Lys Ala Asp Tyr Glu Lys His Lys
           195                 200                 205
   Val Tyr Ala Cys Glu Val Thr His Gln Gly Leu Ser Ser Pro Val Thr
       210                 215                 220
   Lys Ser Phe Asn Arg Gly Glu Cys
   225                 230
<210> 40
   <211> 214
   <212> PRT
   <213> Artificial Sequence
<220>
   <223> HEAVY CHAIN CD3 (VLCH1)
<400> 40
   Gln Thr Val Val Thr Gln Glu Pro Ser Leu Thr Val Ser Pro Gly Gly
   1               5                   10                  15
   Thr Val Thr Leu Thr Cys Gly Ser Ser Thr Gly Ala Val Thr Ser Gly
               20                  25                  30
   Tyr Tyr Pro Asn Trp Val Gln Gln Lys Pro Gly Gln Ala Pro Arg Gly
           35                  40                  45
   Leu Ile Gly Gly Thr Lys Phe Leu Ala Pro Gly Thr Pro Ala Arg Phe
       50                  55                  60
   Ser Gly Ser Leu Leu Gly Gly Lys Ala Ala Leu Thr Leu Ser Gly Val
   65                  70                  75                  80
   Gln Pro Glu Asp Glu Ala Glu Tyr Tyr Cys Ala Leu Trp Tyr Ser Asn
                   85                  90                  95
   Arg Trp Val Phe Gly Gly Gly Thr Lys Leu Thr Val Leu Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
   180 185 190
   Ile Cys Asn Val Asn His Lys Pro Ser Asn Thr Lys Val Asp Lys Lys
   195 200 205
   Val Glu Pro Lys Ser Cys
   210
<210> 41
   <211> 666
   <212> PRT
   <213> Artificial Sequence
<220>
   <223> FAB(MCSP)-FAB(MCSP)-XFAB(CD3)
<400> 41
   Glu Val Gln Leu Gln Glu Ser Gly Pro Gly Leu Val Lys Pro Ser Gln
   1 5 10 15
   Ser Leu Ser Leu Thr Cys Ser Val Thr Gly Tyr Ser Ile Thr Ser Gly
   20 25 30
   Tyr Tyr Trp Asn Trp Ile Arg Gln Phe Pro Gly Asn Lys Leu Glu Trp
   35 40 45
   Met Gly Tyr Ile Thr Tyr Asp Gly Ser Asn Asn Tyr Asn Pro Ser Leu
   50 55 60
   Lys Asn Arg Ile Ser Ile Thr Arg Asp Thr Ser Lys Asn Gln Phe Phe
   65 70 75 80
   Leu Lys Leu Asn Ser Val Thr Thr Glu Asp Thr Ala Thr Tyr Tyr Cys
   85 90 95
   Ala Asp Phe Asp Tyr Trp Gly Gln Gly Thr Thr Leu Thr Val Ser Ser
   100 105 110
   Ala Ser Thr Lys Gly Pro Ser Val Phe Pro Leu Ala Pro Ser Ser Lys
   115 120 125
   Ser Thr Ser Gly Gly Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Gly Gly Gly Gly Ser Gly Gly Gly
       210                 215                 220
   Gly Ser Glu Val Gln Leu Gln Glu Ser Gly Pro Gly Leu Val Lys Pro
   225                 230                 235                 240
   Ser Gln Ser Leu Ser Leu Thr Cys Ser Val Thr Gly Tyr Ser Ile Thr
                   245                 250                 255
   Ser Gly Tyr Tyr Trp Asn Trp Ile Arg Gln Phe Pro Gly Asn Lys Leu
               260                 265                 270
   Glu Trp Met Gly Tyr Ile Thr Tyr Asp Gly Ser Asn Asn Tyr Asn Pro
           275                 280                 285
   Ser Leu Lys Asn Arg Ile Ser Ile Thr Arg Asp Thr Ser Lys Asn Gln
       290                 295                 300
   Phe Phe Leu Lys Leu Asn Ser Val Thr Thr Glu Asp Thr Ala Thr Tyr
   305                 310                 315                 320
   Tyr Cys Ala Asp Phe Asp Tyr Trp Gly Gln Gly Thr Thr Leu Thr Val
                   325                 330                 335
   Ser Ser Ala Ser Thr Lys Gly Pro Ser Val Phe Pro Leu Ala Pro Ser
               340                 345                 350
   Ser Lys Ser Thr Ser Gly Gly Thr Ala Ala Leu Gly Cys Leu Val Lys
           355                 360                 365
   Asp Tyr Phe Pro Glu Pro Val Thr Val Ser Trp Asn Ser Gly Ala Leu
       370                 375                 380
   Thr Ser Gly Val His Thr Phe Pro Ala Val Leu Gln Ser Ser Gly Leu
   385                 390                 395                 400
   Tyr Ser Leu Ser Ser Val Val Thr Val Pro Ser Ser Ser Leu Gly Thr
                   405                 410                 415
   Gln Thr Tyr Ile Cys Asn Val Asn His Lys Pro Ser Asn Thr Lys Val
               420                 425                 430
   Asp Lys Lys Val Glu Pro Lys Ser Cys Asp Gly Gly Gly Gly Ser Gly
               435                 440                 445
   Gly Gly Gly Ser Gln Thr Val Val Thr Gln Glu Pro Ser Leu Thr Val
       450                 455                 460
   Ser Pro Gly Gly Thr Val Thr Leu Thr Cys Gly Ser Ser Thr Gly Ala
   465                 470                 475                 480
   Val Thr Ser Gly Tyr Tyr Pro Asn Trp Val Gln Gln Lys Pro Gly Gln
                   485                 490                 495
   Ala Pro Arg Gly Leu Ile Gly Gly Thr Lys Phe Leu Ala Pro Gly Thr
               500                 505                 510
   Pro Ala Arg Phe Ser Gly Ser Leu Leu Gly Gly Lys Ala Ala Leu Thr
           515                 520                 525
   Leu Ser Gly Val Gln Pro Glu Asp Glu Ala Glu Tyr Tyr Cys Ala Leu
       530                 535                 540
   Trp Tyr Ser Asn Arg Trp Val Phe Gly Gly Gly Thr Lys Leu Thr Val
   545                 550                 555                 560
   Leu Ser Ser Ala Ser Thr Lys Gly Pro Ser Val Phe Pro Leu Ala Pro
                   565                 570                 575
   Ser Ser Lys Ser Thr Ser Gly Gly Thr Ala Ala Leu Gly Cys Leu Val
               580                 585                 590
   Lys Asp Tyr Phe Pro Glu Pro Val Thr Val Ser Trp Asn Ser Gly Ala
           595                 600                 605
   Leu Thr Ser Gly Val His Thr Phe Pro Ala Val Leu Gln Ser Ser Gly
       610                 615                 620
   Leu Tyr Ser Leu Ser Ser Val Val Thr Val Pro Ser Ser Ser Leu Gly
   625                 630                 635                 640
   Thr Gln Thr Tyr Ile Cys Asn Val Asn His Lys Pro Ser Asn Thr Lys
                   645                 650                 655
   Val Asp Lys Lys Val Glu Pro Lys Ser Cys
               660                 665
<210> 42
   <211> 223
   <212> PRT
   <213> Artificial Sequence
<220>
   <223> LIGHT CHAIN CD3
<400> 42
   Glu Val Gln Leu Val Glu Ser Gly Gly Gly Leu Val Gln Pro Gly Lys
   1               5                   10                  15
   Ser Leu Lys Leu Ser Cys Glu Ala Ser Gly Phe Thr Phe Ser Gly Tyr
               20                  25                  30
   Gly Met His Trp Val Arg Gln Ala Pro Gly Arg Gly Leu Glu Ser Val
           35                  40                  45
   Ala Tyr Ile Thr Ser Ser Ser Ile Asn Ile Lys Tyr Ala Asp Ala Val
       50                  55                  60
   Lys Gly Arg Phe Thr Val Ser Arg Asp Asn Ala Lys Asn Leu Leu Phe
   65                  70                  75                  80
   Leu Gln Met Asn Ile Leu Lys Ser Glu Asp Thr Ala Met Tyr Tyr Cys
                   85                  90                  95
   Ala Arg Phe Asp Trp Asp Lys Asn Tyr Trp Gly Gln Gly Thr Met Val
               100                 105                 110
   Thr Val Ser Ser Ala Ser Val Ala Ala Pro Ser Val Phe Ile Phe Pro
           115                 120                 125
   Pro Ser Asp Glu Gln Leu Lys Ser Gly Thr Ala Ser Val Val Cys Leu
       130                 135                 140
   Leu Asn Asn Phe Tyr Pro Arg Glu Ala Lys Val Gln Trp Lys Val Asp
   145                 150                 155                 160
   Asn Ala Leu Gln Ser Gly Asn Ser Gln Glu Ser Val Thr Glu Gln Asp
                   165                 170                 175
   Ser Lys Asp Ser Thr Tyr Ser Leu Ser Ser Thr Leu Thr Leu Ser Lys
               180                 185                 190
   Ala Asp Tyr Glu Lys His Lys Val Tyr Ala Cys Glu Val Thr His Gln
           195                 200                 205
   Gly Leu Ser Ser Pro Val Thr Lys Ser Phe Asn Arg Gly Glu Cys
       210                 215                 220
<210> 43
   <211> 664
   <212> PRT
   <213> Mus musculus
<400> 43
   Asp Ile Gln Met Thr Gln Ser Pro Ser Ser Leu Pro Ala Ser Leu Gly
   1               5                   10                  15
   Asp Arg Val Thr Ile Asn Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Asn Lys Leu Ala Asp Gly Val Pro Ser Arg Phe Ser Gly
       50                  55                  60
   Ser Gly Ser Gly Arg Asp Ser Ser Phe Thr Ile Ser Ser Leu Glu Ser
   65                  70                  75                  80
   Glu Asp Ile Gly Ser Tyr Tyr Cys Gln Gln Tyr Tyr Asn Tyr Pro Trp
                   85                  90                  95
   Thr Phe Gly Pro Gly Thr Lys Leu Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Gly Gly Gly Gly Ser Gly Gly Gly Gly Ser Glu Val
       210                 215                 220
   Gln Leu Gln Glu Ser Gly Pro Gly Leu Val Lys Pro Ser Gln Ser Leu
   225                 230                 235                 240
   Ser Leu Thr Cys Ser Val Thr Gly Tyr Ser Ile Thr Ser Gly Tyr Tyr
                   245                 250                 255
   Trp Asn Trp Ile Arg Gln Phe Pro Gly Asn Lys Leu Glu Trp Met Gly
               260                 265                 270
   Tyr Ile Thr Tyr Asp Gly Ser Asn Asn Tyr Asn Pro Ser Leu Lys Asn
           275                 280                 285
   Arg Ile Ser Ile Thr Arg Asp Thr Ser Lys Asn Gln Phe Phe Leu Lys
       290                 295                 300
   Leu Asn Ser Val Thr Thr Glu Asp Thr Ala Thr Tyr Tyr Cys Ala Asp
   305                 310                 315                 320
   Phe Asp Tyr Trp Gly Gln Gly Thr Thr Leu Thr Val Ser Ser Ala Ser
                   325                 330                 335
   Thr Lys Gly Pro Ser Val Phe Pro Leu Ala Pro Ser Ser Lys Ser Thr
               340                 345                 350
   Ser Gly Gly Thr Ala Ala Leu Gly Cys Leu Val Lys Asp Tyr Phe Pro
           355                 360                 365
   Glu Pro Val Thr Val Ser Trp Asn Ser Gly Ala Leu Thr Ser Gly Val
       370                 375                 380
   His Thr Phe Pro Ala Val Leu Gln Ser Ser Gly Leu Tyr Ser Leu Ser
   385                 390                 395                 400
   Ser Val Val Thr Val Pro Ser Ser Ser Leu Gly Thr Gln Thr Tyr Ile
                   405                 410                 415
   Cys Asn Val Asn His Lys Pro Ser Asn Thr Lys Val Asp Lys Lys Val
               420                 425                 430
   Glu Pro Lys Ser Cys Asp Gly Gly Gly Gly Ser Gly Gly Gly Gly Ser
           435                 440                 445
   Glu Val Gln Leu Gln Glu Ser Gly Pro Gly Leu Val Lys Pro Ser Gln
       450                 455                 460
   Ser Leu Ser Leu Thr Cys Ser Val Thr Gly Tyr Ser Ile Thr Ser Gly
   465                 470                 475                 480
   Tyr Tyr Trp Asn Trp Ile Arg Gln Phe Pro Gly Asn Lys Leu Glu Trp
                   485                 490                 495
   Met Gly Tyr Ile Thr Tyr Asp Gly Ser Asn Asn Tyr Asn Pro Ser Leu
               500                 505                 510
   Lys Asn Arg Ile Ser Ile Thr Arg Asp Thr Ser Lys Asn Gln Phe Phe
   515 520 525
   Leu Lys Leu Asn Ser Val Thr Thr Glu Asp Thr Ala Thr Tyr Tyr Cys
   530 535 540
   Ala Asp Phe Asp Tyr Trp Gly Gln Gly Thr Thr Leu Thr Val Ser Ser
   545 550 555 560
   Ala Ser Thr Lys Gly Pro Ser Val Phe Pro Leu Ala Pro Ser Ser Lys
   565 570 575
   Ser Thr Ser Gly Gly Thr Ala Ala Leu Gly Cys Leu Val Lys Asp Tyr
   580 585 590
   Phe Pro Glu Pro Val Thr Val Ser Trp Asn Ser Gly Ala Leu Thr Ser
   595 600 605
   Gly Val His Thr Phe Pro Ala Val Leu Gln Ser Ser Gly Leu Tyr Ser
   610 615 620
   Leu Ser Ser Val Val Thr Val Pro Ser Ser Ser Leu Gly Thr Gln Thr
   625 630 635 640
   Tyr Ile Cys Asn Val Asn His Lys Pro Ser Asn Thr Lys Val Asp Lys
   645 650 655
   Lys Val Glu Pro Lys Ser Cys Asp
   660
<210> 44
   <211> 321
   <212> DNA
   <213> Artificial Sequence
<220>
   <223> VL MCSP DNA
<400> 44
   gatattgtgc tcacacagtc tccatcctcc ctgtctgcct ctctgggaga cagagtcacc 60
   atcagttgca gtgcaagtca gggcattaga aattatttaa actggtatca gcagagacca 120
   gatggaactg ttaaactcct gatctattac acatcaagtt tacactcagg agtcccatca 180
   aggttcagtg gcagtgggtc tgggacagat tattctctca ccatcagcaa cctggaacct 240
   gaagatattg ccacttacta ttgtcagcag tatagtaagc ttccttggac gttcggtgga 300
   ggcaccaagc tggaaatcaa a 321
<210> 45
   <211> 336
   <212> DNA
   <213> Artificial Sequence
<220>
   <223> VH MCSP DNA
<400> 45
   gaggtgcagc tgcaggaatc tggccctggc ctggtcaagc caagccagag tctgagcctg 60
   acctgcagcg tgaccggcta cagcattacc agcggctact actggaactg gattcggcag 120
   ttccccggca ataagctgga atggatgggc tacatcacct acgacggcag caacaactac 180
   aaccccagcc tgaagaaccg gatcagcatc acccgggaca ccagcaagaa ccagttcttc 240
   ctgaagctga acagcgtgac caccgaggac accgccacat actattgcgc cgacttcgac 300
   tactggggcc agggcaccac cctgaccgtg tccagc 336
<210> 46
   <211> 324
   <212> DNA
   <213> Artificial Sequence
<220>
   <223> CL MCSP DNA
<400> 46
   cgtacggtgg ctgcaccatc tgtcttcatc ttcccgccat ctgatgagca gttgaaatct 60
   ggaactgcct ctgttgtgtg cctgctgaat aacttctatc ccagagaggc caaagtacag 120
   tggaaggtgg ataacgccct ccaatcgggt aactcccagg agagtgtcac agagcaggac 180
   agcaaggaca gcacctacag cctcagcagc accctgacgc tgagcaaagc agactacgag 240
   aaacacaaag tctacgcctg cgaagtcacc catcagggcc tgagctcgcc cgtcacaaag 300
   agcttcaaca ggggagagtg ttag 324
<210> 47
   <211> 312
   <212> DNA
   <213> Artificial Sequence
<220>
   <223> CH1 MCSP DNA
<400> 47
   gccagcacaa agggccctag cgtgttccct ctggccccca gcagcaagag cacaagcggc      60
   ggaacagccg ccctgggctg cctcgtgaag gactacttcc ccgagcccgt gacagtgtct     120
   tggaacagcg gagccctgac aagcggcgtg cacaccttcc ctgccgtgct gcagagcagc     180
   ggcctgtact ccctgagcag cgtggtcacc gtgcctagca gcagcctggg cacccagacc     240
   tacatctgca acgtgaacca caagcccagc aacaccaaag tggacaagaa ggtggagccc     300
   aagagctgtg at                                                         312
<210> 48
   <211> 711
   <212> DNA
   <213> Artificial Sequence
<220>
   <223> LIGHT CHAIN MCSP DNA
<400> 48
   atggacatga gggtccccgc tcagctcctg ggcctcctgc tgctctggtt cccaggtgcc      60
   aggtgtgata ttgtgctcac acagtctcca tcctccctgt ctgcctctct gggagacaga     120
   gtcaccatca gttgcagtgc aagtcagggc attagaaatt atttaaactg gtatcagcag     180
   agaccagatg gaactgttaa actcctgatc tattacacat caagtttaca ctcaggagtc     240
   ccatcaaggt tcagtggcag tgggtctggg acagattatt ctctcaccat cagcaacctg     300
   gaacctgaag atattgccac ttactattgt cagcagtata gtaagcttcc ttggacgttc     360
   ggtggaggca ccaagctgga aatcaaacgt acggtggctg caccatctgt cttcatcttc     420
   ccgccatctg atgagcagtt gaaatctgga actgcctctg ttgtgtgcct gctgaataac     480
   ttctatccca gagaggccaa agtacagtgg aaggtggata acgccctcca atcgggtaac     540
   tcccaggaga gtgtcacaga gcaggacagc aaggacagca cctacagcct cagcagcacc     600
   ctgacgctga gcaaagcaga ctacgagaaa cacaaagtct acgcctgcga agtcacccat     660
   cagggcctga gctcgcccgt cacaaagagc ttcaacaggg gagagtgtta g              711
<210> 49
   <211> 705
   <212> DNA
   <213> Artificial Sequence
<220>
   <223> HEAVY CHAIN MCSP DNA
<400> 49
   atgggatgga gctgtatcat cctcttcttg gtagcaacag ctaccggtgt gcattcggag        60
   gtgcagctgc aggaatctgg ccctggcctg gtcaagccaa gccagagtct gagcctgacc       120
   tgcagcgtga ccggctacag cattaccagc ggctactact ggaactggat tcggcagttc       180
   cccggcaata agctggaatg gatgggctac atcacctacg acggcagcaa caactacaac       240
   cccagcctga agaaccggat cagcatcacc cgggacacca gcaagaacca gttcttcctg       300
   aagctgaaca gcgtgaccac cgaggacacc gccacatact attgcgccga cttcgactac       360
   tggggccagg gcaccaccct gaccgtgtcc agcgccagca caaagggccc tagcgtgttc       420
   cctctggccc ccagcagcaa gagcacaagc ggcggaacag ccgccctggg ctgcctcgtg       480
   aaggactact tccccgagcc cgtgacagtg tcttggaaca gcggagccct gacaagcggc       540
   gtgcacacct tccctgccgt gctgcagagc agcggcctgt actccctgag cagcgtggtc       600
   accgtgccta gcagcagcct gggcacccag acctacatct gcaacgtgaa ccacaagccc       660
   agcaacacca aagtggacaa gaaggtggag cccaagagct gtgat                       705
<210> 50
   <211> 327
   <212> DNA
   <213> Artificial Sequence
<220>
   <223> VL CD3 DNA
<400> 50
   gacatccaga tgacccagag cccctctagc ctgagcgcca gcgtgggcga cagagtgacc        60
   atcacctgtc gggccagcca ggacatcaga aactacctga actggtatca gcagaagccc       120
   ggcaaggccc ccaagctgct gatctactac acctctagac tggaaagcgg cgtgcccagc       180
   cggtttagcg gcagcggctc cggcaccgac tacaccctga ccatcagcag cctgcagccc       240
   gaggacttcg ccacctacta ctgccagcag ggcaacacac tcccctggac cttcggccag       300
   ggcaccaagg tggagatcaa gtccagc                                           327
<210> 51
   <211> 366
   <212> DNA
   <213> Artificial Sequence
<220>
   <223> VH CD3 DNA
<400> 51
   gaggtgcagc tggtcgagag cggaggcggc ctggtgcagc ctggcggcag cctgagactg 60
   agctgcgccg ccagcggcta cagcttcacc ggctacacca tgaactgggt ccggcaggca 120
   cctggcaagg gactggaatg ggtggccctg atcaacccct acaagggcgt gagcacctac 180
   aaccagaagt tcaaggaccg gttcaccatc agcgtggaca agagcaagaa caccgcctat 240
   ctgcagatga acagcctgcg ggccgaggac accgccgtgt actactgcgc cagaagcggc 300
   tactacggcg acagcgactg gtacttcgac gtgtggggcc agggcaccct cgtgaccgtg 360
   tctagc 366
<210> 52
   <211> 318
   <212> DNA
   <213> Artificial Sequence
<220>
   <223> CL CD3 DNA
<400> 52
   gtggctgcac catctgtctt catcttcccg ccatctgatg agcagttgaa atctggaact 60
   gcctctgttg tgtgcctgct gaataacttc tatcccagag aggccaaagt acagtggaag 120
   gtggataacg ccctccaatc gggtaactcc caggagagtg tcacagagca ggacagcaag 180
   gacagcacct acagcctcag cagcaccctg acgctgagca aagcagacta cgagaaacac 240
   aaagtctacg cctgcgaagt cacccatcag ggcctgagct cgcccgtcac aaagagcttc 300
   aacaggggag agtgttga 318
<210> 53
   <211> 306
   <212> DNA
   <213> Artificial Sequence
<220>
   <223> CH1 CD3 DNA
<400> 53
   accaagggcc cctccgtgtt ccccctggcc cccagcagca agagcaccag cggcggcaca 60
   gccgccctcg gctgcctggt caaggactac ttccccgagc ccgtgaccgt gtcctggaac 120
   agcggagccc tgacctccgg cgtgcacacc ttccccgccg tgctgcagag cagcggcctg 180
   tacagcctgt ccagcgtggt caccgtgccc tccagcagcc tgggcaccca gacctacatc 240
   tgcaacgtga accacaagcc cagcaatacc aaggtggaca agaaggtgga gcccaagagc 300
   tgctga 306
<210> 54
   <211> 747
   <212> DNA
   <213> Artificial Sequence
<220>
   <223> LIGHT CHAIN CD3 (VHCL) DNA
<400> 54
   atgggatgga gctgtatcat cctcttcttg gtagcaacag ctaccggtgt gcattccgag        60
   gtgcagctgg tcgagagcgg aggcggcctg gtgcagcctg gcggcagcct gagactgagc       120
   tgcgccgcca gcggctacag cttcaccggc tacaccatga actgggtccg gcaggcacct       180
   ggcaagggac tggaatgggt ggccctgatc aacccctaca agggcgtgag cacctacaac       240
   cagaagttca aggaccggtt caccatcagc gtggacaaga gcaagaacac cgcctatctg       300
   cagatgaaca gcctgcgggc cgaggacacc gccgtgtact actgcgccag aagcggctac       360
   tacggcgaca gcgactggta cttcgacgtg tggggccagg gcaccctcgt gaccgtgtct       420
   agcgctagcg tggctgcacc atctgtcttc atcttcccgc catctgatga gcagttgaaa       480
   tctggaactg cctctgttgt gtgcctgctg aataacttct atcccagaga ggccaaagta       540
   cagtggaagg tggataacgc cctccaatcg ggtaactccc aggagagtgt cacagagcag       600
   gacagcaagg acagcaccta cagcctcagc agcaccctga cgctgagcaa agcagactac       660
   gagaaacaca aagtctacgc ctgcgaagtc acccatcagg gcctgagctc gcccgtcaca       720
   aagagcttca acaggggaga gtgttga                                           747
<210> 55
   <211> 639
   <212> DNA
   <213> Artificial Sequence
<220>
   <223> HEAVY CHAIN (VLCH1) CD3 DNA
<400> 55
   gacatccaga tgacccagag cccctctagc ctgagcgcca gcgtgggcga cagagtgacc      60
   atcacctgtc gggccagcca ggacatcaga aactacctga actggtatca gcagaagccc     120
   ggcaaggccc ccaagctgct gatctactac acctctagac tggaaagcgg cgtgcccagc     180
   cggtttagcg gcagcggctc cggcaccgac tacaccctga ccatcagcag cctgcagccc     240
   gaggacttcg ccacctacta ctgccagcag ggcaacacac tcccctggac cttcggccag     300
   ggcaccaagg tggagatcaa gtccagcgct agcaccaagg gcccctccgt gttccccctg     360
   gcccccagca gcaagagcac cagcggcggc acagccgccc tcggctgcct ggtcaaggac     420
   tacttccccg agcccgtgac cgtgtcctgg aacagcggag ccctgacctc cggcgtgcac     480
   accttccccg ccgtgctgca gagcagcggc ctgtacagcc tgtccagcgt ggtcaccgtg     540
   ccctccagca gcctgggcac ccagacctac atctgcaacg tgaaccacaa gcccagcaat     600
   accaaggtgg acaagaaggt ggagcccaag agctgctga                            639
<210> 56
   <211> 333
   <212> DNA
   <213> Artificial Sequence
<220>
   <223> VL CD3 DNA
<400> 56
   cagaccgtgg tgacacagga acccagcctg accgtctccc ctggcggcac cgtgaccctg      60
   acctgtggaa gcagcacagg cgccgtgacc agcggctact accccaactg ggtgcagcag     120
   aagcccggcc aggcccctag aggactgatc ggcggcacca agtttctggc ccctggcacc     180
   cccgccagat tctctggctc tctgctgggc ggcaaggccg ccctgacact gtctggcgtg     240
   cagcctgagg acgaggccga gtactactgc gccctgtggt acagcaacag atgggtgttc     300
   ggcggaggca ccaagctgac cgtgctgagc agc                                  333
<210> 57
   <211> 375
   <212> DNA
   <213> Artificial Sequence
<220>
   <223> VH CD3 DNA
<400> 57
   gaggtgcagc tggtggaaag cggcggagga ctggtgcagc ctggcggaag cctgaagctg      60
   tcttgcgccg ccagcggctt caccttcaac aaatacgcca tgaactgggt gcgccaggcc     120
   cctggcaagg gactggaatg ggtggcccgg atcagaagca agtacaacaa ctacgccacc     180
   tactacgccg acagcgtgaa ggaccggttc accatcagcc gggacgacag caagaacacc 240
   gcctacctgc agatgaacaa cctgaaaacc gaggacaccg ccgtgtacta ctgcgtgcgg 300
   cacggcaact tcggcaacag ctacatcagc tactgggcct actggggaca gggcaccctg 360
   gtgacagtgt ccagc 375
<210> 58
   <211> 318
   <212> PRT
   <213> Artificial Sequence
<220>
   <223> CL CD3 DNA
<400> 58
   Gly Thr Gly Gly Cys Thr Gly Cys Ala Cys Cys Ala Thr Cys Thr Gly
   1               5                   10                  15
   Thr Cys Thr Thr Cys Ala Thr Cys Thr Thr Cys Cys Cys Gly Cys Cys
               20                  25                  30
   Ala Thr Cys Thr Gly Ala Thr Gly Ala Gly Cys Ala Gly Thr Thr Gly
           35                  40                  45
   Ala Ala Ala Thr Cys Thr Gly Gly Ala Ala Cys Thr Gly Cys Cys Thr
       50                  55                  60
   Cys Thr Gly Thr Thr Gly Thr Gly Thr Gly Cys Cys Thr Gly Cys Thr
   65                  70                  75                  80
   Gly Ala Ala Thr Ala Ala Cys Thr Thr Cys Thr Ala Thr Cys Cys Cys
                   85                  90                  95
   Ala Gly Ala Gly Ala Gly Gly Cys Cys Ala Ala Ala Gly Thr Ala Cys
               100                 105                 110
   Ala Gly Thr Gly Gly Ala Ala Gly Gly Thr Gly Gly Ala Thr Ala Ala
           115                 120                 125
   Cys Gly Cys Cys Cys Thr Cys Cys Ala Ala Thr Cys Gly Gly Gly Thr
       130                 135                 140
   Ala Ala Cys Thr Cys Cys Cys Ala Gly Gly Ala Gly Ala Gly Thr Gly
   145                 150                 155                 160
   Thr Cys Ala Cys Ala Gly Ala Gly Cys Ala Gly Gly Ala Cys Ala Gly
                   165                 170                 175
   Cys Ala Ala Gly Gly Ala Cys Ala Gly Cys Ala Cys Cys Thr Ala Cys
                   180                 185                 190
   Ala Gly Cys Cys Thr Cys Ala Gly Cys Ala Gly Cys Ala Cys Cys Cys
               195                 200                 205
   Thr Gly Ala Cys Gly Cys Thr Gly Ala Gly Cys Ala Ala Ala Gly Cys
       210                 215                 220
   Ala Gly Ala Cys Thr Ala Cys Gly Ala Gly Ala Ala Ala Cys Ala Cys
   225                 230                 235                 240
   Ala Ala Ala Gly Thr Cys Thr Ala Cys Gly Cys Cys Thr Gly Cys Gly
                   245                 250                 255
   Ala Ala Gly Thr Cys Ala Cys Cys Cys Ala Thr Cys Ala Gly Gly Gly
               260                 265                 270
   Cys Cys Thr Gly Ala Gly Cys Thr Cys Gly Cys Cys Cys Gly Thr Cys
           275                 280                 285
   Ala Cys Ala Ala Ala Gly Ala Gly Cys Thr Thr Cys Ala Ala Cys Ala
       290                 295                 300
   Gly Gly Gly Gly Ala Gly Ala Gly Thr Gly Thr Thr Gly Ala
   305                 310                 315
<210> 59
   <211> 306
   <212> DNA
   <213> Artificial Sequence
<220>
   <223> CH1 CD3 DNA
<400> 59
   accaagggcc cctccgtgtt ccccctggcc cccagcagca agagcaccag cggcggcaca       60
   gccgccctcg gctgcctggt caaggactac ttccccgagc ccgtgaccgt gtcctggaac      120
   agcggagccc tgacctccgg cgtgcacacc ttccccgccg tgctgcagag cagcggcctg      180
   tacagcctgt ccagcgtggt caccgtgccc tccagcagcc tgggcaccca gacctacatc      240
   tgcaacgtga accacaagcc cagcaatacc aaggtggaca agaaggtgga gcccaagagc      300
   tgctga                                                                 306
<210> 60
   <211> 756
   <212> DNA
   <213> Artificial Sequence
<220>
   <223> LIGHT CHAIN CD3 (VHCL) DNA
<400> 60
   atgggatgga gctgtatcat cctcttcttg gtagcaacag ctaccggtgt gcattccgag      60
   gtgcagctgg tggaaagcgg cggaggactg gtgcagcctg gcggaagcct gaagctgtct     120
   tgcgccgcca gcggcttcac cttcaacaaa tacgccatga actgggtgcg ccaggcccct     180
   ggcaagggac tggaatgggt ggcccggatc agaagcaagt acaacaacta cgccacctac     240
   tacgccgaca gcgtgaagga ccggttcacc atcagccggg acgacagcaa gaacaccgcc     300
   tacctgcaga tgaacaacct gaaaaccgag gacaccgccg tgtactactg cgtgcggcac     360
   ggcaacttcg gcaacagcta catcagctac tgggcctact ggggacaggg caccctggtg     420
   acagtgtcca gcgctagcgt ggctgcacca tctgtcttca tcttcccgcc atctgatgag     480
   cagttgaaat ctggaactgc ctctgttgtg tgcctgctga ataacttcta tcccagagag     540
   gccaaagtac agtggaaggt ggataacgcc ctccaatcgg gtaactccca ggagagtgtc     600
   acagagcagg acagcaagga cagcacctac agcctcagca gcaccctgac gctgagcaaa     660
   gcagactacg agaaacacaa agtctacgcc tgcgaagtca cccatcaggg cctgagctcg     720
   cccgtcacaa agagcttcaa caggggagag tgttga                               756
<210> 61
   <211> 645
   <212> DNA
   <213> Artificial Sequence
<220>
   <223> HEAVY CHAIN CD3 (VLCH1) DNA
<400> 61
   cagaccgtgg tgacacagga acccagcctg accgtctccc ctggcggcac cgtgaccctg        60
   acctgtggaa gcagcacagg cgccgtgacc agcggctact accccaactg ggtgcagcag       120
   aagcccggcc aggcccctag aggactgatc ggcggcacca agtttctggc ccctggcacc       180
   cccgccagat tctctggctc tctgctgggc ggcaaggccg ccctgacact gtctggcgtg       240
   cagcctgagg acgaggccga gtactactgc gccctgtggt acagcaacag atgggtgttc       300
   ggcggaggca ccaagctgac cgtgctgagc agcgctagca ccaagggccc ctccgtgttc       360
   cccctggccc ccagcagcaa gagcaccagc ggcggcacag ccgccctcgg ctgcctggtc       420
   aaggactact tccccgagcc cgtgaccgtg tcctggaaca gcggagccct gacctccggc       480
   gtgcacacct tccccgccgt gctgcagagc agcggcctgt acagcctgtc cagcgtggtc       540
   accgtgccct ccagcagcct gggcacccag acctacatct gcaacgtgaa ccacaagccc       600
   agcaatacca aggtggacaa gaaggtggag cccaagagct gctga                        645
<210> 62
   <211> 1056
   <212> DNA
   <213> Artificial Sequence
<220>
   <223> FAB(MCSP)-XFAB (CD3) DNA
<400> 62
   atgggatgga gctgtatcat cctcttcttg gtagcaacag ctaccggtgt gcattcggag      60
   gtgcagctgc aggaaagcgg ccctggcctg gtgaaaccca gccagagcct gagcctgacc     120
   tgcagcgtga ccggctacag catcaccagc ggctactact ggaactggat cagacagttc     180
   cccggcaaca agctggaatg gatgggctac atcacctacg acggcagcaa caactacaac     240
   cccagcctga agaacagaat cagcatcacc cgggacacca gcaagaacca gttcttcctg     300
   aagctgaaca gcgtgaccac cgaggacacc gccacctact actgcgccga cttcgactac     360
   tggggccagg gcaccaccct gaccgtgtcc tccgctagca ccaagggacc cagcgtgttc     420
   cccctggcac ccagcagcaa gagcacatct ggcggaacag ccgctctggg ctgtctggtg     480
   aaagactact tccccgagcc cgtgaccgtg tcttggaact ctggcgccct gaccagcggc     540
   gtgcacacct ttccagccgt gctgcagagc agcggcctgt actccctgag cagcgtggtg     600
   acagtgccca gcagcagcct gggaacccag acctacatct gcaacgtgaa ccacaagccc     660
   agcaacacca aggtggacaa gaaggtggaa cccaagagct gcgatggcgg aggaggctcc     720
   ggaggcggag gctctgatat ccagatgacc cagagcccca gctctctgag cgccagcgtg     780
   ggcgacagag tgaccatcac ctgtcgggcc agccaggaca tcagaaacta cctgaactgg     840
   tatcagcaga agcccggcaa ggcccccaag ctgctgatct actacaccag cagactggaa     900
   agcggcgtgc cctccagatt ttccggcagc ggctccggca ccgactacac cctgaccatc     960
   agcagcctgc agcccgagga tttcgccaca tattactgcc agcagggcaa taccctgccc    1020
   tggaccttcg gacagggcac aaaagtggaa atcaag                              1056
<210> 63
   <211> 2052
   <212> DNA
   <213> Artificial Sequence
<220>
   <223> FAB(MCSP)-FAB(MCSP)-XFAB (CD3) DNA
<400> 63
   atgggatgga gctgtatcat cctcttcttg gtagcaacag ctaccggtgt gcattcggag      60
   gtgcagctgc aggaatctgg ccctggcctg gtcaagccaa gccagagtct gagcctgacc     120
   tgcagcgtga ccggctacag cattaccagc ggctactact ggaactggat tcggcagttc     180
   cccggcaata agctggaatg gatgggctac atcacctacg acggcagcaa caactacaac     240
   cccagcctga agaaccggat cagcatcacc cgggacacca gcaagaacca gttcttcctg     300
   aagctgaaca gcgtgaccac cgaggacacc gccacatact attgcgccga cttcgactac     360
   tggggccagg gcaccaccct gaccgtgtcc agcgccagca caaagggccc tagcgtgttc     420
   cctctggccc ccagcagcaa gagcacaagc ggcggaacag ccgccctggg ctgcctcgtg     480
   aaggactact tccccgagcc cgtgacagtg tcttggaaca gcggagccct gacaagcggc 540
   gtgcacacct tccctgccgt gctgcagagc agcggcctgt actccctgag cagcgtggtc 600
   accgtgccta gcagcagcct gggcacccag acctacatct gcaacgtgaa ccacaagccc 660
   agcaacacca aagtggacaa gaaggtggag cccaagagct gtgatggcgg aggagggtcc 720
   ggaggcggtg gctccgaggt gcagctgcag gaatctggcc ctggcctggt caagccaagc 780
   cagagtctga gcctgacctg cagcgtgacc ggctacagca ttaccagcgg ctactactgg 840
   aactggattc ggcagttccc cggcaataag ctggaatgga tgggctacat cacctacgac 900
   ggcagcaaca actacaaccc cagcctgaag aaccggatca gcatcacccg ggacaccagc 960
   aagaaccagt tcttcctgaa gctgaacagc gtgaccaccg aggacaccgc cacatactat 1020
   tgcgccgact tcgactactg gggccagggc accaccctga ccgtgtccag cgccagcaca 1080
   aagggcccta gcgtgttccc tctggccccc agcagcaaga gcacaagcgg cggaacagcc 1140
   gccctgggct gcctcgtgaa ggactacttc cccgagcccg tgacagtgtc ttggaacagc 1200
   ggagccctga caagcggcgt gcacaccttc cctgccgtgc tgcagagcag cggcctgtac 1260
   tccctgagca gcgtggtcac cgtgcctagc agcagcctgg gcacccagac ctacatctgc 1320
   aacgtgaacc acaagcccag caacaccaaa gtggacaaga aggtggagcc caagagctgt 1380
   gatggcggag gagggtccgg cggcggtgga tccgacatcc agatgaccca gagcccctct 1440
   agcctgagcg ccagcgtggg cgacagagtg accatcacct gtcgggccag ccaggacatc 1500
   agaaactacc tgaactggta tcagcagaag cccggcaagg cccccaagct gctgatctac 1560
   tacacctcta gactggaaag cggcgtgccc agccggttta gcggcagcgg ctccggcacc 1620
   gactacaccc tgaccatcag cagcctgcag cccgaggact tcgccaccta ctactgccag 1680
   cagggcaaca cactcccctg gaccttcggc cagggcacca aggtggagat caagtccagc 1740
   gctagcacca agggcccctc cgtgttcccc ctggccccca gcagcaagag caccagcggc 1800
   ggcacagccg ccctcggctg cctggtcaag gactacttcc ccgagcccgt gaccgtgtcc 1860
   tggaacagcg gagccctgac ctccggcgtg cacaccttcc ccgccgtgct gcagagcagc 1920
   ggcctgtaca gcctgtccag cgtggtcacc gtgccctcca gcagcctggg cacccagacc 1980
   tacatctgca acgtgaacca caagcccagc aataccaagg tggacaagaa ggtggagccc 2040
   aagagctgct ga 2052
<210> 64
   <211> 2049
   <212> DNA
   <213> Artificial Sequence
<220>
   <223> FAB(MCSP)-XFAB (CD3)-FAB(MCSP)
<400> 64
   atgggatgga gctgtatcat cctcttcttg gtagcaacag ctaccggtgt gcattcggag      60
   gtgcagctgc aggaaagcgg ccctggcctg gtgaaaccca gccagagcct gagcctgacc     120
   tgcagcgtga ccggctacag catcaccagc ggctactact ggaactggat cagacagttc     180
   cccggcaaca agctggaatg gatgggctac atcacctacg acggcagcaa caactacaac     240
   cccagcctga agaacagaat cagcatcacc cgggacacca gcaagaacca gttcttcctg     300
   aagctgaaca gcgtgaccac cgaggacacc gccacctact actgcgccga cttcgactac     360
   tggggccagg gcaccaccct gaccgtgtcc tccgctagca ccaagggacc cagcgtgttc     420
   cccctggcac ccagcagcaa gagcacatct ggcggaacag ccgctctggg ctgtctggtg     480
   aaagactact tccccgagcc cgtgaccgtg tcttggaact ctggcgccct gaccagcggc     540
   gtgcacacct ttccagccgt gctgcagagc agcggcctgt actccctgag cagcgtggtg     600
   acagtgccca gcagcagcct gggaacccag acctacatct gcaacgtgaa ccacaagccc     660
   agcaacacca aggtggacaa gaaggtggaa cccaagagct gcgatggcgg aggaggctcc     720
   ggaggcggag gctctgatat ccagatgacc cagagcccca gctctctgag cgccagcgtg     780
   ggcgacagag tgaccatcac ctgtcgggcc agccaggaca tcagaaacta cctgaactgg     840
   tatcagcaga agcccggcaa ggcccccaag ctgctgatct actacaccag cagactggaa     900
   agcggcgtgc cctccagatt ttccggcagc ggctccggca ccgactacac cctgaccatc     960
   agcagcctgc agcccgagga tttcgccaca tattactgcc agcagggcaa taccctgccc    1020
   tggaccttcg gacagggcac aaaagtggaa atcaagagca gcgcttccac caaaggccct    1080
   tccgtgtttc ctctggctcc tagctccaag tccacctctg gaggcaccgc tgctctcgga    1140
   tgcctcgtga aggattattt tcctgagcct gtgacagtgt cctggaatag cggagcactg    1200
   acctctggag tgcatacttt ccccgctgtg ctgcagtcct ctggactgta cagcctgagc    1260
   agcgtggtga cagtgcccag cagcagcctg ggcacccaga cctacatctg caacgtgaac    1320
   cacaagccca gcaacaccaa ggtggacaag aaggtggaac ccaagtcttg tggcggaggc    1380
   ggatccggcg gagggggatc tgaggtgcag ctgcaggaaa gcggccctgg cctggtgaaa    1440
   cccagccaga gcctgagcct gacctgcagc gtgaccggct acagcatcac cagcggctac    1500
   tactggaact ggatcagaca gttccccggc aacaagctgg aatggatggg ctacatcacc    1560
   tacgacggca gcaacaacta caaccccagc ctgaagaaca gaatcagcat cacccgggac    1620
   accagcaaga accagttctt cctgaagctg aacagcgtga ccaccgagga caccgccacc    1680
   tactactgcg ccgacttcga ctactggggc cagggcacca ccctgaccgt gtcctccgcc    1740
   tctaccaagg gccccagcgt gttccccctg gcacccagca gcaagagcac atctggcgga    1800
   acagccgctc tgggctgtct ggtgaaagac tacttccccg agcccgtgac cgtgtcttgg    1860
   aactctggcg ccctgaccag cggcgtgcac acctttccag ccgtgctgca gagcagcggc    1920
   ctgtactccc tgtcctccgt ggtcaccgtg ccctctagct ccctgggaac acagacatat    1980
   atctgtaatg tcaatcacaa gccttccaac accaaagtcg ataagaaagt cgagcccaag 2040
   agctgctga 2049
<210> 65
   <211> 2058
   <212> DNA
   <213> Artificial Sequence
<220>
   <223> FAB (MCSP)-FAB(MCSP)-XFAB (CD3) DNA
<400> 65
   atgggatgga gctgtatcat cctcttcttg gtagcaacag ctaccggtgt gcattcggag     60
   gtgcagctgc aggaatctgg ccctggcctg gtcaagccaa gccagagtct gagcctgacc    120
   tgcagcgtga ccggctacag cattaccagc ggctactact ggaactggat tcggcagttc    180
   cccggcaata agctggaatg gatgggctac atcacctacg acggcagcaa caactacaac    240
   cccagcctga agaaccggat cagcatcacc cgggacacca gcaagaacca gttcttcctg    300
   aagctgaaca gcgtgaccac cgaggacacc gccacatact attgcgccga cttcgactac    360
   tggggccagg gcaccaccct gaccgtgtcc agcgccagca caaagggccc tagcgtgttc    420
   cctctggccc ccagcagcaa gagcacaagc ggcggaacag ccgccctggg ctgcctcgtg    480
   aaggactact tccccgagcc cgtgacagtg tcttggaaca gcggagccct gacaagcggc    540
   gtgcacacct tccctgccgt gctgcagagc agcggcctgt actccctgag cagcgtggtc    600
   accgtgccta gcagcagcct gggcacccag acctacatct gcaacgtgaa ccacaagccc    660
   agcaacacca aagtggacaa gaaggtggag cccaagagct gtgatggcgg aggagggtcc    720
   ggaggcggtg gctccgaggt gcagctgcag gaatctggcc ctggcctggt caagccaagc    780
   cagagtctga gcctgacctg cagcgtgacc ggctacagca ttaccagcgg ctactactgg    840
   aactggattc ggcagttccc cggcaataag ctggaatgga tgggctacat cacctacgac    900
   ggcagcaaca actacaaccc cagcctgaag aaccggatca gcatcacccg ggacaccagc    960
   aagaaccagt tcttcctgaa gctgaacagc gtgaccaccg aggacaccgc cacatactat   1020
   tgcgccgact tcgactactg gggccagggc accaccctga ccgtgtccag cgccagcaca   1080
   aagggcccta gcgtgttccc tctggccccc agcagcaaga gcacaagcgg cggaacagcc   1140
   gccctgggct gcctcgtgaa ggactacttc cccgagcccg tgacagtgtc ttggaacagc   1200
   ggagccctga caagcggcgt gcacaccttc cctgccgtgc tgcagagcag cggcctgtac   1260
   tccctgagca gcgtggtcac cgtgcctagc agcagcctgg gcacccagac ctacatctgc   1320
   aacgtgaacc acaagcccag caacaccaaa gtggacaaga aggtggagcc caagagctgt   1380
   gatggcggag gagggtccgg cggcggtgga tcccagaccg tggtgacaca ggaacccagc   1440
   ctgaccgtct cccctggcgg caccgtgacc ctgacctgtg gaagcagcac aggcgccgtg   1500
   accagcggct actaccccaa ctgggtgcag cagaagcccg gccaggcccc tagaggactg 1560
   atcggcggca ccaagtttct ggcccctggc acccccgcca gattctctgg ctctctgctg 1620
   ggcggcaagg ccgccctgac actgtctggc gtgcagcctg aggacgaggc cgagtactac 1680
   tgcgccctgt ggtacagcaa cagatgggtg ttcggcggag gcaccaagct gaccgtgctg 1740
   agcagcgcta gcaccaaggg cccctccgtg ttccccctgg cccccagcag caagagcacc 1800
   agcggcggca cagccgccct cggctgcctg gtcaaggact acttccccga gcccgtgacc 1860
   gtgtcctgga acagcggagc cctgacctcc ggcgtgcaca ccttccccgc cgtgctgcag 1920
   agcagcggcc tgtacagcct gtccagcgtg gtcaccgtgc cctccagcag cctgggcacc 1980
   cagacctaca tctgcaacgt gaaccacaag cccagcaata ccaaggtgga caagaaggtg 2040
   gagcccaaga gctgctga 2058
<210> 66
   <211> 729
   <212> DNA
   <213> Artificial Sequence
<220>
   <223> LIGHT CHAIN CD3 (VHCL) DNA
<400> 66
   atgggatgga gctgtatcat cctcttcttg gtagcaacag ctaccggtgt gcattccgag 60
   gtgcagctgg tggaaagcgg cggaggcctg gtgcagcccg gcaagagcct gaagctgagc 120
   tgcgaggcca gcggcttcac cttcagcggc tacggcatgc actgggtgag acaggcccct 180
   ggcagaggac tggaaagcgt ggcctacatc accagcagca gcatcaacat taagtacgcc 240
   gacgccgtga agggccggtt caccgtgtcc agggataacg ccaagaacct gctgttcctg 300
   cagatgaaca tcctgaagtc cgaggacacc gctatgtatt actgcgccag attcgactgg 360
   gacaagaact actggggcca gggcaccatg gtcacagtgt ctagcgctag cgtggctgca 420
   ccatctgtct tcatcttccc gccatctgat gagcagttga aatctggaac tgcctctgtt 480
   gtgtgcctgc tgaataactt ctatcccaga gaggccaaag tacagtggaa ggtggataac 540
   gccctccaat cgggtaactc ccaggagagt gtcacagagc aggacagcaa ggacagcacc 600
   tacagcctca gcagcaccct gacgctgagc aaagcagact acgagaaaca caaagtctac 660
   gcctgcgaag tcacccatca gggcctgagc tcgcccgtca caaagagctt caacagggga 720
   gagtgttga 729
<210> 67
   <211> 2052
   <212> DNA
   <213> Artificial Sequence
<220>
   <223> XFAB (CD3)-FAB(MCSP)-FAB(MCSP) DNA
<400> 67
   atgggatgga gctgtatcat cctcttcttg gtagcaacag ctaccggtgt gcattccgac      60
   atccagatga cccagagccc cagcagcctg cctgccagcc tgggcgacag agtgaccatc     120
   aactgccagg ccagccagga catcagcaac tacctgaact ggtatcagca gaagcctggc     180
   aaggccccca agctgctgat ctactacacc aacaagctgg ccgacggcgt gcccagcaga     240
   ttcagcggca gcggctccgg cagagacagc agcttcacca tctccagcct ggaaagcgag     300
   gacatcggca gctactactg ccagcagtac tacaactacc cctggacctt cggccctggc     360
   accaagctgg aaatcaagag cagcgcttcc accaaaggcc cttccgtgtt tcctctggct     420
   cctagctcca agtccacctc tggaggcacc gctgctctcg gatgcctcgt gaaggattat     480
   tttcctgagc ctgtgacagt gtcctggaat agcggagcac tgacctctgg agtgcatact     540
   ttccccgctg tgctgcagtc ctctggactg tacagcctga gcagcgtggt gacagtgccc     600
   agcagcagcc tgggcaccca gacctacatc tgcaacgtga accacaagcc cagcaacacc     660
   aaggtggaca agaaggtgga acccaagtct tgtggcggag gcggatccgg cggaggaggg     720
   tccgaggtgc agctgcagga atctggccct ggcctggtca agccaagcca gagtctgagc     780
   ctgacctgca gcgtgaccgg ctacagcatt accagcggct actactggaa ctggattcgg     840
   cagttccccg gcaataagct ggaatggatg ggctacatca cctacgacgg cagcaacaac     900
   tacaacccca gcctgaagaa ccggatcagc atcacccggg acaccagcaa gaaccagttc     960
   ttcctgaagc tgaacagcgt gaccaccgag gacaccgcca catactattg cgccgacttc    1020
   gactactggg gccagggcac caccctgacc gtgtccagcg ccagcacaaa gggccctagc    1080
   gtgttccctc tggcccccag cagcaagagc acaagcggcg gaacagccgc cctgggctgc    1140
   ctcgtgaagg actacttccc cgagcccgtg acagtgtctt ggaacagcgg agccctgaca    1200
   agcggcgtgc acaccttccc tgccgtgctg cagagcagcg gcctgtactc cctgagcagc    1260
   gtggtcaccg tgcctagcag cagcctgggc acccagacct acatctgcaa cgtgaaccac    1320
   aagcccagca acaccaaagt ggacaagaag gtggagccca agagctgtga tggcggagga    1380
   gggtccggag gcggtggctc cgaggtgcag ctgcaggaat ctggccctgg cctggtcaag    1440
   ccaagccaga gtctgagcct gacctgcagc gtgaccggct acagcattac cagcggctac    1500
   tactggaact ggattcggca gttccccggc aataagctgg aatggatggg ctacatcacc    1560
   tacgacggca gcaacaacta caaccccagc ctgaagaacc ggatcagcat cacccgggac    1620
   accagcaaga accagttctt cctgaagctg aacagcgtga ccaccgagga caccgccaca    1680
   tactattgcg ccgacttcga ctactggggc cagggcacca ccctgaccgt gtccagcgcc    1740
   agcacaaagg gccctagcgt gttccctctg gcccccagca gcaagagcac aagcggcgga    1800
   acagccgccc tgggctgcct cgtgaaggac tacttccccg agcccgtgac agtgtcttgg    1860
   aacagcggag ccctgacaag cggcgtgcac accttccctg ccgtgctgca gagcagcggc    1920
   ctgtactccc tgagcagcgt ggtcaccgtg cctagcagca gcctgggcac ccagacctac 1980
   atctgcaacg tgaaccacaa gcccagcaac accaaagtgg acaagaaggt ggagcccaag 2040
   agctgtgatt ga 2052
<210> 68
   <211> 5
   <212> PRT
   <213> Artificial Sequence
<220>
   <223> GA201 HCDR1
<400> 68
   Asp Tyr Lys Ile His
   1 5
<210> 69
   <211> 17
   <212> PRT
   <213> Artificial Sequence
<220>
   <223> GA201 HCDR2
<400> 69
   Tyr Phe Asn Pro Asn Ser Gly Tyr Ser Thr Tyr Ala Gln Lys Phe Gln
   1 5 10 15
   Gly
<210> 70
   <211> 11
   <212> PRT
   <213> Artificial Sequence
<220>
   <223> GA201 HCDR3
<400> 70
   Leu Ser Pro Gly Gly Tyr Tyr Val Met Asp Ala
   1 5 10
<210> 71
   <211> 11
   <212> PRT
   <213> Artificial Sequence
<220>
   <223> GA201 LCDR1
<400> 71
   Arg Ala Ser Gln Gly Ile Asn Asn Tyr Leu Asn
   1 5 10
<210> 72
   <211> 7
   <212> PRT
   <213> Artificial Sequence
<220>
   <223> GA201 LCDR2
<400> 72
   Asn Thr Asn Asn Leu Gln Thr
   1 5
<210> 73
   <211> 8
   <212> PRT
   <213> Artificial Sequence
<220>
   <223> GA201 LCDR3
<400> 73
   Leu Gln His Asn Ser Phe Pro Thr
   1 5
<210> 74
   <211> 120
   <212> PRT
   <213> Artificial Sequence
<220>
   <223> GA201 VH
<400> 74
<210> 75
   <211> 106
   <212> PRT
   <213> Artificial Sequence
<220>
   <223> GA201 VL
<400> 75
<210> 76
   <211> 5
   <212> PRT
   <213> Artificial Sequence
<220>
   <223> 3F2 HCDR1
<400> 76
   Ser Tyr Ala Met Ser
   1 5
<210> 77
   <211> 16
   <212> PRT
   <213> Artificial Sequence
<220>
   <223> 3F2 HCDR2
<400> 77
   Ala Ile Ser Gly Ser Gly Gly Ser Thr Tyr Tyr Ala Asp Ser Val Lys
   1 5 10 15
<210> 78
   <211> 8
   <212> PRT
   <213> Artificial Sequence
<220>
   <223> 3F2 HCDR3
<400> 78
   Tyr Cys Ala Lys Gly Trp Phe Gly
   1               5
<210> 79
   <211> 11
   <212> PRT
   <213> Artificial Sequence
<220>
   <223> 3F2 LCDR1
<400> 79
   Arg Ala Ser Gln Ser Val Thr Ser Ser Tyr Leu
   1               5                   10
<210> 80
   <211> 7
   <212> PRT
   <213> Artificial Sequence
<220>
   <223> 3F2 LCDR2
<400> 80
   Asn Val Gly Ser Arg Arg Ala
   1               5
<210> 81
   <211> 9
   <212> PRT
   <213> Artificial Sequence
<220>
   <223> 3F2 LCDR3
<400> 81
   Cys Gln Gln Gly Ile Met Leu Pro Pro
   1               5
<210> 82
   <211> 117
   <212> PRT
   <213> Artificial Sequence
<220>
   <223> 3F2 VH
<400> 82
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Phe Gly Gly Phe Asn Tyr Trp Gly Gln Gly Thr Leu
   100 105 110
   Val Thr Val Ser Ser
   115
<210> 83
   <211> 108
   <212> PRT
   <213> Artificial Sequence
<220>
   <223> 3F2 VL
<400> 83
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Thr Ser Ser
   20 25 30
   Tyr Leu Ala Trp Tyr Gln Gln Lys Pro Gly Gln Ala Pro Arg Leu Leu
   35 40 45
   Ile Asn Val Gly Ser Arg Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Ile Met Leu Pro
   85 90 95
   Pro Thr Phe Gly Gln Gly Thr Lys Val Glu Ile Lys
   100 105
<210> 84
   <211> 5
   <212> PRT
   <213> Artificial Sequence
<220>
   <223> CH1A1A HCDR1
<400> 84
   Glu Phe Gly Met Asn
   1 5
<210> 85
   <211> 17
   <212> PRT
   <213> Artificial Sequence
<220>
   <223> CH1A1A HCDR2
<400> 85
   Trp Ile Asn Thr Lys Thr Gly Glu Ala Thr Tyr Val Glu Glu Phe Lys
   1 5 10 15
   Gly
<210> 86
   <211> 12
   <212> PRT
   <213> Artificial Sequence
<220>
   <223> CH1A1A HCDR3
<400> 86
   Trp Asp Phe Ala Tyr Tyr Val Glu Ala Met Asp Tyr
   1 5 10
<210> 87
   <211> 11
   <212> PRT
   <213> Artificial Sequence
<220>
   <223> CH1A1A LCDR1
<400> 87
   Lys Ala Ser Ala Ala Val Gly Thr Tyr Val Ala
   1               5                   10
<210> 88
   <211> 7
   <212> PRT
   <213> Artificial Sequence
<220>
   <223> CH1A1A LCDR2
<400> 88
   Ser Ala Ser Tyr Arg Lys Arg
   1               5
<210> 89
   <211> 10
   <212> PRT
   <213> Artificial Sequence
<220>
   <223> CH1A1A LCDR3
<400> 89
   His Gln Tyr Tyr Thr Tyr Pro Leu Phe Thr
   1               5                   10
<210> 90
   <211> 121
   <212> PRT
   <213> Artificial Sequence
<220>
   <223> CH1A1A VH
<400> 90
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Glu Phe
   20 25 30
   Gly Met Asn Trp Val Arg Gln Ala Pro Gly Gln Gly Leu Glu Trp Met
   35 40 45
   Gly Trp Ile Asn Thr Lys Thr Gly Glu Ala Thr Tyr Val Glu Glu Phe
   50 55 60
   Lys Gly Arg Val Thr Phe Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Trp Asp Phe Ala Tyr Tyr Val Glu Ala Met Asp Tyr Trp Gly
   100 105 110
   Gln Gly Thr Thr Val Thr Val Ser Ser
   115 120
<210> 91
   <211> 108
   <212> PRT
   <213> Artificial Sequence
<220>
   <223> CH1A1A VL
<400> 91
   Asp Ile Gln Met Thr Gln Ser Pro Ser Ser Leu Ser Ala Ser Val Gly
   1 5 10 15
   Asp Arg Val Thr Ile Thr Cys Lys Ala Ser Ala Ala Val Gly Thr Tyr
   20 25 30
   Val Ala Trp Tyr Gln Gln Lys Pro Gly Lys Ala Pro Lys Leu Leu Ile
   35 40 45
   Tyr Ser Ala Ser Tyr Arg Lys Arg Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys His Gln Tyr Tyr Thr Tyr Pro Leu
   85 90 95
   Phe Thr Phe Gly Gln Gly Thr Lys Leu Glu Ile Lys
   100 105
<210> 92
   <211> 10
   <212> PRT
   <213> Artificial Sequence
<220>
   <223> Anti-CD33 HCDR1
<400> 92
   Gly Tyr Thr Ile Thr Asp Ser Asn Ile His
   1 5 10
<210> 93
   <211> 13
   <212> PRT
   <213> Artificial Sequence
<220>
   <223> Anti-CD33 HCDR2
<400> 93
   Tyr Ile Tyr Pro Tyr Asn Gly Gly Thr Asp Tyr Asn Gln
   1 5 10
<210> 94
   <211> 7
   <212> PRT
   <213> Artificial Sequence
<220>
   <223> Anti-CD33 HCDR3
<400> 94
   Gly Asn Pro Trp Leu Ala Tyr
   1 5
<210> 95
   <211> 15
   <212> PRT
   <213> Artificial Sequence
<220>
   <223> Anti-CD33 LCDR1
<400> 95
   Arg Ala Ser Glu Ser Leu Asp Asn Tyr Gly Ile Arg Phe Leu Thr
   1 5 10 15
<210> 96
   <211> 7
   <212> PRT
   <213> Artificial Sequence
<220>
   <223> Anti-CD33 LCDR2
<400> 96
   Ala Ala Ser Asn Gln Gly Ser
   1 5
<210> 97
   <211> 9
   <212> PRT
   <213> Artificial Sequence
<220>
   <223> Anti-CD33 LCDR3
<400> 97
   Gln Gln Thr Lys Glu Val Pro Trp Ser
   1 5
<210> 98
   <211> 116
   <212> PRT
   <213> Artificial Sequence
<220>
   <223> Anti-CD33 VH
<400> 98
<210> 99
   <211> 111
   <212> PRT
   <213> Artificial Sequence
<220>
   <223> Anti-CD33 VL
<400> 99
<210> 100
   <211> 218
   <212> PRT
   <213> Artificial Sequence
<220>
   <223> Light Chain antiCD33
<400> 100
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Glu Ser Val Asp Asn Tyr
               20                  25                  30
   Gly Ile Ser Phe Met Asn Trp Phe Gln Gln Lys Pro Gly Lys Ala Pro
           35                  40                  45
   Lys Leu Leu Ile Tyr Ala Ala Ser Asn Gln Gly Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Asp Asp Phe Ala Thr Tyr Tyr Cys Gln Gln Ser Lys
                   85                  90                  95
   Glu Val Pro Trp Thr Phe Gly Gln Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 101
   <211> 229
   <212> PRT
   <213> Artificial Sequence
<220>
   <223> Light Chain V9 (VH-CL)
<400> 101
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Gln Gly Thr Leu Val Thr Val Ser Ser Ala Ser Val Ala Ala Pro
           115                 120                 125
   Ser Val Phe Ile Phe Pro Pro Ser Asp Glu Gln Leu Lys Ser Gly Thr
   130 135 140
   Ala Ser Val Val Cys Leu Leu Asn Asn Phe Tyr Pro Arg Glu Ala Lys
   145 150 155 160
   Val Gln Trp Lys Val Asp Asn Ala Leu Gln Ser Gly Asn Ser Gln Glu
   165 170 175
   Ser Val Thr Glu Gln Asp Ser Lys Asp Ser Thr Tyr Ser Leu Ser Ser
   180 185 190
   Thr Leu Thr Leu Ser Lys Ala Asp Tyr Glu Lys His Lys Val Tyr Ala
   195 200 205
   Cys Glu Val Thr His Gln Gly Leu Ser Ser Pro Val Thr Lys Ser Phe
   210 215 220
   Asn Arg Gly Glu Cys
   225
<210> 102
   <211> 396
   <212> PRT
   <213> Artificial Sequence
<220>
   <223> CD33 Fab-Crossfab (VH-CH1-VL-CH1)
<400> 102
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Thr Phe Thr Asp Tyr
               20                  25                  30
   Asn Met His Trp Val Arg Gln Ala Pro Gly Gln Gly Leu Glu Trp Ile
           35                  40                  45
   Gly Tyr Ile Tyr Pro Tyr Asn Gly Gly Thr Gly Tyr Asn Gln Lys Phe
       50                  55                  60
   Lys Ser Lys Ala Thr Ile Thr Ala Asp Glu Ser Thr Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Arg Pro Ala Met Asp Tyr Trp Gly Gln Gly Thr Leu Val
               100                 105                 110
   Thr Val Ser Ser Ala Ser Thr Lys Gly Pro Ser Val Phe Pro Leu Ala
   115 120 125
   Pro Ser Ser Lys Ser Thr Ser Gly Gly Thr Ala Ala Leu Gly Cys Leu
   130 135 140
   Val Lys Asp Tyr Phe Pro Glu Pro Val Thr Val Ser Trp Asn Ser Gly
   145 150 155 160
   Ala Leu Thr Ser Gly Val His Thr Phe Pro Ala Val Leu Gln Ser Ser
   165 170 175
   Gly Leu Tyr Ser Leu Ser Ser Val Val Thr Val Pro Ser Ser Ser Leu
   180 185 190
   Gly Thr Gln Thr Tyr Ile Cys Asn Val Asn His Lys Pro Ser Asn Thr
   195 200 205
   Lys Val Asp Lys Lys Val Glu Pro Lys Ser Cys Asp Gly Gly Gly Gly
   210 215 220
   Ser Gly Gly Gly Gly Ser Asp Ile Gln Met Thr Gln Ser Pro Ser Ser
   225 230 235 240
   Leu Ser Ala Ser Val Gly Asp Arg Val Thr Ile Thr Cys Arg Ala Ser
   245 250 255
   Gln Asp Ile Arg Asn Tyr Leu Asn Trp Tyr Gln Gln Lys Pro Gly Lys
   260 265 270
   Ala Pro Lys Leu Leu Ile Tyr Tyr Thr Ser Arg Leu Glu Ser Gly Val
   275 280 285
   Pro Ser Arg Phe Ser Gly Ser Gly Ser Gly Thr Asp Tyr Thr Leu Thr
   290 295 300
   Ile Ser Ser Leu Gln Pro Glu Asp Phe Ala Thr Tyr Tyr Cys Gln Gln
   305 310 315 320
   Gly Asn Thr Leu Pro Trp Thr Phe Gly Gln Gly Thr Lys Val Glu Ile
   325 330 335
   Lys Ser Ser Ala Ser Thr Lys Gly Pro Ser Val Phe Pro Leu Ala Pro
   340 345 350
   Ser Ser Lys Ser Thr Ser Gly Gly Thr Ala Ala Leu Gly Cys Leu Val
   355 360 365
   Lys Asp Tyr Phe Pro Glu Pro Val Thr Val Ser Trp Asn Ser Gly Ala
   370 375 380
   Leu Thr Ser Gly Val His Thr Phe Pro Ala Val Leu
   385 390 395
<210> 103
   <211> 105
   <212> PRT
   <213> Homo sapiens
<400> 103
   Gln Asp Gly Asn Glu Glu Met Gly Gly Ile Thr Gln Thr Pro Tyr Lys
   1 5 10 15
   Val Ser Ile Ser Gly Thr Thr Val Ile Leu Thr Cys Pro Gln Tyr Pro
   20 25 30
   Gly Ser Glu Ile Leu Trp Gln His Asn Asp Lys Asn Ile Gly Gly Asp
   35 40 45
   Glu Asp Asp Lys Asn Ile Gly Ser Asp Glu Asp His Leu Ser Leu Lys
   50 55 60
   Glu Phe Ser Glu Leu Glu Gln Ser Gly Tyr Tyr Val Cys Tyr Pro Arg
   65 70 75 80
   Gly Ser Lys Pro Glu Asp Ala Asn Phe Tyr Leu Tyr Leu Arg Ala Arg
   85 90 95
   Val Cys Glu Asn Cys Met Glu Met Asp
   100 105
<210> 104
   <211> 714
   <212> DNA
   <213> Artificial Sequence
<220>
   <223> Light Chain antiCD33 DNA
<400> 104
   atgggatgga gctgtatcat cctcttcttg gtagcaacag ctaccggtgt gcattccgac        60
   atccagatga cccagagccc cagcagcctg agcgccagcg tgggcgacag agtgaccatc       120
   acctgtcggg ccagcgagag cgtggacaac tacggcatca gcttcatgaa ctggttccag       180
   cagaagcccg gcaaggcccc caagctgctg atctacgccg ccagcaatca gggcagcggc       240
   gtgcccagca gattcagcgg ctctggcagc ggcaccgact tcaccctgac catcagcagc       300
   ctgcagcccg acgacttcgc cacctactac tgccagcaga gcaaagaggt gccctggacc       360
   ttcggccagg gcaccaaggt ggaaatcaag cgtacggtgg ctgcaccatc tgtcttcatc       420
   ttcccgccat ctgatgagca gttgaaatct ggaactgcct ctgttgtgtg cctgctgaat       480
   aacttctatc ccagagaggc caaagtacag tggaaggtgg ataacgccct ccaatcgggt       540
   aactcccagg agagtgtcac agagcaggac agcaaggaca gcacctacag cctcagcagc       600
   accctgacgc tgagcaaagc agactacgag aaacacaaag tctacgcctg cgaagtcacc       660
   catcagggcc tgagctcgcc cgtcacaaag agcttcaaca ggggagagtg ttag             714
<210> 105
   <211> 747
   <212> DNA
   <213> Artificial Sequence
<220>
   <223> Light Chain V9 (VH-CL) DNA
<400> 105
   atgggatgga gctgtatcat cctcttcttg gtagcaacag ctaccggtgt gcattccgag        60
   gtgcagctgg tcgagagcgg aggcggcctg gtgcagcctg gcggcagcct gagactgagc       120
   tgcgccgcca gcggctacag cttcaccggc tacaccatga actgggtccg gcaggcacct       180
   ggcaagggac tggaatgggt ggccctgatc aacccctaca agggcgtgag cacctacaac       240
   cagaagttca aggaccggtt caccatcagc gtggacaaga gcaagaacac cgcctatctg       300
   cagatgaaca gcctgcgggc cgaggacacc gccgtgtact actgcgccag aagcggctac       360
   tacggcgaca gcgactggta cttcgacgtg tggggccagg gcaccctcgt gaccgtgtct       420
   agcgctagcg tggctgcacc atctgtcttc atcttcccgc catctgatga gcagttgaaa       480
   tctggaactg cctctgttgt gtgcctgctg aataacttct atcccagaga ggccaaagta       540
   cagtggaagg tggataacgc cctccaatcg ggtaactccc aggagagtgt cacagagcag       600
   gacagcaagg acagcaccta cagcctcagc agcaccctga cgctgagcaa agcagactac       660
   gagaaacaca aagtctacgc ctgcgaagtc acccatcagg gcctgagctc gcccgtcaca       720
   aagagcttca acaggggaga gtgttga                                           747
<210> 106
   <211> 1386
   <212> DNA
   <213> Artificial Sequence
<220>
   <223> CD33 Fab-Crossfab (VH-CH1-VL-CH1) DNA
<400> 106
   atgggatgga gctgtatcat cctcttcttg gtagcaacag ctaccggtgt gcattcccag      60
   gtgcagctgg tgcagtctgg cgccgaagtg aagaaacccg gcagcagcgt gaaggtgtcc     120
   tgcaaggcca gcggctacac cttcaccgac tacaacatgc actgggtccg ccaggcccca     180
   ggccagggac tggaatggat cggctacatc tacccctaca acggcggcac cggctacaac     240
   cagaagttca agagcaaggc caccatcacc gccgacgaga gcaccaacac cgcctacatg     300
   gaactgagca gcctgcggag cgaggacacc gccgtgtact actgcgccag aggcagaccc     360
   gccatggact actggggcca gggcaccctg gtgacagtgt ccagcgccag cacaaagggc     420
   cctagcgtgt tccctctggc ccccagcagc aagagcacaa gcggcggaac agccgccctg     480
   ggctgcctcg tgaaggacta cttccccgag cccgtgacag tgtcttggaa cagcggagcc     540
   ctgacaagcg gcgtgcacac cttccctgcc gtgctgcaga gcagcggcct gtactccctg     600
   agcagcgtgg tcaccgtgcc tagcagcagc ctgggcaccc agacctacat ctgcaacgtg     660
   aaccacaagc ccagcaacac caaagtggac aagaaggtgg agcccaagag ctgtgatggc     720
   ggaggagggt ccggaggcgg tggatccgac atccagatga cccagagccc ctctagcctg     780
   agcgccagcg tgggcgacag agtgaccatc acctgtcggg ccagccagga catcagaaac     840
   tacctgaact ggtatcagca gaagcccggc aaggccccca agctgctgat ctactacacc     900
   tctagactgg aaagcggcgt gcccagccgg tttagcggca gcggctccgg caccgactac     960
   accctgacca tcagcagcct gcagcccgag gacttcgcca cctactactg ccagcagggc    1020
   aacacactcc cctggacctt cggccagggc accaaggtgg agatcaagtc cagcgctagc    1080
   accaagggcc cctccgtgtt ccccctggcc cccagcagca agagcaccag cggcggcaca    1140
   gccgccctcg gctgcctggt caaggactac ttccccgagc ccgtgaccgt gtcctggaac    1200
   agcggagccc tgacctccgg cgtgcacacc ttccccgccg tgctgcagag cagcggcctg    1260
   tacagcctgt ccagcgtggt caccgtgccc tccagcagcc tgggcaccca gacctacatc    1320
   tgcaacgtga accacaagcc cagcaatacc aaggtggaca agaaggtgga gcccaagagc    1380
   tgctga                                                               1386
<210> 107
   <211> 33
   <212> DNA
   <213> Artificial Sequence
<220>
   <223> MCSP CDR1 VH DNA
<400> 107
   ggctactcca tcaccagtgg ttattactgg aac 33
<210> 108
   <211> 48
   <212> DNA
   <213> Artificial Sequence
<220>
   <223> MCSP CDR2 VH
<400> 108
   tacataacct acgacggtag caataactac aacccatctc tcaaaaat 48
<210> 109
   <211> 9
   <212> DNA
   <213> Artificial Sequence
<220>
   <223> MCSP CDR3 VH
<400> 109
   tttgactac 9
<210> 110
   <211> 33
   <212> DNA
   <213> Artificial Sequence
<220>
   <223> MCSP CDR1 VL DNA
<400> 110
   agtgcaagtc agggcattag aaattattta aac 33
<210> 111
   <211> 21
   <212> DNA
   <213> Artificial Sequence
<220>
   <223> MCSP CDR2 VL
<400> 111
   tacacatcaa gtttacactc a 21
<210> 112
   <211> 27
   <212> DNA
   <213> Artificial Sequence
<220>
   <223> MCSP CDR3 VL
<400> 112
   cagcagtata gtaagcttcc ttggacg 27
<210> 113
   <211> 15
   <212> DNA
   <213> Artificial Sequence
<220>
   <223> GA201 CDR1 VH
<400> 113
   gactacaaga tacac 15
<210> 114
   <211> 51
   <212> DNA
   <213> Artificial Sequence
<220>
   <223> GA201 CDR2 VH
<400> 114
   tatttcaacc ctaacagcgg ttatagtacc tacgcacaga agttccaggg c 51
<210> 115
   <211> 33
   <212> DNA
   <213> Artificial Sequence
<220>
   <223> GA201 CDR3 VH
<400> 115
   ctatccccag gcggttacta tgttatggat gcc 33
<210> 116
   <211> 33
   <212> DNA
   <213> Artificial Sequence
<220>
   <223> GA201 CDR1 VL
<400> 116
   cgggcaagtc agggcattaa caattactta aat 33
<210> 117
   <211> 21
   <212> DNA
   <213> Artificial Sequence
<220>
   <223> GA201 CDR2 VL
<400> 117
   aataccaaca acttgcagac a 21
<210> 118
   <211> 24
   <212> DNA
   <213> Artificial Sequence
<220>
   <223> GA201 CDR3 VL
<400> 118
   ttgcagcata atagttttcc cacg 24
<210> 119
   <211> 360
   <212> DNA
   <213> Artificial Sequence
<220>
   <223> GA201 VH
<400> 119
   caggtgcagc tggtgcagtc tggggctgag gtgaagaagc ctgggtcctc ggtgaaggtc     60
   tcctgcaagg cctctggttt cacattcact gactacaaga tacactgggt gcgacaggcc    120
   cctggacaag ggctcgagtg gatgggatat ttcaacccta acagcggtta tagtacctac    180
   gcacagaagt tccagggcag ggtcaccatt accgcggaca aatccacgag cacagcctac    240
   atggagctga gcagcctgag atctgaggac acggccgtgt attactgtgc gagactatcc    300
   ccaggcggtt actatgttat ggatgcctgg ggccaaggga ccaccgtgac cgtctcctca    360
<210> 120
   <211> 318
   <212> DNA
   <213> Artificial Sequence
<220>
   <223> GA201 VL
<400> 120
   gatatccaga tgacccagtc tccatcctcc ctgtctgcat ctgtcggaga ccgggtcacc     60
   atcacctgcc gggcaagtca gggcattaac aattacttaa attggtacca gcagaagcca    120
   gggaaagccc ctaagcgcct gatctataat accaacaact tgcagacagg cgtcccatca    180
   aggttcagcg gcagtggatc cgggacagaa ttcactctca ccatcagcag cctgcagcct    240
   gaagattttg ccacctatta ctgcttgcag cataatagtt ttcccacgtt tggccagggc    300
   accaagctcg agatcaag                                                  318
<210> 121
   <211> 15
   <212> DNA
   <213> Artificial Sequence
<220>
   <223> 3F2 CDR1 VH
<400> 121
   agctacgcca tgagc 15
<210> 122
   <211> 48
   <212> DNA
   <213> Artificial Sequence
<220>
   <223> 3F2 CDR2 VH
<400> 122
   gccatctccg gcagcggagg cagcacctac tacgccgaca gcgtgaag 48
<210> 123
   <211> 24
   <212> DNA
   <213> Artificial Sequence
<220>
   <223> 3F2 CDR3 VH
<400> 123
   tattgcgcca agggatggtt cggc 24
<210> 124
   <211> 33
   <212> DNA
   <213> Artificial Sequence
<220>
   <223> 3F2 CDR1 VL
<400> 124
   agagccagcc agagcgtgac cagcagctac ctg 33
<210> 125
   <211> 21
   <212> DNA
   <213> Artificial Sequence
<220>
   <223> 3F2 CDR2 VL
<400> 125
   aacgtgggca gcagacgggc c 21
<210> 126
   <211> 27
   <212> DNA
   <213> Artificial Sequence
<220>
   <223> 3F2 CDR3 VL
<400> 126
   tgccagcagg gcatcatgct gcccccc 27
<210> 127
   <211> 351
   <212> DNA
   <213> Artificial Sequence
<220>
   <223> 3F2 VH
<400> 127
   gaggtgcagc tgctggaatc tggaggcggc ctggtgcagc ctggcggcag cctgagactg       60
   tcttgcgccg ccagcggctt caccttcagc agctacgcca tgagctgggt ccgacaggct      120
   cctggcaagg gactggaatg ggtgtccgcc atctccggca gcggaggcag cacctactac      180
   gccgacagcg tgaagggccg gttcaccatc agcagagaca acagcaagaa caccctgtac      240
   ctgcagatga acagcctgcg ggccgaggat accgccgtgt attattgcgc caagggatgg      300
   ttcggcggct tcaactactg gggccaggga accctggtga cagtgtccag c               351
<210> 128
   <211> 324
   <212> DNA
   <213> Artificial Sequence
<220>
   <223> 3F2 VL
<400> 128
   gagatcgtgc tgacccagtc tcccggcacc ctgagcctga gccctggcga gagagccacc       60
   ctgagctgca gagccagcca gagcgtgacc agcagctacc tggcctggta tcagcagaag      120
   cccggccagg cccccagact gctgatcaac gtgggcagca gacgggccac cggcatcccc      180
   gatagattca gcggcagcgg ctccggcacc gacttcaccc tgaccatcag ccggctggaa      240
   cccgaggact tcgccgtgta ctactgccag cagggcatca tgctgccccc caccttcggc      300
   cagggcacca aggtggaaat caag                                             324
<210> 129
   <211> 15
   <212> DNA
   <213> Artificial Sequence
<220>
   <223> CH1A1A CDR1 VH
<400> 129
   gagttcggca tgaac 15
<210> 130
   <211> 51
   <212> DNA
   <213> Artificial Sequence
<220>
   <223> CH1A1A CDR2 VH
<400> 130
   tggatcaaca ccaagaccgg cgaggccacc tacgtggaag agttcaaggg c 51
<210> 131
   <211> 36
   <212> DNA
   <213> Artificial Sequence
<220>
   <223> CH1A1A CDR3 VH
<400> 131
   tgggacttcg cctattacgt ggaagccatg gactac 36
<210> 132
   <211> 33
   <212> DNA
   <213> Artificial Sequence
<220>
   <223> CH1A1A CDR1 VL
<400> 132
   aaggccagtg cggctgtggg tacgtatgtt gcg 33
<210> 133
   <211> 21
   <212> DNA
   <213> Artificial Sequence
<220>
   <223> CH1A1A CDR2 VL
<400> 133
   tcggcatcct accgcaaaag g 21
<210> 134
   <211> 30
   <212> DNA
   <213> Artificial Sequence
<220>
   <223> CH1A1A CDR3 VL
<400> 134
   caccaatatt acacctatcc tctattcacg 30
<210> 135
   <211> 363
   <212> DNA
   <213> Artificial Sequence
<220>
   <223> CH1A1A VH
<400> 135
   caggtgcagc tggtgcagtc tggcgccgaa gtgaagaaac ctggagctag tgtgaaggtg 60
   tcctgcaagg ccagcggcta caccttcacc gagttcggca tgaactgggt ccgacaggct 120
   ccaggccagg gcctcgaatg gatgggctgg atcaacacca agaccggcga ggccacctac 180
   gtggaagagt tcaagggcag agtgaccttc accacggaca ccagcaccag caccgcctac 240
   atggaactgc ggagcctgag aagcgacgac accgccgtgt actactgcgc cagatgggac 300
   ttcgcctatt acgtggaagc catggactac tggggccagg gcaccaccgt gaccgtgtct 360
   agc 363
<210> 136
   <211> 324
   <212> DNA
   <213> Artificial Sequence
<220>
   <223> CH1A1A VL
<400> 136
   gatatccaga tgacccagtc tccatcctcc ctgtctgcat ctgtgggaga cagagtcacc 60
   atcacttgca aggccagtgc ggctgtgggt acgtatgttg cgtggtatca gcagaaacca 120
   gggaaagcac ctaagctcct gatctattcg gcatcctacc gcaaaagggg agtcccatca 180
   aggttcagtg gcagtggatc tgggacagat ttcactctca ccatcagcag tctgcaacct 240
   gaagatttcg caacttacta ctgtcaccaa tattacacct atcctctatt cacgtttggc 300
   cagggcacca agctcgagat caag 324
<210> 137
   <211> 30
   <212> DNA
   <213> Artificial Sequence
<220>
   <223> Anti-CD33 CDR1 VH
<400> 137
   ggctacacca tcaccgacag caacatccac 30
<210> 138
   <211> 39
   <212> DNA
   <213> Artificial Sequence
<220>
   <223> Anti-CD33 CDR2 VH
<400> 138
   tacatctacc cctacaacgg cggcaccgac tacaaccag 39
<210> 139
   <211> 21
   <212> DNA
   <213> Artificial Sequence
<220>
   <223> Anti-CD33 CDR3 VH
<400> 139
   ggcaacccct ggctggccta t 21
<210> 140
   <211> 45
   <212> DNA
   <213> Artificial Sequence
<220>
   <223> Anti-CD33 CDR1 VL
<400> 140
   cgggccagcg agagcctgga caactacggc atccggtttc tgacc 45
<210> 141
   <211> 21
   <212> DNA
   <213> Artificial Sequence
<220>
   <223> Anti-CD33 CDR2 VL
<400> 141
   gccgccagca accagggcag c 21
<210> 142
   <211> 27
   <212> DNA
   <213> Artificial Sequence
<220>
   <223> Anti-CD33 CDR3 VL
<400> 142
   cagcagacca aagaggtgcc ctggtcc 27
<210> 143
   <211> 348
   <212> DNA
   <213> Artificial Sequence
<220>
   <223> Anti-CD33 VH
<400> 143
   gaagtgcagc tggtgcagtc tggcgccgaa gtgaagaaac ccggcagcag cgtgaaggtg     60
   tcctgcaagg ccagcggcta caccatcacc gacagcaaca tccactgggt ccgacaggcc    120
   cctgggcaga gcctggaatg gatcggctac atctacccct acaacggcgg caccgactac    180
   aaccagaagt tcaagaaccg ggccaccctg accgtggaca accccaccaa caccgcctac    240
   atggaactga gcagcctgcg gagcgaggac accgccttct actactgcgt gaacggcaac    300
   ccctggctgg cctattgggg ccagggaacc ctggtcaccg tgtctagc                 348
<210> 144
   <211> 333
   <212> DNA
   <213> Artificial Sequence
<220>
   <223> Anti-CD33 VL
<400> 144
   gacatccagc tgacccagag ccccagcacc ctgtctgcca gcgtgggcga cagagtgacc     60
   atcacctgtc gggccagcga gagcctggac aactacggca tccggtttct gacctggttc    120
   cagcagaagc ccggcaaggc ccccaagctg ctgatgtacg ccgccagcaa ccagggcagc    180
   ggcgtgccaa gcagattcag cggcagcggc tccggcaccg agttcaccct gaccatcagc    240
   agcctgcagc ccgacgactt cgccacctac tactgccagc agaccaaaga ggtgccctgg    300
   tccttcggcc agggcaccaa ggtggaagtg aag                                 333
<210> 145
   <211> 15
   <212> PRT
   <213> Artificial Sequence
<220>
   <223> Linker 2
<400> 145
   Glu Pro Lys Ser Cys Gly Gly Gly Gly Ser Gly Gly Gly Gly Ser
   1               5                   10                  15
<210> 146
   <211> 16
   <212> PRT
   <213> Artificial Sequence
<220>
   <223> Linker 3
<400> 146
   Glu Pro Lys Ser Cys Asp Gly Gly Gly Gly Ser Gly Gly Gly Gly Ser
   1               5                   10                  15
<210> 147
   <211> 32
   <212> PRT
   <213> Artificial Sequence
<220>
   <223> Linker 4
<400> 147
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser Gly Gly
               20                  25                  30
<210> 148
   <211> 34
   <212> PRT
   <213> Artificial Sequence
<220>
   <223> Linker 5
<400> 148
   Ser Gly Gly Gly Ser Gly Gly Gly Ser Glu Gly Gly Gly Ser Glu Gly
   1               5                   10                  15
   Gly Gly Ser Glu Gly Gly Gly Ser Glu Gly Gly Gly Ser Gly Gly Gly
               20                  25                  30
   Ser Gly
<210> 149
   <211> 492
   <212> PRT
   <213> Artificial Sequence
<220>
   <223> (scFV)2 CD3-MCSP
<400> 149
   Asp Ile Val Leu Thr Gln Ser Pro Ser Ser Leu Ser Ala Ser Leu Gly
   1 5 10 15
   Asp Arg Val Thr Ile Ser Cys Ser Ala Ser Gln Gly Ile Arg Asn Tyr
   20 25 30
   Leu Asn Trp Tyr Gln Gln Arg Pro Asp Gly Thr Val Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Tyr Ser Leu Thr Ile Ser Asn Leu Glu Pro
   65 70 75 80
   Glu Asp Ile Ala Thr Tyr Tyr Cys Gln Gln Tyr Ser Lys Leu Pro Trp
   85 90 95
   Thr Phe Gly Gly Gly Thr Lys Leu Glu Ile Lys Gly Gly Gly Gly Ser
   100 105 110
   Gly Gly Gly Gly Ser Gly Gly Gly Gly Ser Glu Val Gln Leu Gln Glu
   115 120 125
   Ser Gly Pro Gly Leu Val Lys Pro Ser Gln Ser Leu Ser Leu Thr Cys
   130 135 140
   Ser Val Thr Gly Tyr Ser Ile Thr Ser Gly Tyr Tyr Trp Asn Trp Ile
   145 150 155 160
   Arg Gln Phe Pro Gly Asn Lys Leu Glu Trp Met Gly Tyr Ile Thr Tyr
   165 170 175
   Asp Gly Ser Asn Asn Tyr Asn Pro Ser Leu Lys Asn Arg Ile Ser Ile
   180 185 190
   Thr Arg Asp Thr Ser Lys Asn Gln Phe Phe Leu Lys Leu Asn Ser Val
   195 200 205
   Thr Thr Glu Asp Thr Ala Thr Tyr Tyr Cys Ala Asp Phe Asp Tyr Trp
   210 215 220
   Gly Gln Gly Thr Thr Leu Thr Val Ser Ser Gly Gly Gly Gly Ser Glu
   225 230 235 240
   Val Gln Leu Val Glu Ser Gly Gly Gly Leu Val Gln Pro Gly Gly Ser
                   245                 250                 255
   Leu Arg Leu Ser Cys Ala Ala Ser Gly Tyr Ser Phe Thr Gly Tyr Thr
               260                 265                 270
   Met Asn Trp Val Arg Gln Ala Pro Gly Lys Gly Leu Glu Trp Val Ala
           275                 280                 285
   Leu Ile Asn Pro Tyr Lys Gly Val Ser Thr Tyr Asn Gln Lys Phe Lys
       290                 295                 300
   Asp Arg Phe Thr Ile Ser Val Asp Lys Ser Lys Asn Thr Ala Tyr Leu
   305                 310                 315                 320
   Gln Met Asn Ser Leu Arg Ala Glu Asp Thr Ala Val Tyr Tyr Cys Ala
                   325                 330                 335
   Arg Ser Gly Tyr Tyr Gly Asp Ser Asp Trp Tyr Phe Asp Val Trp Gly
               340                 345                 350
   Gln Gly Thr Leu Val Thr Val Ser Ser Val Glu Gly Gly Ser Gly Gly
           355                 360                 365
   Ser Gly Gly Ser Gly Gly Ser Gly Gly Val Asp Asp Ile Gln Met Thr
       370                 375                 380
   Gln Ser Pro Ser Ser Leu Ser Ala Ser Val Gly Asp Arg Val Thr Ile
   385                 390                 395                 400
   Thr Cys Arg Ala Ser Gln Asp Ile Arg Asn Tyr Leu Asn Trp Tyr Gln
                   405                 410                 415
   Gln Lys Pro Gly Lys Ala Pro Lys Leu Leu Ile Tyr Tyr Thr Ser Arg
               420                 425                 430
   Leu Glu Ser Gly Val Pro Ser Arg Phe Ser Gly Ser Gly Ser Gly Thr
           435                 440                 445
   Asp Tyr Thr Leu Thr Ile Ser Ser Leu Gln Pro Glu Asp Phe Ala Thr
       450                 455                 460
   Tyr Tyr Cys Gln Gln Gly Asn Thr Leu Pro Trp Thr Phe Gly Gln Gly
   465                 470                 475                 480
   Thr Lys Val Glu Ile Lys His His His His His His
                   485                 490
<210> 150
   <211> 1536
   <212> DNA
   <213> Artificial Sequence
<220>
   <223> (scFv)2 MCSP-CD3 DNA
<400> 150
   atgggctggt cctgcatcat cctgtttctg gtggccacag ccaccggtgt gcattccgac      60
   atcgtgctga cccagagccc cagcagcctg agcgccagcc tgggcgacag agtgaccatc     120
   agctgcagcg cctcccaggg catcagaaac tacctgaact ggtatcagca gcggcccgac     180
   ggcaccgtga agctgctgat ctactacacc agctccctgc acagcggcgt gcccagcaga     240
   ttttcaggca gcggcagcgg cactgactac agcctgacca tctccaacct ggaacccgag     300
   gacattgcca cctactactg ccagcagtac agcaagctgc cctggacctt cggcggaggc     360
   accaagctgg aaatcaaggg cggaggcgga tccggcggag gtggaagtgg cggcggaggc     420
   tctgaggtgc aattgcagga aagcggccct ggcctggtga aacccagcca gagcctgagc     480
   ctgacctgca gcgtgaccgg ctactccatc accagcggct actactggaa ctggatcaga     540
   cagttccccg gaaacaagct ggaatggatg ggctacatca cctacgacgg cagcaacaac     600
   tacaacccca gcctgaagaa ccggatcagc atcacccggg acaccagcaa gaaccagttc     660
   ttcctgaagc tgaacagcgt gaccaccgag gataccgcca cctattactg tgccgacttc     720
   gactactggg gccagggcac caccctgacc gtgtcatccg gtggcggcgg atccgaagtg     780
   cagctggtgg agtctggcgg tggactggtg cagccaggcg gctccctgag actgagctgc     840
   gccgcctccg gctacagctt caccggctac accatgaatt gggtccgcca ggcccctgga     900
   aagggactgg aatgggtggc cctgatcaac ccctacaagg gcgtgagcac ctacaaccag     960
   aagttcaagg accggttcac catcagcgtg gacaagagca agaacacagc ctacctgcag    1020
   atgaactccc tgagagccga ggataccgcc gtgtattact gtgcccgcag cggctactac    1080
   ggcgactccg actggtactt cgacgtgtgg gggcagggaa ccctggtcac cgtgtccagc    1140
   gtggaaggcg gcagcggagg atctggcggc tctggcggaa gcggcggagt ggacgatatc    1200
   cagatgacac agtcccccag ctccctgagc gccagcgtgg gcgacagagt gaccatcacc    1260
   tgtcgggcca gccaggacat ccggaattat ctcaattggt atcagcagaa acctggcaaa    1320
   gctcctaaac tgctgatcta ctacacctcc cggctggaaa gcggcgtgcc cagcagattt    1380
   tccggcagcg ggagcggcac cgattacaca ctgaccatca gcagcctgca gcccgaggac    1440
   tttgccacct actattgcca gcagggcaac accctgccct ggacctttgg gcagggcaca    1500
   aaggtggaga tcaagcacca ccaccatcac cactga                              1536
<210> 151
   <211> 218
   <212> PRT
   <213> Artificial Sequence
<220>
   <223> Light Chain VL CD33 (Myelotarg)
<400> 151
   Asp Ile Gln Leu Thr Gln Ser Pro Ser Thr Leu Ser Ala Ser Val Gly
   1               5                   10                  15
   Asp Arg Val Thr Ile Thr Cys Arg Ala Ser Glu Ser Leu Asp Asn Tyr
               20                  25                  30
   Gly Ile Arg Phe Leu Thr Trp Phe Gln Gln Lys Pro Gly Lys Ala Pro
           35                  40                  45
   Lys Leu Leu Met Tyr Ala Ala Ser Asn Gln Gly Ser Gly Val Pro Ser
       50                  55                  60
   Arg Phe Ser Gly Ser Gly Ser Gly Thr Glu Phe Thr Leu Thr Ile Ser
   65                  70                  75                  80
   Ser Leu Gln Pro Asp Asp Phe Ala Thr Tyr Tyr Cys Gln Gln Thr Lys
                   85                  90                  95
   Glu Val Pro Trp Ser Phe Gly Gln Gly Thr Lys Val Glu Val Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 152
   <211> 212
   <212> PRT
   <213> Artificial Sequence
<220>
   <223> Light Chain V9 (VL-CH1)
<400> 152
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys
       210
<210> 153
   <211> 459
   <212> PRT
   <213> Artificial Sequence
<220>
   <223> Fab-Crossfab (VH-CH1(CD33 Myelotarg) -VH-CL [V9])
<400> 153
   Glu Val Gln Leu Val Gln Ser Gly Ala Glu Val Lys Lys Pro Gly Ser
   1               5                   10                  15
   Ser Val Lys Val Ser Cys Lys Ala Ser Gly Tyr Thr Ile Thr Asp Ser
               20                  25                  30
   Asn Ile His Trp Val Arg Gln Ala Pro Gly Gln Ser Leu Glu Trp Ile
           35                  40                  45
   Gly Tyr Ile Tyr Pro Tyr Asn Gly Gly Thr Asp Tyr Asn Gln Lys Phe
       50                  55                  60
   Lys Asn Arg Ala Thr Leu Thr Val Asp Asn Pro Thr Asn Thr Ala Tyr
   65                  70                  75                  80
   Met Glu Leu Ser Ser Leu Arg Ser Glu Asp Thr Ala Phe Tyr Tyr Cys
                   85                  90                  95
   Val Asn Gly Asn Pro Trp Leu Ala Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Gly Gly Gly Gly
       210                 215                 220
   Ser Gly Gly Gly Gly Ser Glu Val Gln Leu Val Glu Ser Gly Gly Gly
   225 230 235 240
   Leu Val Gln Pro Gly Gly Ser Leu Arg Leu Ser Cys Ala Ala Ser Gly
   245 250 255
   Tyr Ser Phe Thr Gly Tyr Thr Met Asn Trp Val Arg Gln Ala Pro Gly
   260 265 270
   Lys Gly Leu Glu Trp Val Ala Leu Ile Asn Pro Tyr Lys Gly Val Ser
   275 280 285
   Thr Tyr Asn Gln Lys Phe Lys Asp Arg Phe Thr Ile Ser Val Asp Lys
   290 295 300
   Ser Lys Asn Thr Ala Tyr Leu Gln Met Asn Ser Leu Arg Ala Glu Asp
   305 310 315 320
   Thr Ala Val Tyr Tyr Cys Ala Arg Ser Gly Tyr Tyr Gly Asp Ser Asp
   325 330 335
   Trp Tyr Phe Asp Val Trp Gly Gln Gly Thr Leu Val Thr Val Ser Ser
   340 345 350
   Ala Ser Val Ala Ala Pro Ser Val Phe Ile Phe Pro Pro Ser Asp Glu
   355 360 365
   Gln Leu Lys Ser Gly Thr Ala Ser Val Val Cys Leu Leu Asn Asn Phe
   370 375 380
   Tyr Pro Arg Glu Ala Lys Val Gln Trp Lys Val Asp Asn Ala Leu Gln
   385 390 395 400
   Ser Gly Asn Ser Gln Glu Ser Val Thr Glu Gln Asp Ser Lys Asp Ser
   405 410 415
   Thr Tyr Ser Leu Ser Ser Thr Leu Thr Leu Ser Lys Ala Asp Tyr Glu
   420 425 430
   Lys His Lys Val Tyr Ala Cys Glu Val Thr His Gln Gly Leu Ser Ser
   435 440 445
   Pro Val Thr Lys Ser Phe Asn Arg Gly Glu Cys
   450 455
<210> 154
   <211> 714
   <212> DNA
   <213> Artificial Sequence
<220>
   <223> Light Chain VL(Myelotarg) DNA
<400> 154
   atgggatgga gctgtatcat cctcttcttg gtagcaacag ctaccggtgt gcattccgac      60
   atccagctga cccagagccc ctccacactc tctgcctcag tgggcgatag ggtcaccatt     120
   acttgcagag ctagcgagtc cctggacaac tacggaatcc gcttccttac atggtttcag     180
   cagaagcctg gaaaagcacc aaagctgctc atgtatgccg cttctaatca aggcagtggt     240
   gtgcccagcc ggttctccgg gtctggctca ggaaccgaat ttactctgac cattagctcc     300
   ttgcagcctg atgacttcgc aacatactat tgtcagcaga ccaaggaggt cccatggtct     360
   tttggtcaag gcacaaaagt ggaggttaag cgtacggtgg ctgcaccatc tgtcttcatc     420
   ttcccgccat ctgatgagca gttgaaatct ggaactgcct ctgttgtgtg cctgctgaat     480
   aacttctatc ccagagaggc caaagtacag tggaaggtgg ataacgccct ccaatcgggt     540
   aactcccagg agagtgtcac agagcaggac agcaaggaca gcacctacag cctcagcagc     600
   accctgacgc tgagcaaagc agactacgag aaacacaaag tctacgcctg cgaagtcacc     660
   catcagggcc tgagctcgcc cgtcacaaag agcttcaaca ggggagagtg ttag           714
<210> 155
   <211> 696
   <212> DNA
   <213> Artificial Sequence
<220>
   <223> Light Chain V9 (VL-CH1) DNA
<400> 155
   atgggatgga gctgtatcat cctcttcttg gtagcaacag ctaccggtgt gcattccgat        60
   attcagatga cccagagccc cagctctctg agcgccagcg tgggcgacag agtgaccatc       120
   acctgtcggg ccagccagga catcagaaac tacctgaact ggtatcagca gaagcccggc       180
   aaggccccca agctgctgat ctactacacc agcagactgg aaagcggcgt gccctccaga       240
   ttttccggca gcggctccgg caccgactac accctgacca tcagcagcct gcagcccgag       300
   gatttcgcca catattactg ccagcagggc aataccctgc cctggacctt cggacagggc       360
   acaaaagtgg aaatcaagag cagcgcttcc accaaaggcc cttccgtgtt tcctctggct       420
   cctagctcca agtccacctc tggaggcacc gctgctctcg gatgcctcgt gaaggattat       480
   tttcctgagc ctgtgacagt gtcctggaat agcggagcac tgacctctgg agtgcatact       540
   ttccccgctg tgctgcagtc ctctggactg tacagcctga gcagcgtggt gacagtgccc       600
   agcagcagcc tgggcaccca gacctacatc tgcaacgtga accacaagcc cagcaacacc       660
   aaggtggaca agaaggtgga acccaagtct tgttga                                 696
<210> 156
   <211> 1437
   <212> DNA
   <213> Artificial Sequence
<220>
   <223> Fab-Crossfab (VH-CH1(CD33 Myelotarg)-VH-CL [V9])
<400> 156
   atgggatgga gctgtatcat cctcttcttg gtagcaacag ctaccggtgt gcattccgag      60
   gtgcagctgg tgcagtctgg cgccgaagtg aagaaacccg gcagcagcgt gaaggtgtcc     120
   tgcaaggcca gcggctacac catcaccgac agcaacatcc actgggtgcg ccaggcccct     180
   ggccagtctc tggaatggat cggctacatc tacccctaca acggcggcac cgactacaac    240
   cagaagttca agaaccgggc caccctgacc gtggacaacc ccaccaatac cgcctacatg     300
   gaactgagca gcctgcggag cgaggacacc gccttctact actgcgtgaa cggcaacccc     360
   tggctggcct attggggcca gggaacactc gtgaccgtgt ccagcgctag caccaagggc     420
   cctagcgtgt tccctctggc ccctagcagc aagagcacct ctggcggaac agccgccctg     480
   ggctgcctcg tgaaggacta ctttcccgag cccgtgacag tgtcctggaa ctctggcgcc     540
   ctgacaagcg gcgtgcacac ctttccagcc gtgctgcagt ctagcggcct gtacagcctg     600
   agcagcgtcg tgactgtgcc cagcagcagc ctgggaaccc agacctacat ctgcaacgtg     660
   aaccacaagc ccagcaacac caaggtggac aagaaggtgg aacccaagag ctgcgacggc     720
   ggaggcggat ccgggggagg gggatctgaa gtgcagctgg tggaaagcgg cggaggcctg     780
   gtgcagcctg ggggatctct gagactgagc tgtgccgcct ccggctacag cttcaccggc    840
   tacacaatga attgggtgcg gcaggctccc ggcaagggcc tggaatgggt ggccctgatc     900
   aacccttaca agggcgtgtc cacctataat cagaagttta aggaccgctt caccatcagc     960
   gtggacaagt ccaagaacac cgcctacctg cagatgaact ccctgcgggc cgaggataca    1020
   gccgtgtact actgtgccag aagcggctac tacggcgaca gcgactggta cttcgacgtg    1080
   tggggacagg gcaccctggt gaccgtgtct agtgcctctg tggccgctcc cagcgtgttc    1140
   atcttcccac ctagcgacga gcagctgaag tccggcaccg cttctgtcgt gtgcctgctg    1200
   aacaacttct acccccgcga ggccaaggtg cagtggaaag tggacaatgc cctgcagagc    1260
   ggcaacagcc aggaaagcgt gaccgagcag gacagcaagg actccaccta cagcctgtcc    1320
   agcaccctga cactgagcaa ggccgactac gagaagcaca aggtgtacgc ctgcgaagtg    1380
   acccaccagg gcctgtctag ccccgtgacc aagagcttca accggggcga gtgctga       1437
<210> 157
   <211> 109
   <212> PRT
   <213> Artificial Sequence
<220>
   <223> CD3 VL
<400> 157
   Gln Ala Val Val Thr Gln Glu Ser Ala Leu Thr Thr Ser Pro Gly Glu
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Glu Lys Pro Asp His Leu Phe Thr Gly
           35                  40                  45
   Leu Ile Gly Gly Thr Asn Lys Arg Ala Pro Gly Val Pro Ala Arg Phe
       50                  55                  60
   Ser Gly Ser Leu Ile Gly Asp Lys Ala Ala Leu Thr Ile Thr Gly Ala
   65                  70                  75                  80
   Gln Thr Glu Asp Glu Ala Ile Tyr Phe Cys Ala Leu Trp Tyr Ser Asn
                   85                  90                  95
   Leu Trp Val Phe Gly Gly Gly Thr Lys Leu Thr Val Leu
               100                 105
<210> 158
   <211> 125
   <212> PRT
   <213> Artificial Sequence
<220>
   <223> CD3 VH
<400> 158
   Glu Val Gln Leu Val Glu Ser Gly Gly Gly Leu Val Gln Pro Lys Gly
   1               5                   10                  15
   Ser Leu Lys Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Gln Ser Ile
   65                  70                  75                  80
   Leu Tyr Leu Gln Met Asn Asn Leu Lys Thr Glu Asp Thr Ala Met Tyr
                   85                  90                  95
   Tyr Cys Val Arg His Gly Asn Phe Gly Asn Ser Tyr Val Ser Trp Phe
               100                 105                 110
   Ala Tyr Trp Gly Gln Gly Thr Leu Val Thr Val Ser Ala
           115                 120                 125
<210> 159
   <211> 121
   <212> PRT
   <213> Artificial Sequence
<220>
   <223> Anti-CEA VH
<400> 159
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser
           115                 120
<210> 160
   <211> 108
   <212> PRT
   <213> Artificial Sequence
<220>
   <223> Anti-CEA VL
<400> 160
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
   35 40 45
   Tyr Ser Ala Ser Tyr Arg Lys Arg Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys His Gln Tyr Tyr Thr Tyr Pro Leu
   85 90 95
   Phe Thr Phe Gly Gln Gly Thr Lys Leu Glu Ile Lys
   100 105
<210> 161
   <211> 112
   <212> PRT
   <213> Artificial Sequence
<220>
   <223> Anti-MCSP VH
<400> 161
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Thr Ser Gly
   20 25 30
   Tyr Tyr Trp Asn Trp Ile Arg Gln His Pro Gly Lys Gly Leu Glu Trp
   35 40 45
   Ile Gly Tyr Ile Thr Tyr Asp Gly Ser Asn Asn Tyr Asn Pro Ser Leu
   50 55 60
   Lys Ser Arg Val Thr Ile Ser Arg Asp Thr Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Asp Phe Asp Tyr Trp Gly Gln Gly Thr Leu Val Thr Val Ser Ser
   100 105 110
<210> 162
   <211> 107
   <212> PRT
   <213> Artificial Sequence
<220>
   <223> Anti-MCSP VL
<400> 162
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Lys Leu Pro Trp
                   85                  90                  95
   Thr Phe Gly Gln Gly Thr Lys Val Glu Ile Lys
               100                 105